(19) Europäisches Patentamt
European Patent Office
Office européen des brevets

(11) EP 3 626 201 A1

(12) EUROPÄISCHE PATENTANMELDUNG

(43) Veröffentlichungstag:
**25.03.2020 Patentblatt 2020/13**

(51) Int Cl.:
***A61C 7/08*** *(2006.01)*

(21) Anmeldenummer: **18195715.0**

(22) Anmeldetag: **20.09.2018**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Covestro Deutschland AG**
**51373 Leverkusen (DE)**

(72) Erfinder: **Die Erfindernennung liegt noch nicht vor**

(74) Vertreter: **Levpat**
**c/o Covestro AG**
**Gebäude 4825**
**51365 Leverkusen (DE)**

(54) **3D-GEDRUCKTE KIEFERORTHOPÄDISCHE ZAHNSCHIENE AUS VERNETZTEN POLYMEREN**

(57) Die vorliegende Erfindung betrifft eine kieferorthopädische Zahnschiene aus einem vernetzten Polymer, wobei das vernetzte Polymer eine Glasübergangstemperatur $T_g$, bestimmt mittels mechanisch dynamischer Analyse (DMA) bei einer Frequenz von 1/s DMA als Peak tan $\delta$, von $\geq$ 25°C und $\leq$ 60°C, ein Elastizitätsmodul, bestimmt mittels mechanisch dynamischer Analyse (DMA) als Speichermodul E' bei einer Frequenz von 1/s bei 35°C, von $\geq$ 500 MPa und $\leq$ 4000 MPa und einen Verlustfaktor tan $\delta$, bestimmt mittels mechanisch dynamischer Analyse (DMA) bei einer Frequenz von 1/s bei 35°C, von $\geq$ 0,08 aufweist. Die Erfindung betrifft weiterhin ein Verfahren zur Herstellung ebensolcher Zahnschienen.

EP 3 626 201 A1

## Beschreibung

Die vorliegende Erfindung betrifft eine kieferorthopädische Zahnschiene aus einem vernetzten Polymer, wobei das vernetzte Polymer eine Glasübergangstemperatur $T_g$, bestimmt mittels mechanisch dynamischer Analyse (DMA) bei einer Frequenz von 1/s DMA als Peak tan $\delta$, von $\geq 25°C$ und $\leq 60°C$, ein Elastizitätsmodul, bestimmt mittels mechanisch dynamischer Analyse (DMA) als Speichermodul E' bei einer Frequenz von 1/s bei 35°C, von $\geq$ 500 MPa und $\leq$ 4000 MPa und einen Verlustfaktor tan $\delta$, bestimmt mittels mechanisch dynamischer Analyse (DMA) bei einer Frequenz von 1/s bei 35°C, von $\geq$ 0,08 aufweist. Die Erfindung betrifft weiterhin ein Verfahren zur Herstellung ebensolcher Zahnschienen.

Die Korrektur von Zahnfehlstellungen kann neben ästhetischen auch auf gesundheitlichen Gründen beruhen. Eine korrekte Zahnstellung kann für eine schmerzfreie, funktionale Ausrichtung zwischen Ober- und Unterkiefer wichtig sein und zudem kann das Auftreten weiterer Zahnkrankheiten, wie beispielsweise Parodontose oder Karies, durch falsch ausgerichtete Zähne und damit einhergehende, ungünstigere Zahnreinigungsmöglichkeiten gefördert werden. Aus diesen Gründen wurden schon sehr früh unterschiedlichste Geräte eingesetzt, um die Stellung einzelner oder sämtlicher Zähne des Zahnapparates eines Anwenders in eine natürliche und ästhetische Form zu bringen. Angefangen von einfachen Holzspateln zur manuellen Korrektur einzelner Zähne über mehr oder minder aufwendige Kieferverdrahtungen bis hin zu Zahnspangen wurden viele Gerätschaften genutzt, um über mechanischen Druck die Zähne von einer ursprünglichen Fehl- hin zu einer "richtigen" Zielposition zu bewegen. Das Behandlungsergebnis ergab sich als Funktion aus Anwendungsdauer und ausgeübtem Druck, wobei letzterer sich aus der Kombination aus dem strukturellen Aufbau der Hilfsmittel und den verwendeten Materialien ergibt.

In den letzten Jahren haben sich neben verklebten Draht-Spangen, gegossene und auch über 3D-Druck individuell herstellbare kieferorthopädische Zahnschienen, sogenannte Aligner, zur Behandlung von Zahnreihen mit komplexen Fehlstellungen etabliert. Üblicherweise werden in diesen Verfahren "zukünftige" Zahnstellungen auf dem geplanten Behandlungspfad über einen rechnergesteuerten Prozess prognostiziert und diese angestrebten Zahnkonfigurationen über einen 3D-Druckprozess hergestellt. Die Produktion der Korrekturhilfen für diese Zahnstati erfolgt dann aber über einen Tiefziehprozess spezieller Folien über das gedruckte 3D-Zahnmodell. Der eigentliche Aligner wird also in den allermeisten Fällen nicht direkt mittels 3D-Druck hergestellt. Im Stand der Technik finden sich jedoch auch verschiedene Herstellverfahren zur direkten Erzeugung von Zahnkorrekturschienen mittels 3D Druck.

So beschreibt beispielsweise die US 2016 025 6240 A1 die Herstellung von direktgedruckten Zahnkorrekturschienen nach verschiedenen 3D Druckverfahren, wobei das benutzte Material ein zugelastisches Verhalten zeigen soll. Als gewünschte Materialeigenschaften werden hierbei Materialeigenschaften genannt, wie sie aus der Literatur zu folienbasierten Zahnkorrekturschienen bekannt sind. Beispiele für Materialien, die diese Eigenschaften in 3D gedruckter Form erfüllen werden nicht genannt.

Die US 2013 0095 446 A1 beschreibt die Herstellung von Zahnkorrekturschienen nach einem 3D Druck Verfahren und listet kommerziell verfügbare Verfahren und eine willkürliche Liste von für die Verfahren geeigneten kommerziellen 3D verdruckbaren bio-kompatiblen Materialien auf, die gegebenenfalls zur Herstellung von Zahnkorrekturschienen geeignet sein könnten.

Die US 2013 0122 448 A1 beschreibt die Herstellung einer Zahnkorrekturschiene über den 3D Druck einer Negativform, die dann mit einem flüssigen Material als positiv Form ausgefüllt wird. Die gehärtete Positivform dient dann als Zahnkorrekturschiene.

U.S. Pat. No. 5,975,893 beschreibt die Herstellung tiefgezogener transparenter Zahnkorrekturschienen, wobei das Gebiss gescannt und verschiedene Korrekturpositionen errechnet werden. Davon werden dann Positivmodelle der zu korrigierenden Zähne und des Gebisses mittels eines 3D Druckers gedruckt. Um dieses gedruckte Modell herum wird eine Folie tiefgezogen und als transparente Zahnschiene aufgearbeitet. Bei der Folie handelt es sich um ein oder mehrere hochschmelzende hochmodulige Thermoplasten.

Trotz der schon vorhandenen Alternativen im Stand der Technik existiert weiterhin Bedarf an geeigneten 3D verdruckbaren Materialien, welche sich zum Einsatz als kieferorthopädische Zahnschienen eignen und welche ein genau auf die Umgebungsbedingungen der Anwendung adaptiertes Eigenschaftsprofil aufweisen.

Eine Aufgabe der vorliegenden Erfindung ist es somit, mindestens einen Nachteil des Standes der Technik wenigstens zu einem Teil zu überwinden und Materialien für additive Fertigungsverfahren bereitzustellen, welche eine hohe Auflösung in der Herstellung ermöglichen, eine ausgezeichnete Biokompatibilität aufweisen und unter den physikalischen Bedingungen im Mundraum verbesserte Anwendungseigenschaften aufweisen. Des Weiteren ist eine Aufgabe der Erfindung, diese Gegenstände kosteneffizient und ressourcenschonend bereitstellen zu können.

Erfindungsgemäß gelöst wird die Aufgabe durch eine Zahnschiene gemäß Anspruch 1 und ein Verfahren gemäß Anspruch 10. Vorteilhafte Weiterbildungen sind in den Unteransprüchen angegeben. Sie können beliebig kombiniert werden, sofern sich aus dem Kontext nicht eindeutig das Gegenteil ergibt

Im Sinne der Erfindung ist eine kieferorthopädische Zahnschiene, wobei die Zahnschiene ein vernetztes Polymer umfasst oder aus einem solchen besteht, wobei das vernetzte Polymer eine Glasübergangstemperatur $T_g$, bestimmt mittels mechanisch dynamischer Analyse (DMA) bei einer Frequenz von 1/s DMA als Peak tan $\delta$, von $\geq$ 25°C und $\leq$ 60°C, ein Elastizitätsmodul, bestimmt mittels mechanisch dynamischer Analyse (DMA) als Speichermodul E' bei einer Frequenz von 1/s bei 35°C, von $\geq$ 500 MPa und $\leq$ 4000 MPa und einen Verlustfaktor tan $\delta$, bestimmt mittels mechanisch dynamischer Analyse (DMA) bei einer Frequenz von 1/s bei 35°C, von $\geq$ 0,08 aufweist.

Überraschenderweise hat sich gezeigt, dass kieferorthopädische Zahnschienen mit oben genannten Parametern einen deutlich besseren Behandlungserfolg zeigen, als die üblicherweise verwendeten Zahnschienen. Ohne durch die Theorie gebunden zu sein kann dies daran liegen, dass das eingesetzte Zahnschienenmaterial ein auf die Anwendungstemperatur abgestimmtes Eigenschaftsprofil zeigt. Dies bedeutet, dass die eingesetzten Polymere temperaturabhängige Eigenschaften aufweisen und die hier beanspruchten bevorzugt vernetzten Polymere bei der Behandlungstemperatur im Mundraum eines Anwenders die erforderlichen mechanischen Eigenschaften aufbringen. Dies im Gegensatz zu dem im Stand der Technik beschriebenen Materialien, über deren Eignung im Wesentlichen anhand der Eigenschaften bei Raumtemperatur geurteilt wird. D.h. üblicherweise wird die Eignung eines Materials im Bereich zwischen 20 und 25 °C getestet und als Funktion der erhaltenen mechanischen Werte dieses Material entweder als geeignet oder ungeeignet eingestuft. Bei diesem Ansatz wird allerdings außer Acht gelassen, dass die Einsatzpolymere häufig stark temperaturabhängige Eigenschaften aufweisen und dass sich die Temperaturen während der Anwendung deutlich von der Raumtemperatur unterscheiden. Insofern weisen die hier beanspruchten Zahnschienen ein temperaturmäßig deutlich angepassteres Eigenschaftsprofil auf. Damit verbunden ist insbesondere eine höhere Zähigkeit des Materials bei 35 °C, so dass durch die hier beanspruchten Zahnschienen im Mundraum über längere Zeit deutlich höhere Kräfte aufgebracht werden, als durch die aus dem Stand der Technik bekannten Materialien. Bei Letzteren ist insbesondere festzustellen, dass bei den relevanten Temperaturen in der Mundhöhle deutlich geringere Festigkeiten und/oder geringere Verformbarkeiten resultieren. Das oben beschrieben und charakterisierte Material zeigt bei der Anwendungstemperatur gleichzeitig einen hohen Modul und eine hohe Zähigkeit, bei reversibler, plastischer Verformbarkeit. Neben der temperaturangepassten Eignung ergibt sich ein weiterer Vorteil durch die Auswahl von Polymeren mit den hier genannten physikalischen Eigenschaften. Überraschenderweise wurde gefunden, dass Zahnschienen aus einem Polymer mit der oben angegebenen Glasübergangstemperatur, den oben angegebenen Zugmodul bei 35°C und dem oben angegebenen Verlustfaktor bei 35°C bevorzugte Anwendungseigenschaften aufweisen, welche die Akzeptanz und den Tragekomfort im Mundbereich erhöhen. Das Material ist zäh-elastisch genug um einfach auf die Zähne gesetzt und wieder entfernt werden zu können. Das Material ist rigide genug, um einen ausreichenden Druck auf die Zähne zur Veränderung der Zahnposition ausüben zu können. Des Weiteren ergibt sich eine hinreichende Verformung des Materials im Temperaturbereich der Anwendung, so dass auch eine gewisse Anpassung der Zahnschiene an die aktuelle Zahlenposition erfolgt. Dies kann in Summe zu einer effizienteren Behandlung von Zahnfehlstellungen führen. Ein weiterer Vorteil der erfindungsgemäßen Zahnschienen besteht zudem darin, dass diese nicht thermoplastisch sind und deshalb auch bei Temperaturen von > 60°C° wärmeformbeständig sind, das heißt ihre Form bei höheren Temperaturen im unverformten Zustand nicht verändern. Bevorzugt können höhere Temperaturen sogar dazu benutzt werden leicht verformte Schienen in ihren Ausgangszustand zurück zu formen, da die erfinderischen Polymere einen ausgeprägten Memoryeffekt haben, der die Druckgeometrie als Formbasis hat. Auch dies ist ein Indiz, dass die mechanischen Eigenschaften der erfindungsgemäßen Zahnschienen im Vergleich zu den Materialien aus dem Stand der Technik temperaturbeständiger sind. Weiterhin vorteilhaft ist, dass die eingesetzten Polymere zur Herstellung der erfindungsgemäßen Zahnschienen transparente Zahnkorrekturschienen liefern.

Eine kieferorthopädische Zahnschiene im Sinne der Erfindung ist eine Aufsatzform für eine oder mehrere Zähne oder eine gesamte Zahnreihe, wobei die Schiene zur Behandlung von Zahnfehlstellungen geeignet ist. Zur Behandlung der Zahnfehlstellungen wird die Zahnschiene auf die Zähne aufgesetzt und über einen gewissen Behandlungszeitraum getragen. Durch die räumliche Ausgestaltung der Zahnschiene wird auf speziellen Zahnkontaktpunkten Druck ausgeübt, so dass die Zähne von einer Anfangs- hin zu einer geänderten Endposition verschoben werden. Die Größenordnung der Positionsänderung der Zähne ergibt sich dabei als Funktion der Tragedauer und des aufgewendeten Druckes. Insbesondere hat sich ergeben, dass über die erfindungsgemäß eingesetzten Zahnschienen, mit den erfindungsgemäßen mechanischen Eigenschaften, eine höhere Kraft zur Positionierung der Zähne aufgewendet werden kann, so dass innerhalb kürzerer Zeiten bessere Behandlungserfolge erreichbar sind.

Die Zahnschiene umfasst ein vernetztes Polymer oder besteht aus einem solchen. Dies bedeutet, dass die Zahnschiene entweder nur aus den vernetzten Polymer besteht oder dass die erfindungsgemäße Zahnschiene das vernetzte Polymer aufweist. Es ist also möglich, dass die Zahnschiene neben dem Polymer noch weitere Substanzen umfasst. Beispielsweise können neben dem eigentlichen Polymer noch weitere Hilfsstoffe eingesetzt werden, welche dem Fachmann zur Ausbildung vernetzter Polymernetzwerke bekannt sind. Dies können beispielsweise Starter für die Vernetzungsreaktion, Katalysatoren für die Vernetzung, Flexibilisatoren, Farbstoffe, Füllstoffe, Weichmacher oder weitere strukturgebende Substanzen sein, welche ein adaptiertes Eigenschaftsprofil der Zahnschiene ermöglichen. Bevorzugt besteht die Zahnschiene zu > 50 Gew.-%, des Weiteren bevorzugt zu > 70 Gew.-%, weiterhin bevorzugt zu > 80 und ganz besonders bevorzugt > 90 Gew.-% aus dem vernetzten Polymer.

Die Glasübergangstemperatur $T_g$ der Zahnschiene wird in Zugbeanspruchung mittels mechanisch dynamischer

Analyse (DMA) bei einer Frequenz von 1/s DMA als Peak tan δ bestimmt und liegt ≥ 25°C bis zu ≤ 60°C. Überraschenderweise wurde gefunden, dass besonders vernetzte Polymere (im Folgenden auch als Harze bezeichnet) mit einem $T_g$ zwischen 25 und 60°C, bevorzugt >26°C und < 50°C; des Weiteren bevorzugt >28°C und <45°C, weiterhin bevorzugt >30°C und < 48°C; und >33°C und <45°C nach einer Aushärtung besonders geeignet sind, um die in der Anwendung der Zahnkorrekturschiene im Mundraum notwendigen mechanischen Eigenschaften abzubilden. Im Falle mehrerer Glasübergangstemperaturen im erfindungsgemäßen Polymeren muss erfindungsgemäß mindestens eine im Bereich >25°C und < 45°C liegen.

Das Elastizitätsmodul der Zahnschiene, bestimmt in Zugbeanspruchung mittels mechanisch dynamischer Analyse (DMA) als Speichermodul E' bei einer Frequenz von 1/s DMA bei 35°C, beträgt von ≥ 500 MPa und ≤ 4000 MPa. Dieser Bereich des Elastizitätsmoduls der Zahnschiene hat sich als besonders geeignet erwiesen um im Rahmen kurzer Behandlungsdauern eine ausreichende Kraft auf die Zähne auszuüben. Dieser Bereich ist also geeignet, um relativ schnell einen verlässlichen Behandlungserfolg zu erreichen.

Der Verlustfaktor tan δ der Zahnschiene, gemessen mittels mechanisch dynamischer Analyse (DMA) in Zugbeanspruchung bei 35°C, beträgt ≥ 0,08. Dieser Größenbereich des Verlustfaktors des Materials der Zahnschiene kann zu einem geeigneten zäh-elastischen Verhalten der Zahnschiene insgesamt beitragen. Das zäh-elastische Verhalten ist dabei eine wichtige Kenngröße, welche sowohl Einfluss auf die Applikationseigenschaften der Zahnschiene wie auch auf die Wechselwirkungen der Zahnschiene mit den Zähnen hat. Bevorzugt kann der Verlustfaktor ≥ 0,1, bevorzugt ≥ 0,15, weiterhin bevorzugt ≥ 0,2 und weiterhin bevorzugt ≥ 0,25 betragen.

In einer bevorzugten Ausgestaltung der Zahnschiene kann das Speichermodul E' im Temperaturbereich von ≥ 30°C und ≤ 40°C größer oder gleich 500 MPa und kleiner oder gleich 4000 MPa betragen. Dieser Bereich des Speichermoduls, ermittelt in Zugbeanspruchung, hat sich für die Anwendungssituation im der menschlichen Mundhöhle als besonders effizient erwiesen. Es können geeignet Kräfte zur Repositionierung der Zähne ausgeübt werden, ohne dass die Anwendungseigenschaften in Bezug auf ein einfaches und sicheres Einsetzen und ein einfaches und sicheres Entfernen der Zahnschiene gemindert würden. Geringere Module sind nicht erfindungsgemäß, da diese eine nur unzureichende Kraft zur Repositionierung bereitstellen können. Höhere Module können nachteilig sein, da diese ein einfaches Aufsetzen und ein einfaches Entfernen der Zahnschiene erschweren können, sowie häufig mit einer nicht ausreichenden Zähelastizität verbunden sind, die in Sprödbruch und scharfen Bruchkanten Ausdruck findet. Geeignete Module können > 600 MPa und <3500 MPa, bevorzugt >650 MPa und < 3000 MPa, des Weiteren bevorzugt > 700 MPa und <2500 MPa, und Weiterhin bevorzugt >750 MPa und <2000 MPa betragen. Diese Bereiche des Elastizitätsmoduls können zu einer verlässlichen Wirkung und einen hohen Tragekomfort beitragen.

In einer weiteren Ausgestaltung der Zahnschiene kann das vernetzte Polymer ein vernetztes Polyisocyanat umfassen. Zu den Vorteilen und den Eigenschaften vernetzter Polyisocyanate wird weiter hinten zum erfindungsgemäßen Herstellverfahren ausgeführt.

Innerhalb einer bevorzugten Ausführungsform der Zahnschiene kann das vernetzte Polymer einen Isocyanuratanteil ermittelt über $^{13}C$-NMR von ≥ 3% aufweisen. Insbesondere der Einsatz vernetzter Polymere mit einem hinreichenden Isocyanuratanteil in oben angegebenen Bereich kann zu den bevorzugten temperaturabhängigen Eigenschaften der Zahnschiene beitragen. Insbesondere kann dieser Isocyanuratanteil zu der bevorzugten Festigkeit, ausgedrückt durch das erfindungsgemäße Speichermodul, führen. Weiter bevorzugte Isocyanuratanteile können bevorzugt bei > 5 %, des Weiteren bevorzugt bei > 8 %, weiterhin bevorzugt > 10 % betragen. Die %-Angaben beziehen sich dabei auf den Gewichts-Anteil des Isocyanuratrings.

Weiterhin kann in einem bevorzugten Aspekt der Zahnschiene das vernetzte Polymer einen Urethananteil ermittelt über $^{13}C$ NMR von ≥ 3% aufweisen. Insbesondere der Einsatz vernetzter Polymere mit einem hinreichenden Urethananteil in oben angegebenen Bereich kann zu den bevorzugten temperaturabhängigen Eigenschaften der Zahnschiene beitragen. Insbesondere kann dieser Urethananteil zu der bevorzugten Festigkeit und Zähelastizität, ausgedrückt durch das erfindungsgemäße Speichermodul und den erfindungsgemäßen tan δ, führen. Weiter bevorzugte Urethananteile können bevorzugt bei > 5 %, des Weiteren bevorzugt bei > 7 %, weiterhin bevorzugt > 8 % betragen. Die %-Angaben beziehen sich dabei auf den Gewichts-Anteil der Urethangruppe.

In einer bevorzugten Ausführungsform der Zahnschiene weist das vernetzte Polymer einen Brechungsindex, gemessen mit einem Abbe Refraktometer, von >1,48 RI und < 1,58 RI auf. Dieser Bereich des Brechungsindex, in Kombination mit einer niedrigen Wasserquellung, bevorzugt von < 3 Gew.-%, oder bevorzugt von < 2 Gew.-%, oder bevorzugt von < 1 Gew.-% und oder bevorzugt von < 0,5 Gew.-% und einer guten Chemikalienbeständigkeit bzw. Fleckbeständigkeit gegen typische Nahrungs- und Genussmittel wie Kaffee, Senf, Rotwein mit einer Bewertung von bevorzugt ≥ 3 bevorzugt ≥ 4 und besonders bevorzugt = 5 (Bewertung nach Auftrag der Substanz auf die Produktoberfläche, nach Einwirkzeit von 10 min, anhand der Verfärbung des Produktes: Bewertung mit 5 = ungefärbt und 0 = stark gefärbt in Analogie zu Lackbeständigkeitsprüfungen), kann dazu beitragen, dass die erfindungsgemäße Zahnschiene unter den physikalischen und chemischen Bedingungen der Mundhöhle, d.h. im Temperaturbereich der Mundhöhle und unter den Feuchtebedingungen der Mundhöhle, nahezu nicht sichtbar ist. Ohne durch die Theorie gebunden zu sein ergibt sich das bevorzugt aus der Wahl des vernetzten Polymeren, welches zudem die erfindungsgemäßen mechanischen Eigenschaften aufweist. Diese Bereiche sind dazu geeignet, das Tragen der Schiene weniger auffällig zu gestalten und insofern kann die Motivation des Anwenders zum Tragen der Zahnschiene gesteigert werden. Dies kann dazu beitragen, dass der gewünschte Behandlungserfolg schneller erreicht wird. In weiteren Ausführungsformen kann der Brechungsindex > 1,49 und < 1,56, weiterhin bevorzugt > 1,495 und < 1,54 und des Weiteren bevorzugt < 1,5 und < 1,53 betragen. Die Messung des Brechungsindex erfolgt bei einer Temperatur von 35 °C.

Innerhalb einer weiteren Charakteristik der Zahnschiene kann das vernetzte Polymer eine mittlere Netzbogenlänge nach Flory und Huggins von > 300 g/mol und < 5000 g/mol, aufweisen. Diese Bereiche der Netzbogenlängen der vernetzten Polymere haben sich zum Erhalt ausreichend zähelastischer Eigenschaften der Zahnschiene als besonders geeignet erwiesen. Innerhalb dieses Bereiches kann die Zahnschiene die erforderliche Rigidität und zudem die in der Anwendung nötige Zäh-Elastizität aufweisen. Dies kann zu bevorzugten Anwendungseigenschaften, wie beispielsweise leichtes Einsetzen der Zahnschiene und eine schnelle Drehpositionierung der Zähne beitragen. Die mittlere Netzbogenlänge kann dabei durch eine Quellungsmessung in Aceton bestimmt werden. Beim Quellen eines Netzwerkes mit einem Lösungsmittel führt dessen Eindringen zu einer Volumenzunahme (attraktive Wechselwirkung zwischen Lösemittel und Polymermatrix), und die Gibbsche Mischungsenthalpie $\Delta Gm$ steigt. Eine entropiegetriebene Rückstellkraft $\Delta Gel$ wirkt diesem Prozess entgegen. Beim Erreichen eines Gleichgewichtszustandes wird die Gibbsche freie Energie $\Delta G$ Null. $\Delta Gm$ lässt sich mit Hilfe der Flory-Huggins-Gleichung bestimmen, $\Delta Gm$ folgt aus der Gausschen Netzwerktheorie. Aus der Bedingung $\Delta G=0$ für den Gleichgewichtszustand der Quellung folgt die Flory-Rehner-Gleichung:

$$M_C = \frac{\rho V_{m1}\left(\frac{\emptyset_2}{2} - \emptyset_2^{\frac{1}{3}}\right)}{\ln(1-\emptyset_2) + \emptyset_2 + (\chi \emptyset_2^{\,2})}$$

Mit der Dichte des Polymers und dem Hugginsschen Wechselwirkungsparameter, lässt sich anhand von Quellungsexperimenten zur Bestimmung des Volumenanteils des Polymers das Molekulargewicht der mittleren Netzbogenlänge MC bestimmen.

Alternativ kann die mittlere Netzbogenlänge Mc sowie die Vernetzungsdichte v aus dem Minimum im Gummiplateau einer DMA Messung bei 1 Hz und 0,1% Auslenkung oberhalb vom Tg des Produktes bestimmt werden.

$$\mathrm{Mc} = \frac{\rho}{\nu} = \frac{3\rho RT}{E'_{min}}$$

$$\nu = \frac{E'_{min}}{3RT}$$

*Mc = Netzbogenlänge* $[\frac{g}{mol}]$

*$\rho$ = Dichte des Polymers* $[\frac{kg}{m^3}]$

*R = molare Gaskonstante* $[\frac{J}{mol*K}]$

*T = absolute Temperatur* [*K*]

*E' = Speichermodul* [-]

*v* = molare Vernetzungsdichte $[\frac{\mathrm{mol}}{\mathrm{cm}^3}]$

Bevorzugt kann die Netzbogenlänge > 400 g/mol und < 2000 g/mol, weiterhin bevorzugt >500 g/mol und < 1600 g/mol, des Weiteren bevorzugt > 550 g/mol und < 1400 g/mol und > 600 g/mol und <1200 g/mol betragen.

Im Rahmen einer weiteren Ausführungsform der Zahnschiene kann das Polymer ein transparentes Polymer mit einer Lichttransmission gemessen in einem UV-VIS Spektrometer an einer Probe mit einer Dicke von 1 mm im Wellenlängenbereich von 400-800 nm von >50% sein. Diese Transmission kann dabei besonders bevorzugt mit einem b* Wert im L*a*b* im CIELab-Farbenraum von < 50, bevorzugt < 30, bevorzugt <20 kombiniert sein. Gerade die Auswahl

transparenter Harze oder Harze, welchen nach der Fertigung transparent und farblos sind, können die Trageneigung des Anwenders deutlich steigern und so innerhalb kürzerer Zeit zu einem besseren Behandlungserfolg beitragen. Bevorzugt kann die Transmission in oben angegebenen Wellenlängenbereich > 60%, bevorzugt > 70%, weiterhin bevorzugt > 80% und ebenfalls bevorzugt > 90% betragen. Die CIELab-Werte können über handelsübliche Geräte bestimmt werden.

Innerhalb einer weiteren Ausführungsform der Zahnschiene kann das Polymer ein transparentes Polymer enthaltend Polyurethane und/oder Polysilicone sein und eine Abbe Zahl von > 20 aufweisen. Neben der reinen Transmission haben sich überraschenderweise für die vernetzten Polymere auch dir Abbe-Zahl als wesentlicher Parameter für die nutzerbezogene "Sichtbarkeit" der Zahnschiene unter Tragebedingungen erweisen. Zahnschienen mit den erfindungsgemäßen Abbe-Zahlen sind dabei besonders unauffällig und können so zu einer erhöhten Anwendungszeit durch den Nutzer beitragen. Bevorzugt kann die Abbe-Zahl für oben angegebene Zahnschiene > 25, bevorzugt >30, weiterhin bevorzugt > 35 und ebenfalls bevorzugt > 38 betragen.

Des Weiteren erfindungsgemäß ist ein Verfahren zur Herstellung einer kieferorthopädischen Zahnschiene mittels eines 3D-Druckverfahrens, wobei ein Harz enthaltend actinisch polymerisierbare Doppelbindungen in Form einer kieferorthopädischen Zahnschiene ausgedruckt und polymerisiert wird. Der Stand der Technik liefert keine Hinweise zur Materialentwicklung von geeigneten flüssigen actinisch härtbaren Materialien (Harzformulierungen) für den Einsatz in 3D Druckverfahren zur Herstellung von Zahnkorrekturschienen auf Basis hochvernetzter Polymere. Veröffentlichte Daten diskutieren Materialeigenschaften immer nur in Bezug auf die Eigenschaften bei Raumtemperatur und lassen die Gebrauchseigenschaften bei erhöhter Temperatur unberücksichtigt.

Überraschenderweise wurde gefunden, dass sich die im Folgenden beschriebenen Harze sehr gut bei den Temperaturbedingungen des 3D-Drucks verarbeiten lassen und zudem, dass die resultierenden vernetzten Polymere bei Anwendungsbedingungen geeignete und verbesserte Anwendungseigenschaften zeigen.

Bevorzugt können die erfindungsgemäß einsetzbaren actinisch härtbaren Harze bei einer typischen Viskosität von < 10.000 mPas, bevorzugt < 5000 mPas, besonders bevorzugt < 1000 mPas bei Verarbeitungstemperatur von >5°C und < 150°C, bevorzugt > 15 und < 120°C, besonders bevorzugt > 20 und < 110 °C und ganz besonders bevorzugt > 30 und < 100°C im 3D Drucker verarbeitet werden.

Ein erfindungsgemäßes Verfahren zur Herstellung eines Gegenstandes aus einem Vorläufer kann die folgenden Schritte umfassen:

I) Abscheiden eines radikalisch vernetzten Harzes auf einem Träger, so dass eine Lage eines mit dem Träger verbundenen Aufbaumaterials erhalten wird, welche einem ersten ausgewählten Querschnitt des Vorläufers entspricht;

II) Abscheiden eines radikalisch vernetzten Harzes auf eine zuvor aufgetragene Lage des Aufbaumaterials, so dass eine weitere Lage des Aufbaumaterials erhalten wird, welche einem weiteren ausgewählten Querschnitt des Vorläufers entspricht und welche mit der zuvor aufgetragenen Lage verbunden ist;

III) Wiederholen des Schritts II), bis der Vorläufer gebildet ist;

wobei das Abscheiden eines radikalisch vernetzten Harzes wenigstens in Schritt II) durch Einwirken von Energie auf einen ausgewählten Bereich eines radikalisch vernetzbaren Harzes, entsprechend dem jeweils ausgewählten Querschnitt des Gegenstandes, erfolgt und wobei das radikalisch vernetzbare Harz eine Viskosität (bei Verarbeitungstemperatur, DIN EN ISO 2884-1) von ≥ 5 mPas bis ≤ 100000 mPas aufweist.

Das radikalisch vernetzbare Harz umfasst bevorzugt eine härtbare Komponente, in der mit einem Blockierungsmittel blockierte NCO-Gruppen, Verbindungen mit mindestens zwei Zerewitinoff-aktiven H-Atomen sowie olefinische C=C-Doppelbindungen vorliegen, wobei das Blockierungsmittel ein Isocyanat ist oder das Blockierungsmittel derart ausgewählt ist, dass nach Deblockierung der NCO-Gruppe keine Freisetzung des Blockierungsmittels als freies Molekül stattfindet.

Im erfindungsgemäßen Verfahren wird nach Schritt III) weiterhin Schritt IV) durchgeführt:

IV) Behandeln des nach Schritt III) erhaltenen Vorläufers bei Bedingungen, die ausreichen, um im radikalisch vernetzten Harz des erhaltenen Vorläufers vorliegende NCO-Gruppen zumindest teilweise zu deblockieren und die dadurch erhaltenen funktionellen Gruppen mit Verbindungen mit mindestens zwei Zerewitinoff-aktiven H-Atomen zur Reaktion zu bringen, so dass der Gegenstand erhalten wird.

Im Verfahren kann der Gegenstand in zwei Herstellungsabschnitten erhalten werden. Der erste Herstellungsabschnitt kann als Aufbauabschnitt angesehen werden. Dieser Aufbauabschnitt lässt sich mittels strahlenoptischer additiver Fertigungsverfahren wie der Stereolithographie oder dem DLP (digital light processing)-Verfahren oder auch mittels Inkjet-Druckverfahren kombiniert mit Strahlenvernetzung realisieren und ist Gegenstand der Schritte I), II) und

III). Der zweite Herstellungsabschnitt kann als Härtungsabschnitt angesehen werden und ist Gegenstand des Schritts IV). Hier wird der nach dem Aufbauabschnitt erhaltene Vorläufer oder intermediäre Gegenstand ohne seine Form weiter zu verändern in einen mechanisch dauerhafteren Gegenstand überführt. Das Material, aus dem der Vorläufer im additiven Fertigungsverfahren erhalten wird, wird im Rahmen der vorliegenden Erfindung allgemein als "Aufbaumaterial" bezeichnet.

In Schritt I) des Verfahrens erfolgt das Abscheiden eines radikalisch vernetzten Harzes auf einem Träger. Dieses ist gewöhnlich der erste Schritt in Stereolithographie- und DLP-Verfahren. Auf diese Weise wird eine Lage eines mit dem Träger verbundenen Aufbaumaterials erhalten wird, welche einem ersten ausgewählten Querschnitt des Vorläufers entspricht.

Gemäß der Anweisung von Schritt III) wird Schritt II) so lange wiederholt, bis der gewünschte Vorläufer gebildet ist. In Schritt II) erfolgt das Abscheiden eines radikalisch vernetzten Harzes auf eine zuvor aufgetragene Lage des Aufbaumaterials, so dass eine weitere Lage des Aufbaumaterials erhalten wird, welche einem weiteren ausgewählten Querschnitt des Vorläufers entspricht und welche mit der zuvor aufgetragenen Lage verbunden ist. Bei der zuvor aufgetragenen Lage des Aufbaumaterials kann es sich um die erste Lage aus Schritt I) oder um eine Lage aus einer vorigen Durchlauf des Schritts II) handeln.

Es kann erfindungsgemäß vorgesehen sein, dass das Abscheiden eines radikalisch vernetzten Harzes wenigstens in Schritt II) (vorzugsweise auch in Schritt I) durch Belichten und/oder Bestrahlen eines ausgewählten Bereichs eines radikalisch vernetzbaren Harzes, entsprechend dem jeweils ausgewählten Querschnitt des Gegenstandes, erfolgt. Im Kontext der vorliegenden Erfindung werden die Begriffe "radikalisch vernetzbares Harz" und "radikalisch vernetztes Harz" benutzt. Hierbei wird das radikalisch vernetzbare Harz durch das Belichten und/oder Bestrahlen, welches radikalische Vernetzungsreaktionen auslöst, in das radikalisch vernetzte Harz überführt. Unter "Belichten" wird hierbei die Einwirkung von Licht im Bereich zwischen nahem IR- und nahem UV-Licht (1400 nm bis 315 nm Wellenlänge) verstanden. Die übrigen kürzeren Wellenlängenbereiche werden durch den Begriff "Bestrahlen" abgedeckt, zum Beispiel fernes UV-Licht, Röntgenstrahlung, Gammastrahlung und auch Elektronenstrahlung.

Das Auswählen des jeweiligen Querschnitts erfolgt zweckmäßigerweise durch ein CAD-Programm, mit dem ein Modell des herzustellenden Gegenstandes erzeugt wurde. Diese Operation wird auch "Slicing" genannt, und dient als Grundlage für die Steuerung der Belichtung und/oder Bestrahlung des radikalisch vernetzbaren Harzes.

Das radikalisch vernetzbare Harz kann eine Viskosität (23 °C, DIN EN ISO 2884-1) von ≥ 5 mPas bis ≤ 1000000 mPas aufweisen. Somit ist es zumindest für die Zwecke der additiven Fertigung als flüssiges Harz anzusehen. Vorzugsweise beträgt die Viskosität ≥ 50 mPas bis ≤ 200000 mPas, mehr bevorzugt ≥ 200 mPas bis ≤ 100000 mPas.

Weiterhin umfasst in dem Verfahren das radikalisch vernetzbare Harz bevorzugt eine härtbare Komponente, in der mit einem Blockierungsmittel blockierte NCO-Gruppen, Verbindungen mit mindestens zwei Zerewitinoff-aktiven H-Atomen sowie olefinische C=C-Doppelbindungen vorliegen, wobei das Blockierungsmittel ein Isocyanat ist oder das Blockierungsmittel derart ausgewählt ist, dass nach Deblockierung der NCO-Gruppe keine Freisetzung des Blockierungsmittels als freies Molekül oder als Teil von anderen Molekülen oder Molekülteilen stattfindet.

Neben der härtbaren Komponente kann das radikalisch vernetzbare Harz auch eine nicht härtbare Komponente umfassen, in der beispielsweise Stabilisatoren, Füllstoffe und dergleichen zusammengefasst sind. In der härtbaren Komponente können die blockierten NCO-Gruppen und die olefinischen C=C-Doppelbindungen in getrennten Molekülen und/oder in einem gemeinsamen Molekül vorliegen. Wenn blockierte NCO-Gruppen und olefinische C=C-Doppelbindungen in getrennten Molekülen vorliegen, kann der nach Schritt IV) des Verfahrens erhaltene Körper ein interpenetrierendes Polymer-Netzwerk aufweisen.

In dem Verfahren kann weiterhin nach Schritt III) der Schritt IV) durchgeführt werden. In diesem Schritt erfolgt das Behandeln des nach Schritt III) erhaltenen Vorläufers bei Bedingungen, die ausreichen, um im radikalisch vernetzten Harz des erhaltenen Vorläufers vorliegende NCO-Gruppen zumindest teilweise zu deblockieren und die dadurch erhaltenen funktionellen Gruppen mit Verbindungen mit mindestens zwei Zerewitinoff-aktiven H-Atomen zur Reaktion zu bringen, so dass der Gegenstand erhalten wird. Insofern muss eine Deblockierung der NCO-Gruppen im Sinne der vorliegenden Erfindung nicht zwangsläufig bedeuten, dass wieder eine NCO-Gruppe erhalten wird. Vielmehr kann dieses auch bedeuten, dass eine funktionelle Gruppe wie eine Acylkation-Gruppe nach Deblockierung erhalten werden kann, welche mit anderen funktionellen Gruppen, die Zerewitinoff-aktive H-Atomen aufweisen, unter Ausbildung einer kovalenten Bindung reagiert.

Vorzugsweise wird die Reaktion durchgeführt, bis ≤ 50%, bevorzugt ≤ 30% und mehr bevorzugt ≤ 20% der ursprünglich in der härtbaren Komponente vorhandenen blockierten Isocyanat-Gruppen noch vorhanden sind. Dieses lässt sich mittels Oberflächen-IR-Spektroskopie bestimmen. Es ist weiter bevorzugt, dass in Schritt IV) ≥ 50%, ≥ 60%, ≥ 70% oder ≥ 80%, der in der härtbaren Komponente deblockierten NCO-Gruppen mit der Verbindung mit mindestens zwei Zerewitinoff-aktiven H-Atomen reagieren.

Es ist bevorzugt, dass Schritt IV) erst dann durchgeführt wird, wenn das gesamte Aufbaumaterial des Vorläufers seinen Gelpunkt erreicht hat. Der Gelpunkt wird als erreicht angesehen, wenn in einer dynamisch-mechanischen Analyse (DMA) mit einem Platte/Platte-Oszillationsviskosimeter gemäß ISO 6721-10 bei 20 °C sich die Graphen des Speichermoduls G' und des Verlustmoduls G" kreuzen. Gegebenenfalls wird der Vorläufer weiterer Belichtung und/oder Bestrahlung zur Vervollständigung der radikalischen Vernetzung ausgesetzt. Das radikalisch vernetzte Harz kann ein Speichermodul G' (DMA, Platte/Platte-Oszillationsviskosimeter gemäß ISO 6721-10 bei 20 °C und einer Scherrate von 1/s) von $\geq 10^6$ Pa aufweisen.

Das radikalisch vernetzbare Harz kann weiterhin Additive wie Füllstoffe, UV-Stabilisatoren, Radikalinhibitoren, Antioxidantien, Formtrennmittel, Wasserfänger, Slipadditive, Entschäumer, Verlaufsmittel, Rheologieadditive, Flammschutzmittel und/oder Pigmente enthalten. Diese Hilfs- und Zusatzmittel, ausgenommen Füllstoffe und Flammschutzmittel, liegen bevorzugt in einer Menge von ≤ 10 Gew.-%, vorzugsweise ≤ 5 Gew.-%, oder bevorzugt ≤ 3 Gew.-%, bezogen auf das radikalisch vernetzbare Harz vor. Flammschutzmittel liegen üblicherweise in Mengen von ≤ 70 Gew.-%, bevorzugt ≤ 50 Gew.-%, oder bevorzugt ≤ 30 Gew.-%, berechnet als Gesamtmenge an eingesetzten Flammschutzmitteln, bezogen auf das Gesamtgewicht des radikalisch vernetzbaren Harzes vor. Diese Hilfs- und Zusatzmittel, ausgenommen Füllstoffe und Flammschutzmittel, liegen bevorzugt in einer Menge von ≥ 0,01 Gew.-%, oder bevorzugt von ≥ 0,05 Gew.-% bezogen auf das radikalisch vernetzbare Harz vor.

Geeignete Füllstoffe sind beispielsweise Ruß, Silica, $AlOH_3$, $CaCO_3$, Metallpigmente wie $TiO_2$ und weitere bekannte übliche Füllstoffe. Die Füllstoffe werden bevorzugt in Mengen von ≤ 70 Gew.-%, bevorzugt ≤ 50 Gew.-%, oder bevorzugt ≤ 30 Gew.-%, berechnet als Gesamtmenge an eingesetzten Füllstoffen bezogen auf das Gesamtgewicht des radikalisch vernetzbaren Harzes, eingesetzt.

Geeignete UV-Stabilisatoren können vorzugsweise ausgewählt werden aus der Gruppe, bestehend aus Piperidinderivaten, wie z.B. 4-Benzoyloxy-2,2,6,6-tetramethylpiperidin, 4-Benzoyloxy-1,2,2,6,6-pentamethylpiperidin, Bis-(2,2,6,6-tetra-methyl-4-piperidyl)-sebacat, Bis(1,2,2,6,6-pentamethyl-1-4-piperidinyl)-sebacat, Bis-(2,2,6,6-tetramethyl-4-piperidyl)-suberat, Bis-(2,2,6,6-tetramethyl-4-piperidyl)-dodecandioat; Benzophenonderivaten, wie z.B. 2,4-Dihydroxy-, 2-Hydroxy-4-methoxy-, 2-Hydroxy-4-octoxy-, 2-Hydroxy-4-dodecyloxy- oder 2,2'-Dihydroxy-4-dodecyloxy-benzophenon; Benztriazolderivaten, wie z.B. 2-(2H-Benzotriazol-2-yl)-4,6-di-tert-pentylphenol, 2-(2H-Benzotriazol-2-yl)-6-dodecyl-4-methylphenol, 2-(2H-Benzotriazol-2-yl)-4,6-bis(1-methyl-1-phenylethyl)phenol, 2-(5-Chlor-2H-benzotriazol-2-yl)-6-(1,1-dimethylethyl)-4-methylphenol, 2-(2H-benzotriazol-2-yl)-4-(1,1,3,3-tetramethylbutyl)phenol, 2-(2H-Benzotriazol-2-yl)-6-(1-methyl-1-phenylethyl)-4-(1,1,3,3-tetramethylbutyl)phenol, Isooctyl-3-(3-(2H-benzotriazol-2-yl)-5-(1,1-dimethylethyl)-4-hydroxyphenylpropionat), 2-(2H-Benzotriazol-2-yl)-4,6-bis(1,1-dimethylethyl)phenol, 2-(2H-Benzotriazol-2-yl)-4,6-bis(1-methyl-1-phenylethyl)phenol, 2-(5-Chlor-2H-benzotriazol-2-yl)-4,6-bis(1,1-dimethylethyl)phenol; Oxalaniliden, wie z.B. 2-Ethyl-2'-ethoxy- oder 4-Methyl-4'-methoxyoxalanilid; Salicylsäureestern, wie z.B. Salicylsäurephenylester, Salicylsäure-4-tert-butylphenylester, Salicylsäure-4-tert-octylphenylester; Zimtsäureesterderivaten, wie z.B. α-Cyano-β-methyl-4-methoxyzimtsäuremethylester, α-Cyano-β-methyl-4-methoxyzimtsäurebutyl-ester, α-Cyano-β-phenylzimtsäureethylester, α-Cyano-β-phenylzimtsäureisooctylester; und Malonesterderivaten, wie z.B. 4-Methoxybenzylidenmalonsäuredimethylester, 4-Methoxybenzylidenmalonsäurediethylester, 4-Butoxybenzylidenmalonsäuredimethylester. Diese bevorzugten Lichtstabilisatoren können sowohl einzeln als auch in beliebigen Kombinationen untereinander zum Einsatz kommen.

Besonders bevorzugte UV-Stabilisatoren sind solche, die Strahlung einer Wellenlänge < 400 nm zu einem großen Anteil absorbieren. Hierzu zählen beispielsweise die genannten Benztriazolderivate. Ganz besonders bevorzugte UV-Stabilisatoren sind 2-(5-Chlor-2H-benzotriazol-2-yl)-6-(1,1-dimethylethyl)-4-methylphenol, 2-(2H-benzotriazol-2-yl)-4-(1,1,3,3-tetramethylbutyl)phenol und/oder 2-(5-Chlor-2H-benzotriazol-2-yl)-4,6-bis(1,1-dimethylethyl)phenol.

Gegebenenfalls werden ein oder mehrere der beispielhaft genannten UV-Stabilisatoren dem radikalisch vernetzbaren Harz vorzugsweise in Mengen von 0,001 bis 3,0 Gew.-%, besonders bevorzugt 0,005 bis 2 Gew.-%, berechnet als Gesamtmenge an eingesetzten UV-Stabilisatoren bezogen auf das Gesamtgewicht des radikalisch vernetzbaren Harzes, zugesetzt.

Geeignete Antioxidantien sind vorzugsweise sterisch gehinderten Phenole, welche vorzugsweise ausgewählt werden können aus der Gruppe, bestehend aus 2,6-Di-tert-butyl-4-methylphenol (Ionol), Pentaerythrit-tetrakis(3-(3,5-di-tert-butyl-4-hydroxy-phenyl)-propionat), Octadecyl-3-(3,5-di-tert-butyl-4-hydroxyphenyl)-propionat, Triethylen-glykol-bis(3-tert-butyl-4-hydroxy-5-methylphenyl)-propionat, 2,2'-Thio-bis(4-methyl-6-tert-butylphenol) und 2,2'-Thiodiethyl-bis[3-(3,5-di-tert-butyl-4-hydroxyphenyl)propionat]. Diese können bei Bedarf sowohl einzeln als auch in beliebigen Kombinationen untereinander eingesetzt werden.

Die Antioxidantien werden bevorzugt in Mengen von 0,01 bis 3,0 Gew.-%, oder bevorzugt von 0,02 bis 2,0 Gew.-%, berechnet als Gesamtmenge an eingesetzten Antioxidantien bezogen auf das Gesamtgewicht des radikalisch vernetzbaren Harzes, eingesetzt.

In einer bevorzugten Ausführungsform ist das Blockierungsmittel ausgewählt aus der Gruppe bestehend aus organischen Isocyanaten, Lactamen, Glycerincarbonat, einer Verbindung gemäß der allgemeinen Formel (I):

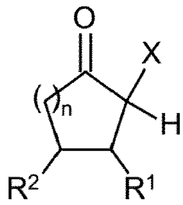
(I)

in welcher X eine elektronenziehende Gruppe, $R^1$ und $R^2$ unabhängig voneinander die Reste H, $C_1$-$C_{20}$-(Cyclo)alkyl, $C_6$-$C_{24}$-Aryl, $C_1$-$C_{20}$-(Cyclo)alkylester oder -amid, $C_6$-$C_{24}$-Arylester oder -amid, gemischt aliphatisch/aromatische Reste mit 1 bis 24 Kohlenstoffatomen, die auch Teil eines 4 bis 8-gliedrigen Ringes sein können, darstellen und n eine ganze Zahl von 0 bis 5 ist, oder einer Kombination aus mindestens zwei hiervon.

Bei der elektronenziehenden Gruppe X kann es sich um alle Substituenten handeln, die zu einer CH-Azidität des $\alpha$-ständigen Wasserstoffes führen. Dies können beispielsweise Estergruppen, Amidgruppen, Sulfoxidgruppen, Sulfongruppen, Nitrogruppen, Phosphonatgruppen, Nitrilgruppen, Isonitrilgruppen, Polyhalogenalkylgruppen, Halogene wie Fluor, Chlor oder Carbonylgruppen sein. Bevorzugt sind Nitril- und Estergruppen, besonders bevorzugt sind Carbonsäuremethylester- und Carbonsäureethylestergruppen. Geeignet sind auch Verbindungen der allgemeinen Formel (I), deren Ring gegebenenfalls Heteroatome, wie Sauerstoff-, Schwefel-, oder Stickstoffatome enthalten. Bevorzugt weist das aktivierte cyclische Keton der Formel (I) eine Ringgröße von 5 (n = 1) und 6 (n = 2) auf.

Bevorzugte Verbindungen der allgemeinen Formel (I) sind Cyclopentanon-2-carboxymethylester und -carboxyethylester, Cyclopentanon-2-carbonsäurenitril, Cyclohexanon-2-carboxymethylester und -carboxyethylester oder Cyclopentanon-2-carbonylmethyl. Besonders bevorzugt sind Cyclopentanon-2-carboxymethylester und -carboxyethylester sowie Cyclohexanon-2-carboxymethylester und-carboxyethylester. Die Cyclopentanonsysteme sind technisch leicht durch eine Dieckmann-Kondensation von Adipinsäuredimethylester oder Adipinsäurediethylester erhältlich. Cyclohexanon-2-carboxymethylester kann durch Hydrierung von Salicylsäuremethylester hergestellt werden.

Bei Verbindungen des Typs (I) verlaufen die Blockierung der NCO-Gruppen, die Deblockierung und die Umsetzung mit Polyolen oder Polyaminen nach dem folgenden beispielhaften Schema:

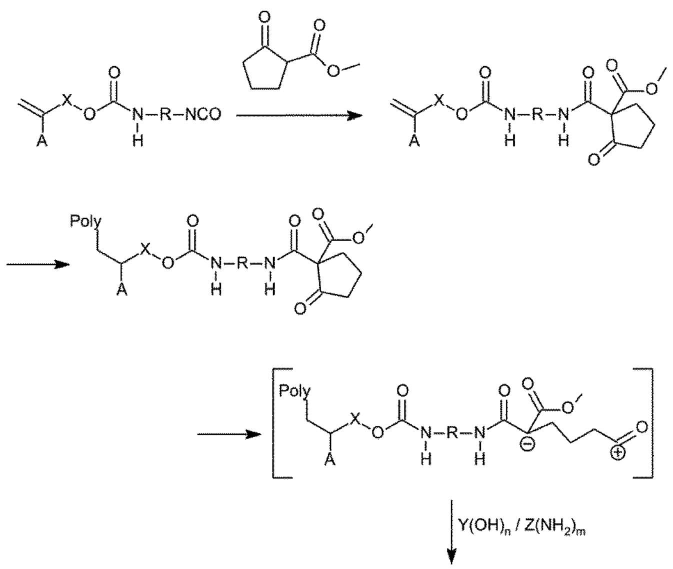


Hierbei steht A für einen beliebigen Rest, vorzugsweise für Wasserstoff oder Alkyl. Die Gruppe X verbindet den Alkenylteil des Moleküls mit dem restlichen Molekül und ist insbesondere eine Carbonylgruppe. Die Gruppe R steht für einen weiteren beliebigen Rest. Beispielsweise kann das Ausgangsmolekül für das obige Schema als das Additionsprodukt von einen Molekül Hydroxyalkyl(meth)acrylat wie 2-Hydroxyalkylmethacrylat (HEMA) an ein Diisocyanat oder ein difunktionelles NCO-terminiertes Prepolymer unter Ausbildung einer Urethangruppe verstanden werden. Das $\beta$-Diketon der allgemeinen Formel (I), in dem $R^1$ und $R^2$ für H und X für $C(O)OCH_3$ steht, addiert mit seinem C-H-aciden C-Atom an die freie NCO-Gruppe unter Ausbildung einer weiteren Urethangruppe. Auf diese Weise wird ein radikalisch polymerisierbares Molekül mit einer blockierten NCO-Gruppe erhalten. Die radikalische Polymerisation der C=C-Doppelbindungen führt zur Ausbildung eines Polymers, dessen Kette schematisch mit "Poly" in dem obigen Schema bezeichnet wurde. Anschließend kann die NCO-Gruppe wieder deblockiert werden. Hierzu wird der Cyclopentanon-Ring geöffnet, wobei sich formal ein Carbanion und ein Acylkation bilden. Dieses ist durch die in eckigen Klammern abgebildete

Zwischenstufe symbolisiert. Ein Polyol $Y(OH)_n$ oder ein Polyamin $Z(NH_2)_m$ (sekundäre Amine sind selbstverständlich auch möglich) mit $n \geq 2$ und $m \geq 2$ addieren formal an das Acylkation mit ihrer OH-Gruppe oder Aminogruppe, wobei weiterhin ein H-Atom zum Carbanion-C-Atom wandert. Wie leicht zu erkennen ist, bleibt das Blockierungsmittel in dem Polymermolekül kovalent gebunden.

Die Blockierung von NCO-Gruppen, deren Deblockierung und Reaktion der nach der Deblockierung erhaltenen funktionellen Gruppen mit Polyolen oder Polyaminen ausgehend von Glycerincarbonat ist beispielhaft in dem nachfolgenden Schema gezeigt:

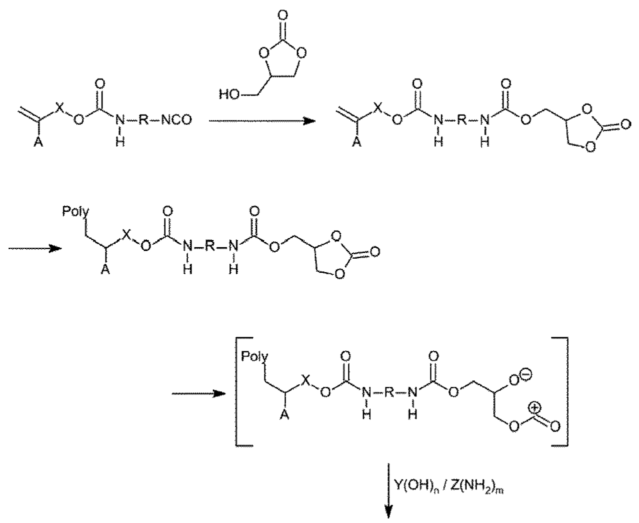


Auch hier steht A für einen beliebigen Rest, vorzugsweise für Wasserstoff oder Alkyl. Die Gruppe X verbindet den Alkenylteil des Moleküls mit dem restlichen Molekül und ist insbesondere eine Carbonylgruppe. Die Gruppe R steht für einen weiteren beliebigen Rest. Beispielsweise kann das Ausgangsmolekül für das obige Schema als das Additionsprodukt von einen Molekül Hydroxyalkyl(meth)acrylat wie 2-Hydroxyalkylmethacrylat (HEMA) an ein Diisocyanat oder ein difunktionelles NCO-terminiertes Prepolymer unter Ausbildung einer Urethangruppe verstanden werden. Das Glycerincarbonat addiert mit seiner freien OH-Gruppe an die freie NCO-Gruppe unter Ausbildung einer weiteren Urethangruppe. Auf diese Weise wird ein radikalisch polymerisierbares Molekül mit einer blockierten NCO-Gruppe erhalten. Die radikalische Polymerisation der C=C-Doppelbindungen führt zur Ausbildung eines Polymers, dessen Kette schematisch mit "Poly" in dem obigen Schema bezeichnet wurde. Anschließend kann die NCO-Gruppe wieder deblockiert werden. Hierzu wird der cyclische Carbonatring geöffnet, wobei sich formal ein Alkoxidion und ein Acylkation bilden. Dieses ist durch die in eckigen Klammern abgebildete Zwischenstufe symbolisiert. Ein Alkohol $Y(OH)_n$ oder ein Amin $Z(NH_2)_m$ (sekundäre Amine sind selbstverständlich auch möglich) mit $n \geq 2$ und $m \geq 2$ addieren formal an das Acylkation mit ihrer OH-Gruppe oder Aminogruppe, wobei weiterhin ein Proton zum Carbanion-C-Atom wandert. Wie leicht zu erkennen ist, bleibt das Blockierungsmittel in dem Polymermolekül kovalent gebunden.

Im Fall der Lactame ist ε-Caprolactam bevorzugt. Das Blockieren und Deblockieren verläuft analog zu den beiden zuvor gezeigten Schemata. Unter Ausbildung einer Harnstoffgruppe addiert die N-H-Gruppe des Lactams an die freie NCO-Gruppe. Nach Polymerisation der C=C-Doppelbindungen kann der Lactamring geöffnet werden. Hierbei entstehen wieder formal ein Acylkation und ein negativ geladenes N-Atom. Alkohole oder Amine können an das Acylkation addieren und das überzählige Proton an das negativ geladene N-Atom übergeben. Auch hier verbleibt das Blockierungsmittel kovalent in dem Polymermolekül gebunden.

Bevorzugt ist der Fall, dass das Blockierungsmittel ein organisches Isocyanat ist. Dann kann die zu blockierende NCO-Gruppe unter Uretdionbildung mit der NCO-Gruppe des Blockierungsmittels reagieren. Die Rückreaktion während Schritt IV) des Verfahrens führt wieder zur Bildung der NCO-Gruppen, welche mit den zur Verfügung stehenden Kettenverlängerern reagieren. Es ist besonders bevorzugt, wenn das Blockierungsmittel und die Verbindung mit der zu blockierenden NCO-Gruppe identisch sind. Dann beinhaltet die Blockierung eine Dimerisierung der betreffenden Verbindung. Dieses und die Reaktion mit Polyol und Polyamin ist in dem nachfolgenden Schema beispielhaft gezeigt.

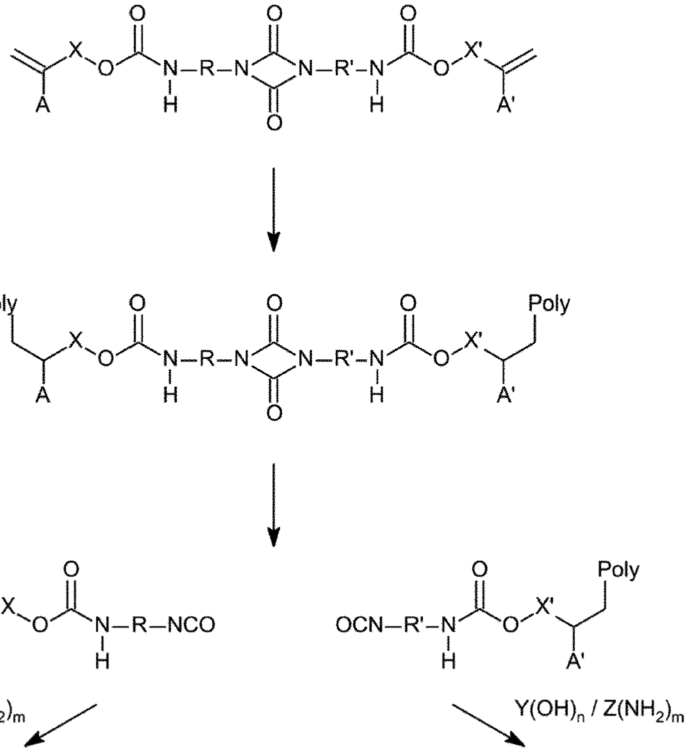


Hier stehen A und A' für einen beliebigen Rest, vorzugsweise für Wasserstoff oder Alkyl. Die Gruppen X und X' verbinden die Alkenylteil der Moleküle mit dem restlichen Molekül und sind insbesondere Carbonylgruppen. Die Gruppen R und R' steht für weitere beliebige Reste. Beispielsweise können die monomeren Ausgangsmoleküle für das obige Schema als Additionsprodukte von einen Molekül Hydroxyalkyl(meth)acrylat wie 2-Hydroxyalkylmethacrylat (HEMA) an ein Diisocyanat oder ein difunktionelles NCO-terminiertes Prepolymer unter Ausbildung einer Urethangruppe verstanden werden. Im Gegensatz zu den vorigen näher erläuterten Blockierungsmitteln erfolgt die Blockierung der NCO-Gruppen durch andere NCO-Gruppen unter Dimerisierung, das heißt unter Ausbildung eines viergliedrigen Uretdion-Rings. Nach radikalischer Polymerisation der C=C-Doppelbindungen ist das Dimer mit den wechselseitig blockierten NCO-Gruppen in die gleiche oder zwei verschiedene Polymerketten "Poly" eingebunden. Die Deblockierung führt zur Öffnung des Uretdion-Ringes unter Rückbildung von zwei NCO-Gruppen. Diese können dann mit Alkoholen oder Aminen umgesetzt werden. Alkohole $Y(OH)_n$ oder Amine $Z(NH_2)_m$ (sekundäre Amine sind selbstverständlich auch möglich) mit $n \geq 2$ und $m \geq 2$ addieren an die NCO-Gruppen unter Ausbildung von Urethan- oder Harnstoffgruppen.

In einer weiteren bevorzugten Ausführungsform sind in der härtbaren Komponente die Verbindungen mit mindestens zwei Zerewitinoff-aktiven H-Atomen ausgewählt aus der Gruppe bestehend aus Polyaminen, Polyolen oder einer Kombination hiervon. Dies können beispielsweise niedermolekulare Diole (z. B. 1,2-Ethandiol, 1,3- bzw. 1,2-Propandiol, 1,4-Butandiol), Triole (z.B. Glycerin, Trimethylolpropan) und Tetraole (z. B. Pentaerythrit) sein, kurzkettige Polyamine aber auch höhermolekulare Polyhydroxyverbindungen wie Polyetherpolyole, Polyesterpolyole, Polycarbonatpolyole, Polysiloxanpolyole, Polyamine und Polyetherpolyamine sowie Polybutadienpolyole.

In einer weiteren bevorzugten Ausführungsform umfasst die härtbare Komponente eine härtbare Verbindung, welche mit dem Blockierungsmittel blockierte NCO-Gruppen und olefinische C=C-Doppelbindungen aufweist.

In einer weiteren bevorzugten Ausführungsform liegen in der härtbaren Verbindung die olefinischen Doppelbindungen zumindest teilweise in Form von (Meth)Acrylatgruppen vor.

Die härtbare Verbindung ist vorzugsweise eine Verbindung, die erhältlich ist aus der Dimerisierung eines Diisocyanats zu einem NCO-terminierten Uretdion, gefolgt von der Umsetzung der NCO-Gruppen mit einem Hydroxyalkyl(meth)acrylat.

Geeignete Diisocyanate zur Herstellung der NCO-terminierten Uretdione sind beispielsweise solche, die ein Molekulargewicht im Bereich von 140 bis 400 g/mol aufweisen, mit aliphatisch, cycloaliphatisch, araliphatisch und/oder aromatisch gebundenen Isocyanatgruppen, wie z. B. 1,4-Diisocyanatobutan (BDI), 1,5-Diisocyanatopentan (PDI), 1,6-Diisocyanatohexan (HDI), 2-Methyl-1,5-diisocyanatopentan, 1,5-Diisocyanato-2,2-dimethylpentan, 2,2,4- bzw. 2,4,4-Trimethyl-1,6-diisocyanatohexan, 1,10-Diisocyanatodecan, 1,3- und 1,4-Diisocyanatocyclohexan, 1,4-Diisocyanato-3,3,5-trimethylcyclohexan, 1,3-Diisocyanato-2-methylcyclohexan, 1,3-Diisocyanato-4-methylcyclohexan, 1-Isocyanato-3,3,5-trimethyl-5-isocyanatomethyl-cyclohexan (Isophorondiisocyanat; IPDI), 1-Isocyanato-1-methyl-4(3)-isocyanatomethylcyclohexan, 2,4'- und 4,4'-Diisocyanatodicyclohexylmethan ($H_{12}MDI$), 1,3- und 1,4-Bis(isocyanatomethyl)cyclohexan, Bis-(isocyanatomethyl)-norbornan (NBDI), 4,4'-Diisocyanato-3,3'-dimethyldicyclohexylmethan, 4,4'-Diisocyanato-3,3',5,5'-tetramethyldicyclohexylmethan, 4,4'-Diisocyanato-1,1'-bi(cyclohexyl), 4,4'-Diisocyanato-3,3'-dimethyl-1,1'-bi(cyclohexyl), 4,4'-Diisocyanato-2,2',5,5'-tetra-methyl-l,1'-bi(cyclohexyl), 1,8-Diisocyanato-p-menthan, 1,3-Diisocyanato-adamantan, 1,3-Dimethyl-5,7-diisocyanatoadamantan, 1,3- und 1,4-Bis-(isocyanatomethyl)benzol (Xyxlylendiisocyanat; XDI), 1,3- und 1,4-Bis(1-isocyanato-1-methylethyl)-benzol (TMXDI) und Bis(4-(1-isocyanato-1-methylethyl)phenyl)-carbonat, 2,4- und 2,6-Diisocyanatotoluol (TDI), 2,4'- und 4,4'-Diisocyanatodiphenylmethan (MDI), 1,5-Diisocyanatonaphthalin sowie beliebige Gemische solcher Diisocyanate.

Ferner können erfindungsgemäß auch aliphatische und/oder aromatische Isocyanat-Endgruppen tragende Prepolymere, wie beispielsweise aliphatische oder aromatische Isocyanat-Endgruppentragende Polyether-, Polyester-, Polyacrylat, Polyepoxyd oder Polycarbonat-Prepolymere als Edukte der Uretdion-Bildung eingesetzt werden.

Geeignete Hydroxyalkyl(meth)acrylate sind unter anderem Alkoxyalkyl(meth)acrylate mit 2 bis 12 Kohlenstoffatomen im Hydroxyalkylrest. Bevorzugt sind 2-Hydroxyethylacrylat, das bei der Anlagerung von Propylenoxid an Acrylsäure entstehende Isomerengemisch oder 4-Hydroxybutylacrylat.

Die Reaktion zwischen dem Hydroxyalkyl(meth)acrylat und dem NCO-terminierten Uretdion kann durch die üblichen Urethanisierungskatalysatoren wie DBTL katalysiert werden. Die erhaltene härtbare Verbindung kann ein zahlenmittleres Molekulargewicht $M_n$ von $\geq$ 200 g/mol bis $\leq$ 5000 g/mol aufweisen. Vorzugsweise beträgt dieses Molekulargewicht $\geq$ 300 g/mol bis $\leq$ 4000 g/mol, mehr bevorzugt $\geq$ 400 g/mol bis $\leq$ 3000 g/mol.

Besonders bevorzugt ist eine härtbare Verbindung, die aus der Reaktion eines NCO-terminierten Uretdions mit Hydroxethyl(meth)acrylat erhalten wurde, wobei das NCO-terminierte Uretdion aus der Dimerisierung von 1,6-Hexamethylendiisocyanat, 1,5-Pentamethylendiisocyanat oder IPDI erhalten wurde. Diese härtbare Verbindung hat ein zahlenmittleres Molekulargewicht $M_n$ von $\geq$ 400 g/mol bis $\leq$ 3000 g/mol und

In einer weiteren bevorzugten Ausführungsform umfasst das radikalisch vernetzbare Harz weiterhin einen Radikalstarter, gegebenenfalls auch einen Katalysator und/oder einen Inhibitor. Um eine unerwünschte Erhöhung der Viskosität des radikalisch vernetzbaren Harzes zu verhindern, kann der Radikalstarter erst unmittelbar vor Beginn des erfindungsgemäßen Verfahrens dem Harz hinzugefügt werden.

Als Radikalstarter kommen thermische und/oder photochemische Radikalstarter (Photoinitiatoren) in Betracht. Es ist auch möglich, dass gleichzeitig thermische und photochemische Radikalstarter eingesetzt werden. Geeignete thermische Radikalstarter sind beispielsweise Azobisisobutyronitril (AIBN), Dibenzoylperoxid (DBPO), Di-tert-butylperoxid und/oder anorganische Peroxide wie Peroxodisulfate.

Bei den Photoinitiatoren wird prinzipiell zwischen zwei Typen unterschieden, dem unimolekularen Typ (I) und dem bimolekularen Typ (II). Geeignete Typ (I)-Systeme sind aromatische Ketonverbindungen, wie z. B. Benzophenone in Kombination mit tertiären Aminen, Alkylbenzophenone, 4,4'-Bis(dimethylamino)benzophenon (Michlers Keton), Anthron und halogenierte Benzophenone oder Mischungen der genannten Typen. Weiter geeignet sind Typ (II)-Initiatoren wie Benzoin und seine Derivate, Benzilketale, Acylphosphinoxide, 2,4,6-Trimethylbenzoyldiphenylphosphinoxid, Bisacylphosphinoxide, Phenylglyoxylsäureester, Campherchinon, $\alpha$-Aminoalkylphenone, $\alpha,\alpha$-Dialkoxyacetophenone und $\alpha$-Hydroxyalkylphenone. Spezielle Beispiele sind Irgacur®500 (eine Mischung von Benzophenon und (1-Hydroxycyclohexyl)phenylketon, Fa. Ciba, Lampertheim, DE), Irgacure®819 DW (Phenylbis-(2, 4, 6-trimethylbenzoyl)phosphinoxid, Fa. Ciba, Lampertheim, DE) oder Esacure® KIP EM (Oligo-[2-hydroxy-2-methyl-1-[4-(1-methylvinyl)-phenyl]-propanone], Fa. Lamberti, Aldizzate, Italien) und Bis-(4-methoxybenzoyl)diethylgerman. Es können auch Gemische dieser Verbindungen eingesetzt werden.

In einer weiteren bevorzugten Ausführungsform ist der Radikalstarter ausgewählt aus der Gruppe: $\alpha$-Hydroxyphenylketon, Benzyldimethylketal, Bis-(4-methoxybenzoyl)diethylgerman und/oder 2,4,6-Trimethylbenzoyldiphenylphosphinoxid

In einer weiteren bevorzugten Ausführungsform beträgt in dem Harz das molare Verhältnis von freien NCO-Gruppen zu Zerewitinoff-aktiven H-Atomen $\leq$ 0,05 (bevorzugt $\leq$ 0,01, mehr bevorzugt $\leq$ 0,005). Das molare Verhältnis von NCO-Gruppen und Zerewitinoff-aktiven H-Atomen wird auch als NCO-Index oder Kennzahl bezeichnet. Als Träger von Zerewitinoff-aktiven H-Atomen kommen insbesondere Verbindungen mit O-H, N-H oder S-H-Bindungen in Frage. Alternativ oder zusätzlich kann dieser Umstand dadurch ausgedrückt werden, dass das Harz Verbindungen mit freien NCO-Gruppen in einer Menge von $\leq$ 20 Gew.-%, bevorzugt $\leq$ 10 Gew.-%, oder bevorzugt $\leq$ 5 Gew.-%, bezogen auf die Masse des Harzes, enthält. Bevorzugt enthält das Harz Verbindungen mit freien NCO-Gruppen in einer Menge von $\geq$ 0,01 bis $\leq$ 20 Gew.-%, bezogen auf die Masse des Harzes.

In einer weiteren bevorzugten Ausführungsform weist die härtbare Komponente ein zahlenmittleres Molekulargewicht $M_n$ von $\geq$ 200 g/mol bis $\leq$ 5000 g/mol auf. Vorzugsweise beträgt dieses Molekulargewicht $\geq$ 300 g/mol bis $\leq$ 4000 g/mol, mehr bevorzugt $\geq$ 400 g/mol bis $\leq$ 3000 g/mol.

In einer weiteren bevorzugten Ausführungsform umfasst in Schritt IV) das Behandeln des nach Schritt III) erhaltenen Vorläufers bei Bedingungen, die ausreichen, um im radikalisch vernetzten Harz des erhaltenen Vorläufers vorliegende NCO-Gruppen zumindest teilweise zu deblockieren und die dadurch erhaltenen funktionellen Gruppen mit Verbindungen mit mindestens zwei Zerewitinoff-aktiven H-Atomen zur Reaktion zu bringen, ein Erwärmen des Körpers

auf eine Temperatur von ≥ 60°C. Vorzugsweise beträgt diese Temperatur ≥ 80 °C bis ≤ 250 °C, mehr bevorzugt ≥ 90 °C bis ≤ 190 °C. Die gewählte Temperatur oder der gewählte Temperaturbereich in Schritt IV) kann beispielsweise für ≥ 5 Minuten bis ≤ 48 Stunden, bevorzugt ≥ 15 Minuten bis ≤ 24 Stunden und mehr bevorzugt ≥ 1 Stunde bis ≤ 12 Stunden gehalten werden.

In einer weiteren bevorzugten Ausführungsform wird die Oberfläche des nach Schritt III) erhaltenen Vorläufers und/oder des nach Schritt IV) erhaltenen Gegenstands mit einer Zerewitinoff-aktive H-Atome aufweisenden Verbindung kontaktiert, wobei als natürliche Luftfeuchtigkeit in der den Vorläufer und/oder den Gegenstand umgebenden Atmosphäre vorkommendes Wasser ausgenommen ist. Bei einer Reaktion von noch verfügbaren blockierten oder deblockierten NCO-Gruppen mit diesen Verbindungen kann eine Funktionalisierung der Oberflächen erreicht werden. Die Zerewitinoff-aktive H-Atome aufweisende Verbindung kann beispielsweise durch Eintauchen, Aufsprühen oder Bestreichen mit der Oberfläche des Vorläufers in Kontakt gebracht werden. Eine weitere Möglichkeit ist die Kontaktierung über die Gasphase, beispielsweise mittels Ammoniak oder Wasserdampf. Optional kann ein Katalysator die Reaktion beschleunigen.

Beispiele für als Funktionalisierungsreagenz geeignete Verbindungen sind Alkohole, Amine, Säuren und ihre Derivate, Epoxide und insbesondere Polyole, wie zum Beispiel Zucker, Polyacrylatpolyole, Polyesterpolyole, Polyetherpolyole, Polyvinylalkohole, Polycarbonatpolyole, Polyethercarbonatpolyole und Polyestercarbonatpolyole. Weitere Beispiele sind Polyacrylsäure, Polyamide, Polysiloxane, Polyacrylamide, Polyvinylpyrrolidone, Polyvinylbutyrat, Polyketone, Polyetherketone, Polyacetale und Polyamine. Auch zur gezielten Ausbildung von Harnstoffen können Amine eingesetzt werden.

Vorzugsweise wird ein langkettiger Alkylalkohol, ein langkettiges (sekundäres) Alkylamin, eine Fettsäure, ein epoxidierter Fettsäureester, ein (per)fluorierter langkettiger Alkohol oder deren Mischungen eingesetzt. "Langkettig" ist hier als ab 6 C-Atomen, vorzugsweise ab 8 C-Atomen, mehr bevorzugt ab 10 C-Atomen in der längsten Kette der Verbindung zu verstehen. Die Herstellung von modifizierten Polyisocyanaten ist prinzipiell bekannt und beispielsweise in EP-A 0 206 059 und EP-A 0 540 985 beschrieben. Sie erfolgt vorzugsweise bei Temperaturen von 40 bis 180 °C.

In einer weiteren bevorzugten Ausführungsform weist das Verfahren mindestens eine, bevorzugt zwei, drei oder alle der folgenden Merkmale auf:

- der Träger ist innerhalb eines Behälters angeordnet und vertikal in Schwerkraftrichtung absenkbar,
- der Behälter enthält das radikalisch vernetzbare Harz bereitstellt,
- vor jedem Schritt II) wird der Träger um eine vorbestimmte Strecke abgesenkt, wobei über der in vertikaler Richtung gesehen obersten Lage des Aufbaumaterials sich eine Schicht des radikalisch vernetzbaren Harzes bildet und
- in Schritt II) belichtet und/oder bestrahlt ein Energiestrahl den ausgewählten Bereich der Schicht des radikalisch vernetzbaren Harzes, entsprechend dem jeweils ausgewählten Querschnitt des Gegenstandes.

Somit wird gemäß dieser Ausführungsform das additive Fertigungsverfahren der Stereolithographie (SLA) abgedeckt. Der Träger wird bevorzugt jeweils um eine vorbestimmte Strecke von ≥ 1 μm bis ≤ 500 μm abgesenkt.

In einer weiteren bevorzugten Ausführungsform weist das Verfahren mindestens eine, bevorzugt zwei, drei oder alle der folgenden Merkmale auf:

- der Träger ist innerhalb eines Behälters angeordnet und vertikal entgegen der Schwerkraftrichtung anhebbar,
- der Behälter stellt das radikalisch vernetzbare Harz bereit,
- vor jedem Schritt II) wird der Träger um eine vorbestimmte Strecke angehoben, so dass unter der in vertikaler Richtung gesehen untersten Lage des Aufbaumaterials sich eine Schicht des radikalisch vernetzbaren Harzes bildet und
- in Schritt II) belichtet und/oder bestrahlt eine Mehrzahl von Energiestrahlen den ausgewählten Bereich der Schicht des radikalisch vernetzbaren Harzes, entsprechend dem jeweils ausgewählten Querschnitt des Gegenstandes, gleichzeitig.

Somit wird gemäß dieser Ausführungsform das additive Fertigungsverfahren der DLP-Technologie abgedeckt, wenn die Mehrzahl von Energiestrahlen über ein Array von einzeln ansteuerbaren Mikrospiegeln das per Belichtung und/oder Bestrahlung bereitzustellende Bild erzeugen. Der Träger wird bevorzugt jeweils um eine vorbestimmte Strecke von ≥ 1 μm bis ≤ 500 μm angehoben.

Die Erfindung betrifft ebenfalls die Verwendung eines radikalisch vernetzbaren Harzes, welches eine Viskosität

(23 °C, DIN EN ISO 2884-1) von ≥ 5 mPas bis ≤ 1000000 mPas aufweist, in einem additiven Fertigungsverfahren, wobei das radikalisch vernetzbare Harz eine härtbare Komponente umfasst, in der mit einem Blockierungsmittel blockierte NCO-Gruppen, Verbindungen mit mindestens zwei Zerewitinoff-aktiven H-Atomen sowie olefinische C=C-Doppelbindungen vorliegen und wobei das Blockierungsmittel ein Isocyanat ist oder das Blockierungsmittel derart ausgewählt ist, dass nach Deblockierung der NCO-Gruppe keine Freisetzung des Blockierungsmittels als freies Molekül oder als Teil von anderen Molekülen oder Molekülteilen stattfindet.

In einer bevorzugten Ausführungsform der Verwendung umfasst das Harz weiterhin einen Radikalstarter und/oder einen Isocyanat-Trimerisierungskatalysator. Vorzugsweise ist der Radikalstarter ausgewählt aus der Gruppe: $\alpha$-Hydroxyphenylketon, Benzyldimethylketal und/oder 2,4,6-Trimethylbenzoyldiphenylphosphinoxid und/oder der Isocyanurat-Trimerisierungskatalysator ist ausgewählt ist aus: Kaliumacetat, Kaliumacetat in Kombination mit einem Kronenether, Kaliumacetat in Kombination mit einem Polyethylenglykol, Kaliumacetat in Kombination mit einem Polypropylenglykol, Zinnoctoat, Trioctylphosphin und/oder Tributylzinnoxid.

Hinsichtlich der härtbaren Verbindung gelten bei der erfindungsgemäßen Verwendung die gleichen Überlegungen und bevorzugten Ausführungsformen wie zuvor in Hinblick auf das erfindungsgemäße Verfahren. Zur Vermeidung unnötiger Wiederholungen werden sie nicht erneut wiedergegeben. Es sei lediglich angemerkt, dass in einer weiteren bevorzugten Ausführungsform in der härtbaren Verbindung die olefinischen Doppelbindungen zumindest teilweise in Form von (Meth)Acrylatgruppen vorliegen und dass in einer weiteren bevorzugten Ausführungsform die härtbare Verbindung aus der Reaktion eines NCO-terminierten Polyisocyanats, bevorzugt Polyisocyanurats, mit einem, bezogen auf die freien NCO-Gruppen, molaren Unterschuss eines Hydroxyalkyl(meth)acrylats erhältlich ist.

In einer weiteren bevorzugten Ausführungsform der Verwendung umfasst das additive Fertigungsverfahren das Belichten und/oder Bestrahlen eines zuvor ausgewählten Bereichs oder aufgetragenen Bereichs des radikalisch vernetzbaren Harzes. Das additive Fertigungsverfahren kann beispielsweise ein Stereolithographie- oder ein DLP (digital light processing)-Verfahren sein. Ebenfalls kann es ein Inkjet-Verfahren sein. Unter "Belichten" wird hierbei die Einwirkung von Licht im Bereich zwischen nahem IR- und nahem UV-Licht (1400 nm bis 315 nm Wellenlänge) verstanden. Die übrigen kürzeren Wellenlängenbereiche werden durch den Begriff "Bestrahlen" abgedeckt, zum Beispiel fernes UV-Licht, Röntgenstrahlung, Gammastrahlung und auch Elektronenstrahlung.

Ein weiterer Gegenstand der Erfindung ist ein Polymer, erhältlich durch die Vernetzung eines radikalisch vernetzbaren Harzes, welches eine Viskosität (23 °C, DIN EN ISO 2884-1) von ≥ 5 mPas bis ≤ 1000000 mPas aufweist, wobei das radikalisch vernetzbare Harz eine härtbare Komponente umfasst, in der mit einem Blockierungsmittel blockierte NCO-Gruppen, Verbindungen mit mindestens zwei Zerewitinoff-aktiven H-Atomen sowie olefinische C=C-Doppelbindungen vorliegen und wobei das Blockierungsmittel ein Isocyanat ist oder das Blockierungsmittel derart ausgewählt ist, dass nach Deblockierung der NCO-Gruppe keine Freisetzung des Blockierungsmittels als freies Molekül oder als Teil von anderen Molekülen oder Molekülteilen stattfindet. Zur Vermeidung unnötiger Wiederholungen wird hinsichtlich der Details zum radikalisch vernetzbaren Harz, der härtbaren Komponente, der Blockierungsmittel, der Polyisocyanate und der Verbindungen mit mindestens zwei Zerewitinoff-aktiven H-Atomen auf die vorstehenden Ausführungen verwiesen.

Weitere geeignete Harze mit actinisch polymerisierbaren Doppelbindungen sind beispielsweise auch radikalisch vernetzbare Harze in denen NCO-Gruppen und olefinische C=C-Doppelbindungen vorliegen.

Ein bevorzugtes Verfahren umfasst ein radikalisch vernetzbares Harz eine härtbare Komponente, in der NCO-Gruppen und olefinische C=C-Doppelbindungen vorliegen, wobei in der härtbaren Komponente das molare Verhältnis von NCO-Gruppen und olefinischen C=C-Doppelbindungen in einem Bereich von ≥ 1:20 bis ≤ 10:1 liegen kann.

In dem Verfahren wird weiterhin nach Schritt III) weiterhin Schritt IV) durchgeführt:

IV) Behandeln des nach Schritt III) erhaltenen Vorläufers bei Bedingungen, die ausreichen, um im radikalisch vernetzten Harz des erhaltenen Vorläufers vorliegende NCO-Gruppen zumindest teilweise zu Isocyanurat-Gruppen zu trimerisieren, so dass der Gegenstand erhalten wird.

Im erfindungsgemäßen Verfahren wird somit der Gegenstand in zwei Herstellungsabschnitten erhalten. Der erste Herstellungsabschnitt kann als Aufbauabschnitt angesehen werden. Dieser Aufbauabschnitt lässt sich mittels strahlenoptischer additiver Fertigungsverfahren wie dem Inkjet-Verfahren, der Stereolithographie oder dem DLP (digital light processing)-Verfahren realisieren und ist Gegenstand der Schritte I), II) und III). Der zweite Herstellungsabschnitt kann als Härtungsabschnitt angesehen werden und ist Gegenstand des Schritts IV). Hier wird der nach dem Aufbauabschnitt erhaltene Vorläufer oder intermediäre Gegenstand ohne seine Form weiter zu verändern in einen mechanisch dauerhafteren Gegenstand überführt. Das Material, aus dem der Vorläufer im additiven Fertigungsverfahren erhalten wird, wird im Rahmen der vorliegenden Erfindung allgemein als "Aufbaumaterial" bezeichnet.

In Schritt I) des Verfahrens erfolgt das Abscheiden eines radikalisch vernetzten Harzes auf einem Träger. Dieses ist gewöhnlich der erste Schritt in Inkjet-, Stereolithographie- und DLP-Verfahren. Auf diese Weise wird eine Lage eines mit dem Träger verbundenen Aufbaumaterials erhalten, welche einem ersten ausgewählten Querschnitt des Vorläufers entspricht.

Gemäß der Anweisung von Schritt III) wird Schritt II) so lange wiederholt, bis der gewünschte Vorläufer gebildet ist. In Schritt II) erfolgt das Abscheiden eines radikalisch vernetzten Harzes auf eine zuvor aufgetragene Lage des Aufbaumaterials, so dass eine weitere Lage des Aufbaumaterials erhalten wird, welche einem weiteren ausgewählten Querschnitt des Vorläufers entspricht und welche mit der zuvor aufgetragenen Lage verbunden ist. Bei der zuvor aufgetragenen Lage des Aufbaumaterials kann es sich um die erste Lage aus Schritt I) oder um eine Lage aus einer vorigen Durchlauf des Schritts II) handeln.

Es ist kann vorgesehen sein, dass das Abscheiden eines radikalisch vernetzten Harzes wenigstens in Schritt II) (vorzugsweise auch in Schritt I) durch Belichten und/oder Bestrahlen eines ausgewählten Bereichs eines radikalisch vernetzbaren Harzes, entsprechend dem jeweils ausgewählten Querschnitt des Gegenstandes, erfolgt. Dieses kann sowohl durch selektives Belichten (Stereolithographie, DLP) des Harzes als auch durch selektives Auftragen des Harzes, gefolgt von einem Belichtungsschritt, der aufgrund des vorigen selektiven Auftragens des Harzes nicht mehr selektiv sein muss (Inkjet-Verfahren).

Im Kontext der vorliegenden Erfindung werden die Begriffe "radikalisch vernetzbares Harz" und "radikalisch vernetztes Harz" benutzt. Hierbei wird das radikalisch vernetzbare Harz durch das Belichten und/oder Bestrahlen, welches radikalische Vernetzungsreaktionen auslöst, in das radikalisch vernetzte Harz überführt. Unter "Belichten" wird hierbei die Einwirkung von Licht im Bereich zwischen nahem IR- und nahem UV-Licht (1400 nm bis 315 nm Wellenlänge) verstanden. Die übrigen kürzeren Wellenlängenbereiche werden durch den Begriff "Bestrahlen" abgedeckt, zum Beispiel fernes UV-Licht, Röntgenstrahlung, Gammastrahlung und auch Elektronenstrahlung.

Das Auswählen des jeweiligen Querschnitts erfolgt zweckmäßigerweise durch ein CAD-Programm, mit dem ein Modell des herzustellenden Gegenstandes erzeugt wurde. Diese Operation wird auch "Slicing" genannt, und dient als Grundlage für die Steuerung der Belichtung und/oder Bestrahlung des radikalisch vernetzbaren Harzes.

Das radikalisch vernetzbare Harz kann eine Viskosität (23 °C, DIN EN ISO 2884-1) von ≥ 5 mPas bis ≤ 1000000 mPas aufweisen. Somit ist es zumindest für die Zwecke der additiven Fertigung als flüssiges Harz anzusehen. Vorzugsweise beträgt die Viskosität ≥ 50 mPas bis ≤ 200000 mPas, mehr bevorzugt ≥ 500 mPas bis ≤ 100000 mPas.

Weiterhin umfasst in dem Verfahren das radikalisch vernetzbare Harz eine härtbare Komponente, in der NCO-Gruppen und olefinische C=C-Doppelbindungen vorliegen, wobei in der härtbaren Komponente das molare Verhältnis von NCO-Gruppen und olefinischen C=C-Doppelbindungen in einem Bereich von ≥ 1:20 bis ≤ 10:1 (bevorzugt ≥ 1:10 bis ≤ 5:1, mehr bevorzugt ≥ 1:5 bis ≤ 3:1) liegt. Das molekulare Verhältnis dieser funktionellen Gruppen lässt sich durch die Integration der Signale einer Probe im $^{13}C$-NMR-Spektrum ermitteln.

Neben der härtbaren Komponente kann das radikalisch vernetzbare Harz auch eine nicht härtbare Komponente umfassen, in der beispielsweise Stabilisatoren, Füllstoffe und dergleichen zusammengefasst sind. In der härtbaren Komponente können die NCO-Gruppen und die olefinischen C=C-Doppelbindungen in getrennten Molekülen und/oder in einem gemeinsamen Molekül vorliegen. Wenn NCO-Gruppen und olefinische C=C-Doppelbindungen in getrennten Molekülen vorliegen, weist der nach Schritt IV) des erfindungsgemäßen Verfahrens erhaltene Körper ein interpenetrierendes Polymer-Netzwerk auf.

In dem Verfahren wird weiterhin nach Schritt III) weiterhin Schritt IV) durchgeführt. In diesem Schritt erfolgt das Behandeln des nach Schritt III) erhaltenen Vorläufers bei Bedingungen, die ausreichen, um im radikalisch vernetzten Harz des erhaltenen Vorläufers vorliegende NCO-Gruppen zumindest teilweise zu Isocyanurat-Gruppen zu trimerisieren, so dass der Gegenstand erhalten wird.

Das Behandeln in Schritt IV) kann im einfachsten Fall ein Lagern bei Raumtemperatur (20°C) sein. Es ist auch möglich, bei einer Temperatur oberhalb der Raumtemperatur zu lagern. Während des Schritts IV) reagieren die NCO-Gruppen miteinander unter weiterer Vernetzung des zuvor schon radikalisch vernetzten Materials. Diese Reaktion führt zumindest teilweise zur Trimerisierung zu Isocyanurat-Gruppen. Es ist erfindungsgemäß eingeschlossen, dass auch Uretdion-, Allophanat-, Urea-, Urethan-, Biuret-, Iminooxadiazindion- und/oder Oxadiazintriongruppen aus den NCO-Gruppen gebildet werden können. Solche Nebenreaktionen können gezielt eingesetzt werden, um beispielsweise die Glasübergangstemperatur $T_g$ des erhaltenen Materials zu beeinflussen.

Vorzugsweise wird die Reaktion durchgeführt, bis ≤ 20 Gew.-%, bevorzugt ≤ 10 Gew.-% oder bevorzugt ≤ 5 Gew.-% der ursprünglich in der härtbaren Komponente vorhandenen NCO-Gruppen noch vorhanden sind. Dieses lässt sich mittels quantitativer IR-Spektroskopie bestimmen. Es ist weiter bevorzugt, dass in Schritt IV) ≥ 50 Gew.-%, ≥ 60 Gew.-%, ≥ 70 Gew.-% oder ≥ 80 Gew.-%, der in der härtbaren Komponente vorliegenden Isocyanatgruppen in Isocyanurat-Gruppen überführt werden.

Es ist bevorzugt, dass Schritt IV) erst dann durchgeführt wird, wenn das gesamte Aufbaumaterial des Vorläufers seinen Gelpunkt erreicht hat. Der Gelpunkt wird als erreicht angesehen, wenn in einer dynamisch-mechanischen Analyse (DMA) mit einem Platte/Platte-Oszillationsviskosimeter gemäß ISO 6721-10 bei 20 °C sich die Graphen des Speichermoduls G' und des Verlustmoduls G" kreuzen. Gegebenenfalls wird der Vorläufer weiterer Belichtung und/oder Bestrahlung zur Vervollständigung der radikalischen Vernetzung ausgesetzt. Das radikalisch vernetzte Harz kann ein Speichermodul G' (DMA, Platte/Platte-Oszillationsviskosimeter gemäß ISO 6721-10 bei 20 °C und einer Scherrate von 1/s) von ≥ $10^6$ Pa aufweisen.

Das radikalisch vernetzbare Harz enthält bevorzugt Additive wie Füllstoffe, UV-Stabilisatoren, Radikalinhibitoren, Antioxidantien, Formtrennmittel, Wasserfänger, Slipadditive, Entschäumer, Verlaufsmittel, Rheologieadditive, Flammschutzmittel und/oder Pigmente. Die Hilfs- und Zusatzmittel, ausgenommen Füllstoffe und Flammschutzmittel, liegen bevorzugt in einer Menge von ≤ 10 Gew.-%, vorzugsweise ≤ 5 Gew.-%, oder bevorzugt ≤ 3 Gew.-%, bezogen auf das radikalisch vernetzbare Harz vor. Flammschutzmittel liegen bevorzugt in Mengen von ≤ 70 Gew.-%, vorzugsweise ≤ 50 Gew.-%, oder bevorzugt ≤ 30 Gew.-%, berechnet als Gesamtmenge an eingesetzten Flammschutzmitteln bezogen auf das Gesamtgewicht des radikalisch vernetzbaren Harzes vor. Die Additive, ausgenommen Füllstoffe und Flammschutzmittel, liegen bevorzugt in einer Menge von ≥ 0,01 Gew.-%, oder bevorzugt von ≥ 0,05 Gew.-%, bezogen auf das radikalisch vernetzbare Harz vor.

Geeignete Füllstoffe sind beispielsweise $AlOH_3$, $CaCO_3$, Metallpigmente wie $TiO_2$ und weitere bekannte übliche Füllstoffe. Das radikalisch vernetzbare Harz beinhaltet Füllstoffe in Mengen von ≤ 70 Gew.-%, bevorzugt ≤ 50 Gew.-%, oder bevorzugt ≤ 30 Gew.-%, berechnet als Gesamtmenge an eingesetzten Füllstoffen bezogen auf das Gesamtgewicht des radikalisch vernetzbaren Harzes.

Geeignete UV-Stabilisatoren können vorzugsweise ausgewählt werden aus der Gruppe, bestehend aus Piperidinderivaten, wie z.B. 4-Benzoyloxy-2,2,6,6-tetramethylpiperidin, 4-Benzoyloxy-1,2,2,6,6-pentamethylpiperidin, Bis-(2,2,6,6-tetra-methyl-4-piperidyl)-sebacat, Bis(1,2,2,6,6-pentamethyl-1-4-piperidinyl)-sebacat, Bis-(2,2,6,6-tetramethyl-4-piperidyl)-suberat, Bis-(2,2,6,6-tetramethyl-4-piperidyl)-dodecandioat; Benzophenonderivaten, wie z.B. 2,4-Dihydroxy-, 2-Hydroxy-4-methoxy-, 2-Hydroxy-4-octoxy-, 2-Hydroxy-4-dodecyloxy- oder 2,2'-Dihydroxy-4-dodecyloxy-benzophenon; Benztriazolderivaten, wie z.B. 2-(2H-Benzotriazol-2-yl)-4,6-di-tert-pentylphenol, 2-(2H-Benzotriazol-2-yl)-6-dodecyl-4-methylphenol, 2-(2H-Benzotriazol-2-yl)-4,6-bis(1-methyl-1-phenylethyl)phenol, 2-(5-Chlor-2H-benzotriazol-2-yl)-6-(1,1-dimethylethyl)-4-methylphenol, 2-(2H-benzotriazol-2-yl)-4-(1,1,3,3-tetramethylbutyl)phenol, 2-(2H-Benzotriazol-2-yl)-6-(1-methyl-1-phenylethyl)-4-(1,1,3,3-tetramethylbutyl)phenol, Isooctyl-3-(3-(2H-benzotriazol-2-yl)-5-(1,1-dimethylethyl)-4-hydroxyphenylpropionat), 2-(2H-Benzotriazol-2-yl)-4,6-bis(1,1-dimethylethyl)phenol, 2-(2H-Benzotriazol-2-yl)-4,6-bis(1-methyl-1-phenylethyl)phenol, 2-(5-Chlor-2H-benzotriazol-2-yl)-4,6-bis(1,1-dimethylethyl)phenol; Oxalaniliden, wie z.B. 2-Ethyl-2'-ethoxy- oder 4-Methyl-4'-methoxyoxalanilid; Salicylsäureestern, wie z.B. Salicylsäurephenylester, Salicylsäure-4-tert-butylphenylester, Salicylsäure-4-tert-octylphenylester; Zimtsäureesterderivaten, wie z.B. α-Cyano-β-methyl-4-methoxyzimtsäuremethylester, α-Cyano-β-methyl-4-methoxyzimtsäurebutyl-ester, α-Cyano-β-phenylzimtsäureethylester, α-Cyano-β-phenylzimtsäureisooctylester; und Malonesterderivaten, wie z.B. 4-Methoxybenzylidenmalonsäuredimethylester, 4-Methoxybenzylidenmalonsäurediethylester, 4-Butoxybenzylidenmalonsäuredimethylester. Diese bevorzugten Lichtstabilisatoren können sowohl einzeln als auch in beliebigen Kombinationen untereinander zum Einsatz kommen.

Besonders bevorzugte UV-Stabilisatoren sind solche, die Strahlung einer Wellenlänge < 400 nm vollständig absorbieren. Hierzu zählen beispielsweise die genannten Benztriazolderivate. Ganz besonders bevorzugte UV-Stabilisatoren sind 2-(5-Chlor-2H-benzotriazol-2-yl)-6-(1,1-dimethylethyl)-4-methylphenol, 2-(2H-benzotriazol-2-yl)-4-(1,1,3,3-tetramethylbutyl)phenol und/oder 2-(5-Chlor-2H-benzotriazol-2-yl)-4,6-bis(1,1-dimethylethyl)phenol.

Gegebenenfalls werden ein oder mehrere der beispielhaft genannten UV-Stabilisatoren dem radikalisch vernetzbaren Harz vorzugsweise in Mengen von 0,001 bis 3,0 Gew.-%, oder bevorzugt von 0,005 bis 2 Gew.-%, berechnet als Gesamtmenge an eingesetzten UV-Stabilisatoren bezogen auf das Gesamtgewicht des radikalisch vernetzbaren Harzes, zugesetzt.

Geeignete Antioxidantien sind vorzugsweise sterisch gehinderten Phenole, welche vorzugsweise ausgewählt werden können aus der Gruppe, bestehend aus 2,6-Di-tert-butyl-4-methylphenol (Ionol), Pentaerythrit-tetrakis(3-(3,5-di-tert-butyl-4-hydroxy-phenyl)-propionat), Octadecyl-3-(3,5-di-tert-butyl-4-hydroxyphenyl)-propionat, Triethylen-glykol-bis(3-tert-butyl-4-hydroxy-5-methylphenyl)propionat, 2,2'-Thio-bis(4-methyl-6-tert-butylphenol) und 2,2'-Thiodiethyl-bis[3-(3,5-di-tert-butyl-4-hydroxyphenyl)propionat]. Diese können bei Bedarf sowohl einzeln als auch in beliebigen Kombinationen untereinander eingesetzt werden. Diese Antioxidantien werden vorzugsweise in Mengen von 0,01 bis 3,0 Gew.-%, oder bevorzugt 0,02 bis 2,0 Gew.-%, berechnet als Gesamtmenge an eingesetzten Antioxidantien bezogen auf das Gesamtgewicht des radikalisch vernetzbaren Harze, eingesetzt.

Geeignete Radikalinhibitoren bzw. Verzögerer sind besonders solche, die eine unkontrollierte radikalische Polymerisation der Harzformulierung außerhalb des gewünschten (bestrahlten) Bereiches gezielt inhibieren. Diese sind für eine gute Randschärfe und Abbildungsgenauigkeit im Vorläufer entscheidend. Geeignete Radikalinhibitoren müssen entsprechend der gewünschten Radikalausbeute aus dem Bestrahlungs-/Belichtungsschritt und der Polymerisationsgeschwindigkeit und Reaktivität/Selektivität der Doppelbindungsträger ausgesucht werden. Geeignete Radikalinhibitoren sind z. B. 2,2-(2,5-thiophendiyl)bis(5-tertbutylbenzoxazol), Phenothiazin, Hydrochinone, Hydrochinonether, Quinonalkyde und Nitroxylverbindungen sowie Mischungen davon, Benzoquinone, Kupfer Salze, Brenzcatechine, Cresole, Nitrobenzol und Sauerstoff. Die Antioxidantien werden vorzugsweise in Mengen von 0,001 Gew.-% bis 3 Gew.-% eingesetzt.

In einer bevorzugten Ausführungsform liegen in der härtbaren Komponente weiterhin Isocyanurat-Gruppen vor, in der härtbaren Komponente liegt das molare Verhältnis von NCO-Gruppen zu Isocyanurat-Gruppen in einem

Bereich von ≤ 100:1 bis ≥ 1:2 (bevorzugt ≤ 70:1 bis ≥ 1:1, mehr bevorzugt ≤ 50:1 bis ≥ 2:1) liegt und in der härtbaren Komponente liegt das molare Verhältnis von olefinischen C=C-Doppelbindungen zu Isocyanurat-Gruppen in einem Bereich von ≤ 100:1 bis ≥ 1:5 (bevorzugt ≤ 70:1 bis ≥ 1:3, mehr bevorzugt ≤ 50:1 bis ≥ 1:2). Die Isocyanuratgruppen sind vorzugsweise Teil eines Polyisocyanurats. Das molare Verhältnis von NCO-Gruppen oder C=C-Doppelbindungen zu Isocyanurat-Gruppen lässt sich aus der Integration der entsprechenden Signale im $^{13}$C-NMR-Spektrum der Probe bestimmen.

In einer weiteren bevorzugten Ausführungsform umfasst die härtbare Komponente eine härtbare Verbindung, welche NCO-Gruppen und olefinische C=C-Doppelbindungen aufweist, wobei in der härtbaren Verbindung das molare Verhältnis von NCO-Gruppen und olefinischen C=C-Doppelbindungen in einem Bereich von ≥1:20 bis ≤ 10:1 (bevorzugt ≥ 1:10 bis ≤ 5:1, mehr bevorzugt ≥ 1:5 bis ≤ 3:1) liegt. Diese Verbindung enthält somit die zwei genannten Gruppen in einem Molekül.

In einer weiteren bevorzugten Ausführungsform umfasst die härtbare Komponente eine härtbare Verbindung, welche Isocyanuratgruppen, NCO-Gruppen und olefinische C=C-Doppelbindungen aufweist, wobei in der härtbaren Verbindung das molare Verhältnis von NCO-Gruppen und olefinischen C=C-Doppelbindungen in einem Bereich von von ≥1:20 bis ≤ 10:1 (bevorzugt ≥ 1:10 bis ≤ 5:1, mehr bevorzugt ≥ 1:5 bis ≤ 3:1) liegt, in der härtbaren Verbindung das molare Verhältnis von NCO-Gruppen zu Isocyanurat-Gruppen in einem Bereich von ≤ 100:1 bis ≥ 1:2 (bevorzugt ≤70:1 bis ≥ 1:1, mehr bevorzugt ≤50:1 bis ≥ 2:1) liegt und in der härtbaren Verbindung das molare Verhältnis von olefinischen C=C-Doppelbindungen zu Isocyanurat-Gruppen in einem Bereich von ≤ 100:1 bis ≥ 1:5 (bevorzugt ≤ 70:1 bis ≥ 1:3, mehr bevorzugt ≤ 50:1 bis ≥ 1:2) liegt.

Diese Verbindung enthält somit die drei genannten Gruppen in einem Molekül. Mit eingeschlossen sind Polymere mit ihrer Molekulargewichtsverteilung, welche in jedem Molekül des Polymers diese Gruppen aufweisen.

Zur Erhöhung des NCO-Gruppengehalts in der härtbaren Komponente können weitere NCOfunktionelle Verbindungen wie Polyisocyanate und NCO-terminierte Prepolymere hinzugefügt werden. Auf diese Weise können die mechanischen Eigenschaften des erhaltenen Gegenstands weiter angepasst werden.

Zur Erhöhung des Doppelbindungsgehaltes in der härtbaren Komponente können zudem weitere doppelbindungsfunktionelle Verbindungen mit 1 bis 6 C=C-Doppelbindungen mit hinzugefügt werden. Auf diese Weise können die mechanischen Eigenschaften des erhaltenen Gegenstandes sowie die Viskosität des vernetzbaren Harzes weiter angepasst werden.

In einer weiteren bevorzugten Ausführungsform liegen in der härtbaren Verbindung die olefinischen Doppelbindungen zumindest teilweise in Form von (Meth)Acrylatgruppen vor.

In einer weiteren bevorzugten Ausführungsform ist die härtbare Verbindung aus der Reaktion eines NCO-terminierten Polyisocyanatprepolymers mit einem, bezogen auf die freien NCO-Gruppen, molaren Unterschuss eines Hydroxyalkyl(meth)acrylats erhältlich.

In einer weiteren bevorzugten Ausführungsform ist die härtbare Verbindung aus der Reaktion eines NCO-terminierten Polyisocyanurats mit einem, bezogen auf die freien NCO-Gruppen, molaren Unterschuss eines Hydroxyalkyl(meth)acrylats erhältlich.

Geeignete Polyisocyanate zur Herstellung der NCO-terminierten Polyisocyanurate sind beispielsweise solche, die ein Molekulargewicht im Bereich von 140 bis 400 g/mol aufweisen, mit aliphatisch, cycloaliphatisch, araliphatisch und/oder aromatisch gebundenen Isocyanatgruppen, wie z. B. 1,4-Diisocyanatobutan (BDI), 1,5-Diisocyanatopentan (PDI), 1,6-Diisocyanatohexan (HDI), 2-Methyl-1,5-diisocyanatopentan, 1,5-Diisocyanato-2,2-dimethylpentan, 2,2,4- bzw. 2,4,4-Trimethyl-1,6-diisocyanatohexan, 1,10-Diisocyanatodecan, 1,3- und 1,4-Diisocyanatocyclohexan, 1,4-Diisocyanato-3,3,5-trimethylcyclohexan, 1,3-Diisocyanato-2-methylcyclohexan, 1,3-Diisocyanato-4-methylcyclohexan, 1-Isocyanato-3,3,5-trimethyl-5-isocyanatomethyl-cyclohexan (Isophorondiisocyanat; IPDI), 1-Isocyanato-1-methyl-4(3)-isocyanatomethylcyclohexan, 2,4'- und 4,4'-Diisocyanatodicyclohexylmethan ($H_{12}$MDI), 1,3- und 1,4-Bis(isocyanatomethyl)cyclohexan, Bis-(isocyanatomethyl)-norbornan (NBDI), 4,4'-Diisocyanato-3,3'-dimethyldicyclohexylmethan, 4,4'-Diisocyanato-3,3',5,5'-tetramethyldicyclohexylmethan, 4,4'-Diisocyanato-1,1'-bi(cyclohexyl), 4,4'-Diisocyanato-3,3'-dimethyl-1,1'-bi(cyclohexyl), 4,4'-Diisocyanato-2,2',5,5'-tetra-methyl-1,1'-bi(cyclohexyl), 1,8-Diisocyanato-p-menthan, 1,3-Diisocyanato-adamantan, 1,3-Dimethyl-5,7-diisocyanatoadamantan, 1,3- und 1,4-Bis-(isocyanatomethyl)benzol (Xyxlylendiisocyanat; XDI), 1,3- und 1,4-Bis(1-isocyanato-1-methylethyl)-benzol (TMXDI) und Bis(4-(1-isocyanato-1-methylethyl)phenyl)-carbonat, 2,4- und 2,6-Diisocyanatotoluol (TDI), 2,4'- und 4,4'-Diisocyanatodiphenylmethan (MDI), 1,5-Diisocyanatonaphthalin sowie beliebige Gemische solcher Diisocyanate.

Ferner können erfindungsgemäß auch aliphatische und/oder aromatische Isocyanat-Endgruppen tragende Prepolymere, wie beispielsweise aliphatische oder aromatische Isocyanat-Endgruppentragende Polyether-, Polyester-, Polyacrylat, Polyepoxyd oder Polycarbonat-Prepolymere als Edukte der Isocyanurat-Bildung eingesetzt werden. Geeignete Trimerisierungskatalysatoren werden weiter unten im Zusammenhang mit einer anderen Ausführungsform beschrieben.

Geeignete Hydroxyalkyl(meth)acrylate sind unter anderem Alkoxyalkyl(meth)acrylate mit 2 bis 12 Kohlenstoffatomen im Hydroxyalkylrest. Bevorzugt sind 2-Hydroxyethylacrylat, das bei der Anlagerung von Propylenoxid an Acrylsäure entstehende Isomerengemisch oder 4-Hydroxybutylacrylat.

Die Reaktion zwischen dem Hydroxyalkyl(meth)acrylat und dem NCO-terminierten Polyisocyanurat kann durch die üblichen Urethanisierungskatalysatoren wie DBTL katalysiert werden. Bei dieser Reaktion kann das molare Verhältnis zwischen NCO-Gruppen und OH-Gruppen des Hydroxyalkyl(meth)acrylats in einem Bereich von ≥10:1 bis ≤ 1,1:1 (bevorzugt ≥ 5:1 bis ≤ 1,5:1, mehr bevorzugt ≥ 4:1 bis ≤ 2:1) liegen. Die erhaltene härtbare Verbindung kann ein zahlenmittleres Molekulargewicht $M_n$ von ≥ 200 g/mol bis ≤ 5000 g/mol aufweisen. Vorzugsweise beträgt dieses Molekulargewicht ≥ 300 g/mol bis ≤ 4000 g/mol, mehr bevorzugt ≥ 400 g/mol bis ≤ 3000 g/mol.

Besonders bevorzugt ist eine härtbare Verbindung, die aus der Reaktion eines NCO-terminierten Polyisocyanurats mit Hydroxethyl(meth)acrylat erhalten wurde, wobei das NCO-terminierte Polyisocyanurat aus 1,6-Hexamethylendiisocyanat in Gegenwart eines Isocyanat-Trimerisierungskatalysators erhalten wurde. Diese härtbare Verbindung hat ein zahlenmittleres Molekulargewicht $M_n$ von ≥ 400 g/mol bis ≤ 3000 g/mol und ein molares Verhältnis von NCO-Gruppen und olefinischen C=C-Doppelbindungen in einem Bereich von ≥ 1:5 bis ≤ 5:1. besonders bevorzugt ≥1:3 bis ≤ 3:1, ganz besonders bevorzugt. ≥1:2 bis ≤ 2:1.

In einer weiteren bevorzugten Ausführungsform umfasst das radikalisch vernetzbare Harz weiterhin einen Radikalstarter und/oder einen Isocyanat-Trimerisierungskatalysator. Um eine unerwünschte Erhöhung der Viskosität des radikalisch vernetzbaren Harzes zu verhindern, können Radikalstarter und/oder Isocyanat-Trimerisierungskatalysator erst unmittelbar vor Beginn des erfindungsgemäßen Verfahrens dem Harz hinzugefügt werden.

Als Radikalstarter kommen thermische und/oder photochemische Radikalstarter (Photoinitiatoren) in Betracht. Es ist auch möglich, dass gleichzeitig thermische und photochemische Radikalstarter eingesetzt werden. Geeignete thermische Radikalstarter sind beispielsweise Azobisisobutyronitril (AIBN), Dibenzoylperoxid (DBPO), Di-tert-butylperoxid und/oder anorganische Peroxide wie Peroxodisulfate.

Bei den Photoinitiatoren wird prinzipiell zwischen zwei Typen unterschieden, dem unimolekularen Typ (I) und dem bimolekularen Typ (II). Geeignete Typ (I)-Systeme sind aromatische Ketonverbindungen, wie z. B. Benzophenone in Kombination mit tertiären Aminen, Alkylbenzophenone, 4,4'-Bis(dimethylamino)benzophenon (Michlers Keton), Anthron und halogenierte Benzophenone oder Mischungen der genannten Typen. Weiter geeignet sind Typ (II)-Initiatoren wie Benzoin und seine Derivate, Benzilketale, Acylphosphinoxide, 2,4,6-Trimethylbenzoyldiphenylphosphinoxid, Bisacylphosphinoxide, Phenylglyoxylsäureester, Campherchinon, $\alpha$-Aminoalkylphenone, $\alpha,\alpha$-Dialkoxyacetophenone und $\alpha$-Hydroxyalkylphenone. Spezielle Beispiele sind Irgacur®500 (eine Mischung von Benzophenon und (1-Hydroxycyclohexyl)phenylketon, Fa. Ciba, Lampertheim, DE), Irgacure®819 DW (Phenylbis-(2, 4, 6-trimethylbenzoyl)phosphinoxid, Fa. Ciba, Lampertheim, DE) oder Esacure® KIP EM (Oligo-[2-hydroxy-2-methyl-1-[4-(1-methylvinyl)-phenyl]-propanone], Fa. Lamberti, Aldizzate, Italien) und Bis-(4-methoxybenzoyl)diethylgerman. Es können auch Gemische dieser Verbindungen eingesetzt werden.

Bei den Photoinitiatoren sollte darauf geachtet werden, dass sie eine ausreichende Reaktivität gegenüber der verwendeten Strahlenquelle haben. Es sind eine Vielzahl von Photoinitiatoren auf dem Markt bekannt. Durch kommerziell verfügbare Photoinitiatoren wird der Wellenlängenbereich im gesamten UV-VIS Spektrum abgedeckt. Photoinitiatoren finden Einsatz bei der Herstellung von Lacken, Druckfarben und Klebstoffen sowie im Dentalbereich.

Im einer bevorzugten Ausführungsform des Verfahren kommt der Photoinitiator in einer auf die Menge der eingesetzten härtbaren olefinisch ungesättigte Doppelbindungen tragenden Komponente bezogenen Konzentration von 0,01 bis 6,0 Gew.-%, bevorzugt von 0,05 bis 4,0 Gew.-% oder bevorzugt von 0,1 bis 3,0 Gew.-% zum Einsatz.

Geeignete Isocyanat-Trimerisierungskatalysatoren sind prinzipiell alle Verbindungen, die die Addition von Isocyanatgruppen zu Isocyanuratgruppen beschleunigen und dadurch die vorliegenden isocyanatgruppenhaltigen Moleküle vernetzen.

Geeignete Isocyanat-Trimerisierungskatalysatoren sind beispielsweise einfache tertiäre Amine, wie z. B. Triethylamin, Tributylamin, N,N-Dimethylanilin, N-Ethylpiperidin oder N, N'-Dimethylpiperazin. Geeignete Katalysatoren sind auch die in der GB 2 221 465 beschriebenen tertiären Hydroxyalkylamine, wie z. B. Triethanolamin, N-Methyl-diethanolamin, Dimethylethanolamin, N-Isopropyldiethanolamin und 1-(2-Hydroxyethyl)pyrrolidin, oder die aus der GB 2 222 161 bekannten, aus Gemischen tertiärer bicyclischer Amine, wie z. B. DBU, mit einfachen niedermolekularen aliphatischen Alkoholen bestehenden Katalysatorsysteme.

Als Isocyanat-Trimerisierungskatalysatoren ebenfalls geeignet ist eine Vielzahl unterschiedlicher Metallverbindungen. Geeignet sind beispielsweise die in der DE-A 3 240 613 als Katalysatoren beschriebenen Oktoate und Naphthenate von Mangan, Eisen, Cobalt, Nickel, Kupfer, Zink, Zirkonium, Cer oder Blei oder deren Gemische mit Acetaten von Lithium, Natrium, Kalium, Calcium oder Barium, die aus DE-A 3 219 608 bekannten Natrium- und Kalium-Salze von linearen oder verzweigten Alkancarbonsäuren mit bis zu 10 C-Atomen, wie z. B. von Propionsäure, Buttersäure, Valeriansäure, Capronsäure, Heptansäure, Caprylsäure, Pelargonsäure, Caprinsäure und Undecylsäure, die aus der EP-A 0 100 129 bekannten Alkali- oder Erdalkalimetallsalze von aliphatischen, cycloaliphatischen oder aromatischen Mono- und Polycarbonsäuren mit 2 bis 20 C-Atomen, wie z. B. Natrium- oder Kaliumbenzoat, die aus der GB-PS 1 391 066 und GB-PS 1 386 399 bekannten Alkaliphenolate, wie z. B. Natrium- oder Kaliumphenolat, die aus der GB 809 809 bekannten Alkali- und Erdalkalioxide, -hydroxide, -carbonate, -alkoholate und -phenolate, Alkalimetallsalze von enolisierbaren Verbindungen sowie Metallsalze schwacher aliphatischer bzw. cycloaliphatischer Carbonsäuren, wie z. B. Natriummethoxid, Natriumacetat, Kaliumacetat, Natriumacetoessigester, Blei-2-ethylhexanoat und Bleinaphthenat, die aus der EP-A 0 056 158 und EP-A 0 056 159 bekannten, mit Kronenethern oder Polyetheralkoholen komplexierten basischen Alkalimetallverbindungen, wie z. B. komplexierte Natrium- oder Kaliumcarboxylate, das aus der EP-A 0 033 581 bekannte Pyrrolidinon-Kaliumsalz, die aus der Anmeldung EP 13196508.9 bekannten ein- oder mehrkernigen Komplexverbindung von Titan, Zirkonium und/oder Hafnium, wie z. B. Zirkoniumtetra-n-butylat, Zirkoniumtetra-2-ethylhexanoat und Zirkoniumtetra-2-ethylhexylat, sowie Zinnverbindungen der in European Polymer Journal, Vol. 16, 147 - 148 (1979) beschriebenen Art, wie z. B. Dibutylzinndichlorid, Diphenylzinndichlorid, Triphenylstannanol, Tributylzinnacetat, Tributylzinnoxid, Zinnoctoat, Dibutyl(dimethoxy)stannan und Tributylzinnimidazolat.

Die Isocyanat-Trimerisierungskatalysatoren können sowohl einzeln als auch in Form beliebiger Gemische untereinander im erfindungsgemäßen Verfahren eingesetzt werden.

Als Isocyanat-Trimerisierungskatalysatoren hervorgehoben seien Natrium- und Kaliumsalze aliphatischer Carbonsäuren mit 2 bis 20 C-Atomen in Kombination mit Komplexbildnern wie Kronenethern oder Polyethylen- oder Polypropylenglykolen sowie aliphatisch substituierte Zinnverbindungen oder Phosphine.

Bevorzugt kommt der Isocyanat-Trimerisierungskatalysator in dem Verfahren in einer auf die Menge der eingesetzten härtbaren Komponente bezogenen Konzentration von 0,0005 bis 5,0 Gew.-%, bevorzugt von 0,0010 bis 2,0 Gew.-% oder bevorzugt von 0,0015 bis 1,0 Gew.-% zum Einsatz kommen.

Die beim Verfahren zum Einsatz gelangenden Isocyanat-Trimerisierungskatalysatoren sind in den Mengen, die zur Initiierung der Trimerisierungsreaktion benötigt werden, in der Regel ausreichend in dem radikalisch vernetzbaren Harz löslich. Die Zugabe des Isocyanat-Trimerisierungskatalysators erfolgt daher vorzugsweise in Substanz.

In einer weiteren bevorzugten Ausführungsform des Verfahrens ist der Radikalstarter ausgewählt aus der Gruppe: $\alpha$-Hydroxyphenylketon, Benzyldimethylketal und/oder 2,4,6-Trimethylbenzoyldiphenylphosphinoxid, Bis-(4-methoxybenzoyl)diethylgerman (Ivocerin) und/oder der Isocyanurat-Trimerisierungskatalysator ist ausgewählt ist aus: Kaliumacetat, Kaliumacetat in Kombination mit einem Kronenether, Kaliumacetat in Kombination mit einem Polyethylenglykol, Kaliumacetat in Kombination mit einem Polypropylenglykol, Zinnoctoat, Natriumphenolat, Kaliumhydroxid, Trioctylphosphin und/oder Tributylzinnoxid.

In einer weiteren bevorzugten Ausführungsform des Verfahrens beträgt in dem Harz das molare Verhältnis von NCO-Gruppen zu Zerewitinoff-aktiven H-Atomen $\geq$ 500 (bevorzugt $\geq$ 1000, mehr bevorzugt $\geq$ 2000). Das molare Verhältnis von NCO-Gruppen und Zerewitinoff-aktiven H-Atomen wird auch als NCO-Index oder Kennzahl bezeichnet. Als Träger von Zerewitinoff-aktiven H-Atomen kommen insbesondere Verbindungen mit O-H, N-H oder S-H-Bindungen in Frage. Ein möglichst geringer Anteil an Verbindungen mit Zerewitinoff-aktiven H-Atomen führt dazu, dass mehr NCO-Gruppen nach Schritt III) für die Isocyanurat-Bildung zur Verfügung stehen.

Alternativ oder zusätzlich kann dieser Umstand dadurch ausgedrückt werden, dass das Harz Verbindungen mit Zerewitinoff-aktiven H-Atomen in einer Menge von $\leq$ 20 Gew.-%, bevorzugt $\leq$ 10 Gew.-%, oder bevorzugt $\leq$ 5 Gew.-%, bezogen auf die Masse des Harzes, enthält.

In einer weiteren bevorzugten Ausführungsform der Erfindung kann die härtbare Komponente bis zu 8 unterschiedliche reaktive Gruppen aufweisen. Beispielhaft genannt seien actinisch härtbare Doppelbindungen, Epoxide, aliphatische/araliphatische/aromatische/alicyclische Isocyanate, acrylische und methacrylische Doppelbindungen, Alkohole, Thiole, Amine, Carbonsäure, Carbonsäureanhydride u.a), welche ein zahlenmittleres Molekulargewicht $M_n$ von $\geq$ 100 g/mol bis $\leq$ 5000 g/mol aufweisen. Vorzugsweise beträgt dieses Molekulargewicht $\geq$ 150 g/mol bis $\leq$ 4000 g/mol, mehr bevorzugt $\geq$ 200 g/mol bis $\leq$ 3000 g/mol.

In einer weiteren bevorzugten Ausführungsform umfasst in Schritt IV) das Behandeln des nach Schritt III) erhaltenen Vorläufers bei Bedingungen, die ausreichen, um im radikalisch vernetzten Harz des erhaltenen Vorläufers vorliegende NCO-Gruppen zumindest teilweise zu Isocyanurat-Gruppen zu trimerisieren, ein Erwärmen des Körpers auf eine Temperatur von $\geq$ 60°C. Vorzugsweise beträgt diese Temperatur $\geq$ 80°C bis $\leq$ 250°C, mehr bevorzugt $\geq$ 90°C bis $\leq$ 190°C. Die gewählte Temperatur oder der gewählte Temperaturbereich in Schritt IV) kann beispielsweise für $\geq$ 5 Minuten bis $\leq$ 48 Stunden, bevorzugt $\geq$ 15 Minuten bis $\leq$ 24 Stunden und mehr bevorzugt $\geq$ 1 Stunde bis $\leq$ 12 Stunden gehalten werden.

In einer bevorzugten Ausführungsform des Verfahrens wird die Oberfläche des nach Schritt III) erhaltenen Vorläufers und/oder des nach Schritt IV) erhaltenen Gegenstands mit einer Zerewitinoff-aktive H-Atome aufweisenden Verbindung kontaktiert, wobei als natürliche Luftfeuchtigkeit in der den Vorläufer und/oder den Gegenstand umgebenden Atmosphäre vorkommendes Wasser ausgenommen ist. Bei einer Reaktion von noch freien NCO-Gruppen mit diesen Verbindungen kann eine Funktionalisierung der Oberflächen erreicht werden. Die Zerewitinoff-aktive H-Atome aufweisende Verbindung kann beispielsweise durch Eintauchen, Aufsprühen oder Bestreichen mit der Oberfläche des Vorläufers in Kontakt gebracht werden. Eine weitere Möglichkeit ist die Kontaktierung über die Gasphase, beispielsweise mittels Ammoniak oder Wasserdampf. Optional kann ein Katalysator die Reaktion beschleunigen.

Beispiele für als Funktionalisierungsreagenz geeignete Verbindungen sind Alkohole, Amine, Säuren und ihre Derivate, Epoxide und insbesondere Polyole, wie zum Beispiel Zucker, Polyacrylatpolyole, Polyesterpolyole, Polyetherpolyole, Polyvinylalkohole, Polylactone, Polyamide, Copolyamide, Polycarbonatpolyole, Polyethercarbonatpolyole und Polyestercarbonatpolyole, langkettige aliphatische Alkohole, fluorierte oder chlorierte Alkohole. Weitere Beispiele sind Polyacrylsäure, Polyamide, Polysiloxane, Polyacrylamide, Polyvinylpyrrolidone, Polyvinylbutyrat, Polyketone, Polyetherketone, Polyacetale und Polyamine. Auch zur gezielten Ausbildung von Harnstoffen können Amine eingesetzt werden.

Bevorzugt wird ein langkettiger Alkylalkohol, ein langkettiges (sekundäres) Alkylamin, eine Fettsäure, ein epoxidierter Fettsäureester, ein (per)fluorierter langkettiger Alkohol oder deren Mischungen eingesetzt. "Langkettig" ist hier als ab 6 C-Atomen, vorzugsweise ab 8 C-Atomen, mehr bevorzugt ab 10 C-Atomen in der längsten Kette der Verbindung zu verstehen. Die Herstellung von modifizierten Polyisocyanaten ist prinzipiell bekannt und beispielsweise in EP-A 0 206 059 und EP-A 0 540 985 beschrieben. Sie erfolgt vorzugsweise bei Temperaturen von 40 bis 180 °C.

In einer weiteren bevorzugten Ausführungsform weist das Verfahren mindestens eines, bevorzugt mindestens zwei oder drei der folgenden Merkmale auf:

- der Träger ist innerhalb eines Behälters angeordnet und vertikal in Schwerkraftrichtung absenkbar,
- der Behälter enthält das radikalisch vernetzbare Harz in einer Menge, welche ausreicht, um wenigstens den Träger und auf dem Träger abgeschiedenes vernetztes Harz zu bedecken,
- vor jedem Schritt II) wird der Träger um eine vorbestimmte Strecke abgesenkt, so dass über der in vertikaler Richtung gesehen obersten Lage des Aufbaumaterials sich eine Schicht des radikalisch vernetzbaren Harzes bildet und
- in Schritt II) belichtet und/oder bestrahlt ein Energiestrahl den ausgewählten Bereich der Schicht des radikalisch vernetzbaren Harzes, entsprechend dem jeweils ausgewählten Querschnitt des Vorläufers.

Somit wird gemäß dieser Ausführungsform das additive Fertigungsverfahren der Stereolithographie (SLA) abgedeckt. Der Träger kann beispielsweise jeweils um eine vorbestimmte Strecke von $\geq 1$ $\mu$m bis $\leq 2000$ $\mu$m abgesenkt werden.

In einer weiteren bevorzugten Ausführungsform weist das Verfahren mindestens eines, bevorzugt mindestens zwei oder drei der folgenden Merkmale auf:

- der Träger ist innerhalb eines Behälters angeordnet und vertikal entgegen der Schwerkraftrichtung anhebbar,
- der Behälter stellt das radikalisch vernetzbare Harz bereit,
- vor jedem Schritt II) wird der Träger um eine vorbestimmte Strecke angehoben, so dass unter der in vertikaler Richtung gesehen untersten Lage des Aufbaumaterials sich eine Schicht des radikalisch vernetzbaren Harzes bildet und
- in Schritt II) belichtet und/oder bestrahlt eine Mehrzahl von Energiestrahlen den ausgewählten Bereich der Schicht des radikalisch vernetzbaren Harzes, entsprechend dem jeweils ausgewählten Querschnitt des Vorläufers, gleichzeitig.

Somit wird gemäß dieser Ausführungsform das additive Fertigungsverfahren der DLP-Technologie abgedeckt, wenn die Mehrzahl von Energiestrahlen über ein Array von einzeln ansteuerbaren Mikrospiegeln das per Belichtung und/oder Bestrahlung bereitzustellende Bild erzeugen. Der Träger kann beispielsweise jeweils um eine vorbestimmte Strecke von $\geq 1$ $\mu$m bis $\leq 2000$ $\mu$m angehoben werden.

In einer weiteren bevorzugten Ausführungsform weist das Verfahren die zusätzlichen Merkmale auf:

- in Schritt II) wird das radikalisch vernetzbare Harz aus einem Druckkopf, entsprechend dem jeweils ausgewählten Querschnitt des Vorläufers, aufgetragen wird und anschließend belichtet und/oder bestrahlt.

Somit wird gemäß dieser Ausführungsform das additive Fertigungsverfahren der Inkjet-Methode abgedeckt: es wird das vernetzbare Harz gegebenenfalls separat von den erfindungsgemäßen Katalysatoren selektiv durch einen oder mehrere Druckköpfe aufgetragen und die anschließende Härtung durch Bestrahlen und/oder Belichtung kann unselektiv sein, beispielsweise durch eine UV-Lampe. Bei dem oder den Druckköpfen zum Auftragen des Harzes kann es sich um einen (modifizierten) Druckkopf für Tintenstrahldruckverfahren handeln. Der Träger kann vom Druckkopf weg bewegbar ausgestaltet sein oder der Druckkopf kann vom Träger weg bewegbar ausgestaltet sein. Die Inkremente der Abstandsbewegungen zwischen Träger und Druckkopf können beispielsweise in einem Bereich von $\geq 1$ $\mu$m bis $\leq$

2000 μm liegen.

Insbesondere bei dieser Ausführungsform kann durch eine geringe Anzahl von Wiederholungen des Schritts II) ein sehr dünner Vorläufer aufgebaut werden. Dieser Vorläufer kann auch auf einem Substrat als Träger aufgebaut werden, welches eine Funktion in der späteren Verwendung des hergestellten Gegenstandes einnimmt. Dann ist es gerechtfertigt, von dem Auftragen einer Oberfläche auf den Träger oder das Substrat zu sprechen. Das Substrat kann beispielsweise ein Innen- oder Außenteil eines Fahrzeugs sein. Das erfindungsgemäße Verfahren gemäß dieser Ausführungsform lässt sich dann auch als Lackierverfahren ansehen.

Die Erfindung betrifft ebenfalls die Verwendung eines radikalisch vernetzbaren Harzes, welches eine Viskosität (23 °C, DIN EN ISO 2884-1) von ≥ 5 mPas bis ≤ 1000000 mPas aufweist, in einem additiven Fertigungsverfahren, wobei das Harz eine härtbare Verbindung umfasst, welche Isocyanuratgruppen, NCO-Gruppen und olefinische C=C-Doppelbindungen aufweist, wobei das molare Verhältnis von NCO-Gruppen und olefinischen C=C-Doppelbindungen in einem Bereich von ≥ 1:20 bis ≤ 10:1 (bevorzugt ≥ 1:10 bis ≤ 5:1, mehr bevorzugt ≥ 1:5 bis ≤ 3:1) liegt; das molare Verhältnis von NCO-Gruppen zu Isocyanurat-Gruppen in einem Bereich von ≤ 100:1 bis ≥ 1:2 (bevorzugt ≤ 70:1 bis ≥ 1:1, mehr bevorzugt ≤ 50:1 bis ≥ 2:1) liegt und das molare Verhältnis von olefinischen C=C-Doppelbindungen zu Isocyanurat-Gruppen in einem Bereich von ≤ 100:1 bis ≥ 1:5 (bevorzugt ≤ 70:1 bis ≥ 1:3, mehr bevorzugt ≤ 50:1 bis ≥ 1:2) bis ≤ 1:20) liegt.

In einer bevorzugten Ausführungsform der Verwendung umfasst das Harz weiterhin einen Radikalstarter und/oder einen Isocyanat-Trimerisierungskatalysator. Vorzugsweise ist der Radikalstarter ausgewählt aus der Gruppe: α-Hydroxyphenylketon, Benzyldimethylketal und/oder 2,4,6-Trimethylbenzoyldiphenylphosphinoxid, Bis-(4-methoxybenzoyl)diethylgerman, und/oder der Isocyanurat-Trimerisierungskatalysator ist ausgewählt ist aus: Kaliumacetat, Kaliumacetat in Kombination mit einem Kronenether, Kaliumacetat in Kombination mit einem Polyethylenglykol, Kaliumacetat in Kombination mit einem Polypropylenglykol, Zinnoctoat, Natriumphenolat, Kaliumhydroxid, Trioctylphosphin und/oder Tributylzinnoxid.

Hinsichtlich der härtbaren Verbindung gelten bei der erfindungsgemäßen Verwendung die gleichen Überlegungen und bevorzugten Ausführungsformen wie zuvor in Hinblick auf das erfindungsgemäße Verfahren. Zur Vermeidung unnötiger Wiederholungen werden sie nicht erneut wiedergegeben. Es sei lediglich angemerkt, dass in einer weiteren bevorzugten Ausführungsform in der härtbaren Verbindung die olefinischen Doppelbindungen zumindest teilweise in Form von (Meth)Acrylatgruppen vorliegen und dass in einer weiteren bevorzugten Ausführungsform die härtbare Verbindung aus der Reaktion eines NCO-terminierten Polyisocyanurats mit einem, bezogen auf die freien NCO-Gruppen, molaren Unterschuss eines Hydroxyalkyl(meth)acrylats erhältlich ist.

In einer weiteren bevorzugten Ausführungsform der Verwendung umfasst das additive Fertigungsverfahren das Belichten und/oder Bestrahlen eines zuvor ausgewählten Bereichs des radikalisch vernetzbaren Harzes. Das additive Fertigungsverfahren kann beispielsweise ein Stereolithographie- oder ein DLP (digital light processing)-Verfahren sein. Unter "Belichten" wird hierbei die Einwirkung von Licht im Bereich zwischen nahem IR- und nahem UV-Licht (1400 nm bis 315 nm Wellenlänge) verstanden. Die übrigen kürzeren Wellenlängenbereiche werden durch den Begriff "Bestrahlen" abgedeckt, zum Beispiel fernes UV-Licht, Röntgenstrahlung, Gammastrahlung und auch Elektronenstrahlung.

Ein weiterer Gegenstand der Erfindung ist ein Polymer, erhältlich durch die Vernetzung eines Harzes, welches eine Viskosität (23 °C, DIN EN ISO 2884-1) von ≥ 5 mPas bis ≤ 1000000 mPas aufweist, wobei das Harz eine härtbare Komponente umfasst, welche NCO-Gruppen und olefinische C=C-Doppelbindungen aufweist, wobei das molare Verhältnis von NCO-Gruppen und olefinischen C=C-Doppelbindungen in einem Bereich von ≥ 1:20 bis ≤ 10:1 (bevorzugt ≥ 1:10 bis ≤ 5:1, mehr bevorzugt ≥ 1:5 bis ≤ 3:1) liegt.

Vorzugsweise handelt es sich bei dem Polymer um ein Polymer, erhältlich durch die Vernetzung eines Harzes, welches eine Viskosität (23 °C, DIN EN ISO 2884-1) von ≥ 5 mPas bis ≤ 100000 mPas aufweist, wobei das Harz eine härtbare Komponente umfasst, welche Isocyanurat, NCO-Gruppen und olefinische C=C-Doppelbindungen aufweist, wobei das molare Verhältnis von NCO-Gruppen und olefinischen C=C-Doppelbindungen in einem Bereich von ≥ 1:20 bis ≤ 10:1 (bevorzugt ≥ 1:10 bis ≤ 5:1, mehr bevorzugt ≥ 1:5 bis ≤ 3:1) liegt., das molare Verhältnis von NCO-Gruppen zu Isocyanurat-Gruppen in einem Bereich von ≤ 100:1 bis ≥ 1:2 (bevorzugt ≤ 70:1 bis ≥ 1:1, mehr bevorzugt ≤ 50:1 bis ≥ 2:1) liegt und das molare Verhältnis von olefinischen C=C-Doppelbindungen zu Isocyanurat-Gruppen in einem Bereich von ≤ 100:1 bis ≥ 1:5 (bevorzugt ≤ 70:1 bis ≥ 1:3, mehr bevorzugt ≤ 50:1 bis ≥ 1:2) bis ≤ 1:20) liegt.

Die Vernetzung kann insbesondere durch ein zweistufiges Verfahren aus radikalischer Vernetzung der C=C-Doppelbindungen, gefolgt von Trimerisierung (einschließlich der Trimerisierungs-Nebenreaktionen) der NCO-Gruppen zu Isocyanuratgruppen, erfolgen.

Hinsichtlich der härtbaren Verbindung gelten bei der erfindungsgemäßen Verwendung die gleichen Überlegungen und bevorzugten Ausführungsformen wie zuvor in Hinblick auf das erfindungsgemäße Verfahren. Zur Vermeidung unnötiger Wiederholungen werden sie nicht erneut wiedergegeben. Es sei lediglich angemerkt, dass in einer bevorzugten Ausführungsform in der härtbaren Verbindung die olefinischen Doppelbindungen zumindest teilweise in Form von (Meth)Acrylatgruppen vorliegen und dass in einer weiteren bevorzugten Ausführungsform die härtbare Verbindung aus der Reaktion eines NCO-terminierten Polyisocyanurats mit einem, bezogen auf die freien NCO-Gruppen, molaren Unterschuss eines Hydroxyalkyl(meth)acrylats erhältlich ist.

Die erfindungsgemäßen Zahnschienen können auch über ein "Dual Cure" Verfahren hergestellt werden. Beschichtungsmittel, die durch zwei unabhängige Prozesse aushärten, werden allgemein als Dual Cure-Systeme bezeichnet. Üblicherweise besitzen die enthaltenen Bindemittelkomponenten dabei unterschiedliche funktionelle Gruppen die unter geeigneten Bedingungen in der Regel unabhängig voneinander miteinander vernetzen. Übliche, zum Stand der Technik gehörende Dual Cure-Systeme besitzen strahlen- sowie thermisch härtbare Gruppen, wobei besonders vorteilhafte Eigenschaften bei Verwendung von Isocyanat- und Hydroxygruppen als thermisch vernetzende Funktionen erhalten werden. Nachteilig an solchen Lösungen ist jedoch, dass die Reaktivität der NCO Gruppen und/oder die Anwesenheit von Katalysatoren für den zweiten Härtungsmechnismus die Topfzeit des Beschichtungsmittels begrenzt.

Eine Klasse von Dual Cure-Systemen enthält blockierte Isocyanate. Nach einer Deblockierung bei geeigneter Temperatur stehen die NCO-Gruppen für Reaktionen mit Polyolen zur Verfügung. Nachteilig bei der Verwendung von blockierten Isocyanaten sind die für blockierte Isocyanate typische hohe Viskosität und die üblicherweise sehr hohe Deblockierungstemperatur.

Dual Cure-Systeme können in Beschichtungsanwendungen und bei Verwendung als Klebstoffe Vorteile bei der sogenannten Schattenhärtung aufweisen. Hierunter ist derjenige Härtungsmechanismus zu verstehen, welcher nicht photochemisch, sondern beispielsweise thermisch abläuft. Dann kann das Beschichtungs- oder Klebmittel auch bei komplex geformten Substraten mit Abschattungen gegenüber einer Belichtungslampe weiter aushärten.

Im Lack- und Klebstoffbereich existieren mehrere Hauptgruppen der Dual Cure-Technologie: zwei verschiedene Radikalstarter (UV und thermisch), UV- und Feuchtigkeitsnachhärtung, UV- und PUR-2K-Härtung und eine kationisch katalysierte UV- und thermisch Härtung. Von der Firma Berlac AG wird beispielsweise unter der Bezeichnung Berlac 082.907 ein Dual Cure-Lacksystem angeboten, in dem zunächst eine Reaktion zwischen NCO-Gruppen und OH-Gruppen ausgelöst wird und dann einer UV-Härtung unterworfen wird.

Eine wichtige Anwendung von Dual Cure-Systemen liegt in additiven Fertigungsverfahren ("3D-Druck"). Als additive Fertigungsverfahren werden solche Verfahren bezeichnet, mit denen Gegenstände schichtweise aufgebaut werden. Sie unterscheiden sich daher deutlich von anderen Verfahren zur Herstellung von Gegenständen wie Fräsen, Bohren oder Zerspanen. Bei letztgenannten Verfahren wird ein Gegenstand so bearbeitet, dass er durch Wegnahme von Material seine Endgeometrie erhält.

Additive Fertigungsverfahren nutzen unterschiedliche Materialien und Prozesstechniken, um Gegenstände schichtweise aufzubauen. Eine Gruppe von additiven Fertigungsverfahren setzt radikalisch vernetzbare Harze ein, welche gegebenenfalls über einen zweiten Härtungsmechanismus ihre Endfestigkeit im gebildeten Gegenstand erhalten. Beispiele für solche Verfahren sind Stereolithographieverfahren und das davon abgeleitete sogenannte DLP-Verfahren.

Für den 3D-Druck bedeuten die Nachteile von konventionellen Dual Cure-Systemen hinsichtlich der Topfzeit, dass ein nicht gebrauchtes Aufbaumaterial schlecht wiederverwendet werden kann bzw. die geplanten Bauzeiten für ein Produkt die Topfzeit nicht überschreiten dürfen.

DE 10 2009 051445 A1 offenbart Polyisocyanat-Polyadditionsprodukte erhältlich aus

a) mindestens einem aliphatischen, cycloaliphatischen, araliphatischen und/oder aromatischen Polyisocyanat

b) mindestens einer NCO-reaktiven Verbindung

c) mindestens einem thermolatenten anorganischen Zinn-enthaltenden Katalysator

d) gegebenenfalls weiteren von c) verschiedenen Katalysatoren und/oder Aktivatoren

e) gegebenenfalls Füllstoffen, Pigmenten, Additiven, Verdickern, Entschäumern und/oder anderen Hilfs- und Zusatzstoffen,

wobei das Verhältnis des Gewichts des Zinns aus Komponente c) und des Gewichts der Komponente a) weniger als 3000 ppm beträgt, wenn Komponente a) ein aliphatisches Polyisocyanat ist und weniger als 95 ppm beträgt, wenn Komponente a) ein aromatisches Polyisocyanat ist und wobei als thermolatente Katalysatoren die folgenden zyklischen Zinnverbindungen eingesetzt werden:

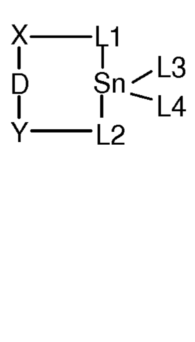
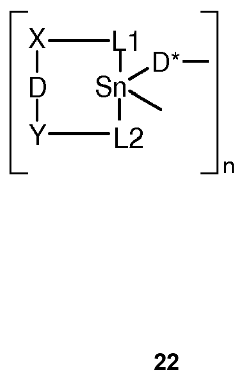
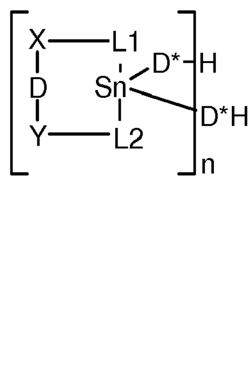

Ein Verfahren zur Herstellung eines Gegenstands aus einem Aufbaumaterial, wobei das Aufbaumaterial radikalisch vernetzbare Gruppen, NCO-Gruppen sowie Gruppen mit Zerewitinoff-aktiven H-Atomen umfasst und der Gegenstand ein dreidimensionaler Gegenstand und/oder eine Schicht ist, zeichnet sich dadurch aus, dass während und/oder nach der Herstellung des Gegenstands das Aufbaumaterial auf eine Temperatur von ≥ 50 °C erwärmt wird und dass das Aufbaumaterial eine oder mehrere zyklische Zinnverbindungen der Formel F-I, F-II und/oder F-III umfasst:

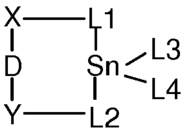
(F-I)

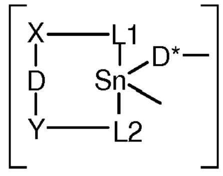
(F-II), mit n > 1

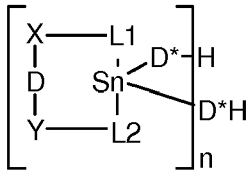
(F-III), mit n > 1

wobei gilt:

D steht für -O-, -S- oder -N(R1)-

wobei R1 für einen gesättigten oder ungesättigten, linearen oder verzweigten, aliphatischen oder cycloaliphatischen oder einen gegebenenfalls substituierten, aromatischen oder araliphatischen Rest mit bis zu 20 Kohlenstoffatomen steht, der gegebenenfalls Heteroatome aus der Reihe Sauerstoff, Schwefel, Stickstoff enthalten kann, oder für Wasserstoff oder den Rest

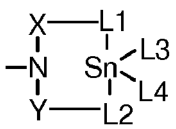

steht,

oder R1 und L3 zusammen für -Z-L5- stehen;

D* steht für -O- oder -S-;

X, Y und Z stehen für gleiche oder unterschiedliche Reste ausgewählt aus Alkylenresten der Formeln C(R2)(R3)-, -C(R2)(R3)-C(R4)(R5)- oder -C(R2)(R3)-C(R4)(R5)C(R6)(R7)- oder ortho-Arylenresten der Formeln

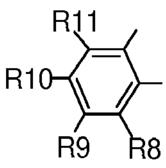
oder
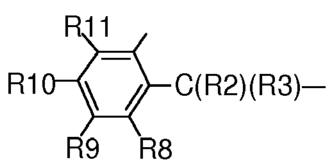
,

wobei R2 bis R11 unabhängig voneinander für gesättigte oder ungesättigte, lineare oder verzweigte, aliphatische oder cycloaliphatische oder gegebenenfalls substituierte, aromatische oder araliphatische Reste mit bis zu 20 Kohlenstoffatomen stehen, die gegebenenfalls Heteroatome aus der Reihe Sauerstoff, Schwefel, Stickstoff enthalten können, oder für Wasserstoff stehen; L1, L2 und L5 stehen unabhängig voneinander für -O-, -S-, -OC(=O)-, -OC(=S)-, SC(=O)-, -SC(=S)-, $OS(=O)_2O$-, $-OS(=O)_2$- oder -N(R12)-, wobei R12 für einen gesättigten oder ungesättigten, linearen oder verzweigten, aliphatischen oder cycloaliphatischen oder einen gegebenenfalls substituierten, aromatischen oder araliphatischen Rest mit bis zu 20 Kohlenstoffatomen steht, der gegebenenfalls Heteroatome aus der Reihe Sauerstoff, Schwefel, Stickstoff enthalten kann, oder für Wasserstoff steht; L3 und L4 stehen unabhängig voneinander für -OH, -SH, -OR13, -Hal, $-OC(=O)R_{14}$, SR15, -OC(=S)R16, $-OS(=O)_2OR17$, $-OS(=O)_2R18$ oder -NR19R20, oder L3 und L4 zusammen stehen für -L1-X-D-Y-L2-, wobei für R13 bis R20 unabhängig voneinander für gesättigte oder ungesättigte, lineare oder verzweigte, aliphatische oder cycloaliphatische oder gegebenenfalls

substituierte, aromatische oder araliphatische Reste mit bis zu 20 Kohlenstoffatomen stehen, die gegebenenfalls Heteroatome aus der Reihe Sauerstoff, Schwefel, Stickstoff enthalten können, oder für Wasserstoff stehen.

Durch das erfindungsgemäße Verfahren gebildete dreidimensionale Gegenstände können in Aufbaurichtung deren Herstellungsverfahren wenigstens abschnittsweise eine Höhe von ≥ 1 mm aufweisen. So erhaltene Beschichtungen und Klebeverbindungen können Dicken von ≥ 5 μm bis ≤ 800 μm aufweisen und so erhaltene Filme Dicken von ≥ 30 μm bis ≤ 500 μm.

Die Zinnverbindungen der Formeln F-I, F-II und F-III sind thermisch labil. Unterhalb einer bestimmten Temperatur weisen sie keine technisch sinnvolle katalytische Aktivität für die Reaktion von NCO-Gruppen mit funktionellen Gruppen, welche Zerewitinoff-aktive H-Atome tragen, auf. Insbesondere seien hierbei Urethanisierungen und Harnstoffbildungen zu nennen. Oberhalb einer bestimmten Temperatur steigt jedoch die katalytische Aktivität stark an. Ohne auf eine Theorie beschränkt zu sein wird angenommen, dass dann die Liganden Sn-Zentrum ganz oder teilweise dissoziieren und daher das Sn-Zentrum als Katalysator zur Verfügung steht. Insofern lässt sich von thermisch latenten Katalysatoren sprechen. Dadurch, dass die im Aufbaumaterial vorliegenden NCO-Gruppen unterhalb dieser Temperatur nicht abreagieren, kann das Aufbaumaterial auch leicht wiederverwertet werden. Erfindungsgemäß wird zur Aktivierung des Sn-Katalysators auf eine Temperatur von ≥ 50 °C, vorzugsweise ≥ 65 °C, mehr bevorzugt ≥ 80 °C, besonders bevorzugt ≥ 80 °C bis ≤ 200 °C erwärmt, so dass nach erfolgter Reaktion der NCO-Gruppen der Gegenstand erhalten wird. Das Erwärmen kann für eine Zeitspanne von ≥ 1 Minute, bevorzugt ≥ 5 Minuten, mehr bevorzugt ≥ 10 Minuten bis ≤ 24 Stunden bevorzugt ≤ 8 Stunden, besonders bevorzugt < 4 Stunden, erfolgen.

Vorzugsweise ist die katalytische Aktivität des thermolatenten Katalysators in dem Aufbaumaterial für das erfindungsgemäße Verfahren so gestaltet, das das Aufbaumaterial eine Topfzeit (definiert als die Zeit, in der sich die Viskosität des Materials verdoppelt) bei 23°C von > 1 h, bevorzugt > 2 h, besonders bevorzugt > 4 h und ganz besonders bevorzugt > 6 h aufweist.

In den Fällen, in denen die Zinnverbindungen der Formeln F-I, F-II und/oder F-III Liganden mit freien OH- und/oder NH-Resten aufweisen, kann der Katalysator bei der Polyisocyanat-Polyadditionsreaktion in das Produkt eingebaut werden. Besonderer Vorteil dieser einbaubaren Katalysatoren ist ihr stark reduziertes Fogging-Verhalten.

Die verschiedenen Herstellungsmethoden für die erfindungsgemäß zu verwendenden Zinn(IV)-Verbindungen bzw. ihrer Zinn(II)-Precursoren sind u.a. beschrieben in: J. Organomet. Chem. 2009 694 3184-3189, Chem. Heterocycl. Comp. 2007 43 813-834, Indian J. Chem. 1967 5 643-645 sowie in darin angeführter Literatur.

Der Gehalt der Zinnverbindungen der Formeln F-I, F-II und/oder F-III im Aufbaumaterial kann vom Typ der dem Aufbaumaterial zugrundeliegenden Isocyanate abhängig gemacht werden. So kann, wenn an ein aromatisches C-Atom gebundene NCO-Gruppen dominieren, der Gehalt ≤ 100 ppm, bezogen auf das Gesamtgewicht des Aufbaumaterials, betragen. Wenn an ein aliphatisches C-Atom gebundene NCO-Gruppen dominieren, der Gehalt ≤ 3000 ppm, bezogen auf das Gesamtgewicht des Aufbaumaterials, betragen.

Als Quelle von NCO-Gruppen im Aufbaumaterial eignen sich die dem Fachmann an sich bekannten organischen aliphatischen, cycloaliphatischen, araliphatischen und/oder aromatischen Polyisocyanate mit mindestens zwei Isocyanatgruppen pro Molekül sowie Gemische davon. Beispielsweise können NCO-terminierte Prepolymere eingesetzt werden.

Als NCO-reaktive Verbindungen mit Zerewitinoff-aktiven H-Atomen können alle dem Fachmann bekannten Verbindungen eingesetzt werden, welche eine mittlere OH- bzw. NH-Funktionalität von mindestens 1,5 aufweisen. Dies können beispielsweise niedermolekulare Diole (z. B. 1,2-Ethandiol, 1,3- bzw. 1,2-Propandiol, 1,4-Butandiol, 1,5-Petandiol, 1,6-Hexandiol), Triole (z. B. Glycerin, Trimethylolpropan) und Tetraole (z. B. Pentaerythrit) sein, kurzkettige Aminoalkohole, Polyamine aber auch höhermolekulare Polyhydroxyverbindungen wie Polyetherpolyole, Polyesterpolyole, Polycarbonatpolyole, Polysiloxanpolyole, Polyamine und Polyetherpolyamine sowie Polybutadienpolyole.

Das Aufbaumaterial kann radikalisch vernetzbare Gruppen, vorzugsweise (Meth)Acrylatgruppen, umfassen. Sie können durch thermische und/oder durch photochemische Radikalstarter eine Vernetzungsreaktion untereinander eingehen. Daher lässt sich das Aufbaumaterial auch als radikalisch vernetzbares Aufbaumaterial oder radikalisch vernetzbares Harz beschreiben. Ferner handelt es sich gemäß der obigen Definition um ein Dual Cure-System.

Vorzugsweise umfasst das radikalisch vernetzbare Aufbaumaterial eine Verbindung, die aus der Reaktion eines NCO-terminierten Polyisocyanatprepolymers mit einem, bezogen auf die freien NCO-Gruppen, molaren Unterschuss eines Hydroxyalkyl(meth)acrylats erhältlich ist.

Ebenfalls vorzugsweise umfasst das radikalisch vernetzbare Aufbaumaterial eine Verbindung, die aus der Reaktion eines NCO-terminierten Polyisocyanurats mit einem, bezogen auf die freien NCO-Gruppen, molaren Unterschuss eines Hydroxyalkyl(meth)acrylats erhältlich ist.

Geeignete Polyisocyanate zur Herstellung der NCO-terminierten Polyisocyanurate und Prepolymere sind beispielsweise solche, die ein Molekulargewicht im Bereich von 140 bis 400 g/mol aufweisen, mit aliphatisch, cycloaliphatisch, araliphatisch und/oder aromatisch gebundenen Isocyanatgruppen, wie z. B. 1,4-Diisocyanatobutan (BDI), 1,5-Diisocyanatopentan (PDI), 1,6-Diisocyanatohexan (HDI), 2-Methyl-1,5-diisocyanatopentan, 1,5-Diisocyanato-2,2-dimethylpentan, 2,2,4- bzw. 2,4,4-Trimethyl-1,6-diisocyanatohexan, 1,10-Diisocyanatodecan, 1,3- und 1,4-Diisocyanatocyclohexan, 1,4-Diisocyanato-3,3,5-trimethylcyclohexan, 1,3-Diisocyanato-2-methylcyclohexan, 1,3-Diisocyanato-4-methylcyclohexan, 1-Isocyanato-3,3,5-trimethyl-5-isocyanatomethyl-cyclohexan (Isophorondiisocyanat; IPDI), 1-Isocyanato-1-methyl-4(3)-isocyanatomethylcyclohexan, 2,4'- und 4,4'-Diisocyanatodicyclohexylmethan ($H_{12}$MDI), 1,3- und 1,4-Bis(isocyanatomethyl)cyclohexan, Bis-(isocyanatomethyl)-norbornan (NBDI), 4,4'-Diisocyanato-3,3'-dimethyldicyclohexylmethan, 4,4'-Diisocyanato-3,3',5,5'-tetramethyldicyclohexylmethan, 4,4'-Diisocyanato-1,1'-bi(cyclohexyl), 4,4'-Diisocyanato-3,3'-dimethyl-1,1'-bi(cyclohexyl), 4,4'-Diisocyanato-2,2',5,5'-tetra-methyl-1,1'-bi(cyclohexyl), 1,8-Diisocyanato-p-menthan, 1,3-Diisocyanato-adamantan, 1,3-Dimethyl-5,7-diisocyanatoadamantan, 1,3- und 1,4-Bis-(isocyanatomethyl)benzol (Xyxlylendiisocyanat; XDI), 1,3- und 1,4-Bis(1-isocyanato-l-methylethyl)-benzol (TMXDI) und Bis(4-(1-isocyanato-1-methylethyl)phenyl)-carbonat, 2,4- und 2,6-Diisocyanatotoluol (TDI), 2,4'- und 4,4'-Diisocyanatodiphenylmethan (MDI), 1,5-Diisocyanatonaphthalin sowie beliebige Gemische solcher Diisocyanate.

Ferner können erfindungsgemäß auch aliphatische und/oder aromatische Isocyanat-Endgruppen tragende Prepolymere, wie beispielsweise aliphatische oder aromatische Isocyanat-Endgruppentragende Polyether-, Polyester-, Polyacrylat, Polyepoxyd oder Polycarbonat-Prepolymere als Edukte der Isocyanurat-Bildung eingesetzt werden. Geeignete Trimerisierungskatalysatoren werden weiter unten im Zusammenhang mit einer anderen Ausführungsform beschrieben.

Geeignete Hydroxyalkyl(meth)acrylate sind unter anderem Alkoxyalkyl(meth)acrylate mit 2 bis 12 Kohlenstoffatomen im Hydroxyalkylrest. Bevorzugt sind 2-Hydroxyethylacrylat, das bei der Anlagerung von Propylenoxid an Acrylsäure entstehende Isomerengemisch oder 4-Hydroxybutylacrylat.

Die Reaktion zwischen dem Hydroxyalkyl(meth)acrylat und dem NCO-terminierten Polyisocyanurat kann durch die üblichen Urethanisierungskatalysatoren wie DBTL katalysiert werden. Bei dieser Reaktion kann das molare Verhältnis zwischen NCO-Gruppen und OH-Gruppen des Hydroxyalkyl(meth)acrylats in einem Bereich von ≥10:1 bis ≤ 1,1:1 (bevorzugt ≥ 5:1 bis ≤ 1,5:1, mehr bevorzugt ≥ 4:1 bis ≤ 2:1) liegen. Die erhaltene härtbare Verbindung kann ein zahlenmittleres Molekulargewicht $M_n$ von ≥ 200 g/mol bis ≤ 5000 g/mol aufweisen. Vorzugsweise beträgt dieses Molekulargewicht ≥ 300 g/mol bis ≤ 4000 g/mol, mehr bevorzugt ≥ 400 g/mol bis ≤ 3000 g/mol.

Besonders bevorzugt ist eine härtbare Verbindung, die aus der Reaktion eines NCO-terminierten Polyisocyanurats mit Hydroxethyl(meth)acrylat erhalten wurde, wobei das NCO-terminierte Polyisocyanurat aus 1,6-Hexamethylendiisocyanat in Gegenwart eines Isocyanat-Trimerisierungskatalysators erhalten wurde. Diese härtbare Verbindung hat ein zahlenmittleres Molekulargewicht $M_n$ von ≥ 400 g/mol bis ≤ 3000 g/mol und ein molares Verhältnis von NCO-Gruppen und olefinischen C=C-Doppelbindungen in einem Bereich von ≥ 1:5 bis ≤ 5:1. besonders bevorzugt ≥1:3 bis ≤ 3:1, ganz besonders bevorzugt. ≥1:2 bis ≤ 2:1.

Das radikalisch vernetzbare Aufbaumaterial kann weiterhin Reaktivverdünner enthalten, besonders zu nennen sei hier die Gruppe von Verbindungen auf Basis der Umsetzung von Diolen, Triolen oder Polyolen mit (Meth)Acrylsäure. Typische Vertreter sind Polyesteracrylate (Alkohol ein Polyester), Polyetheracrylate (Alkohol ein Polyether), Polyurethanacrylate (Alkohol ein Polyurethan), Polycarbonatacrylate (Alkohol ein Polycarbonat).

Das radikalisch vernetzbare Aufbaumaterial kann weiterhin Additive wie Füllstoffe, UV-Stabilisatoren, Radikalinhibitoren, Antioxidantien, Formtrennmittel, Wasserfänger, Slipadditive, Entschäumer, Verlaufsmittel, Rheologieadditive, Flammschutzmittel und/oder Pigmente enthalten. Diese Hilfs- und Zusatzmittel, ausgenommen Füllstoffe und Flammschutzmittel, liegen üblicherweise in einer Menge von weniger als 50 Gew.-%, vorzugsweise weniger als 30 Gew.-%, besonders bevorzugt bis zu 20 Gew.-%, besonders bevorzugt bis zu 10 Gew.-%, bezogen auf das radikalisch vernetzbare Harz vor. Flammschutzmittel liegen üblicherweise in Mengen von höchstens 70 Gew.-%, vorzugsweise höchstens 50 Gew.-%, besonders bevorzugt höchstens 30 Gew.-%, berechnet als Gesamtmenge an eingesetzten Flammschutzmitteln bezogen auf das Gesamtgewicht des radikalisch vernetzbaren Aufbaumaterials vor.

Geeignete Füllstoffe sind beispielsweise $AlOH_3$, $CaCO_3$, geschnittene Glasfasern, Karbonfasern, Polymerfasern, Metallpigmente wie $TiO_2$ und weitere bekannte übliche Füllstoffe. Diese Füllstoffe werden vorzugsweise in Mengen von höchstens 70 Gew.-%, bevorzugt höchstens 50 Gew.-%, besonders bevorzugt höchstens 30 Gew.-%, berechnet als Gesamtmenge an eingesetzten Füllstoffen bezogen auf das Gesamtgewicht des radikalisch vernetzbaren Harzes, eingesetzt.

Geeignete UV-Stabilisatoren können vorzugsweise ausgewählt werden aus der Gruppe, bestehend aus Piperidinderivaten, wie z.B. 4-Benzoyloxy-2,2,6,6-tetramethylpiperidin, 4-Benzoyloxy-1,2,2,6,6-pentamethylpiperidin, Bis-(2,2,6,6-tetra-methyl-4-piperidyl)-sebacat, Bis(1,2,2,6,6-pentamethyl-1-4-piperidinyl)-sebacat, Bis-(2,2,6,6-tetramethyl-4-piperidyl)-suberat, Bis-(2,2,6,6-tetramethyl-4-piperidyl)-dodecandioat; Benzophenonderivaten, wie z.B. 2,4-Dihydroxy-, 2-Hydroxy-4-methoxy-, 2-Hydroxy-4-octoxy-, 2-Hydroxy-4-dodecyloxy- oder 2,2'-Dihydroxy-4-dodecyloxy-benzophenon; Benztriazolderivaten, wie z.B. 2-(2H-Benzotriazol-2-yl)-4,6-di-tert-pentylphenol, 2-(2H-Benzotriazol-2-yl)-6-dodecyl-4-methylphenol, 2-(2H-Benzotriazol-2-yl)-4,6-bis(1-methyl-1-phenylethyl)phenol, 2-(5-Chlor-2H-benzotriazol-2-yl)-6-(1,1-dimethylethyl)-4-methylphenol, 2-(2H-benzotriazol-2-yl)-4-(1,1,3,3-tetramethylbutyl)phenol, 2-(2H-Benzotriazol-2-yl)-6-(1-methyl-1-phenylethyl)-4-(1,1,3,3-tetramethylbutyl)phenol, Isooctyl-3-(3-(2H-benzotriazol-2-yl)-5-(1,1-dimethylethyl)-4-hydroxyphenylpropionat), 2-(2H-Benzotriazol-2-yl)-4,6-bis(1,1-dimethylethyl)phenol, 2-(2H-Benzotriazol-2-yl)-4,6-bis(1-methyl-1-phenylethyl)phenol, 2-(5-Chlor-2H-benzotriazol-2-yl)-4,6-bis(1,1-dimethylethyl)phenol; Oxalaniliden, wie z.B. 2-Ethyl-2'-ethoxy- oder 4-Methyl-4'-methoxyoxalanilid; Salicylsäureestern, wie z.B. Salicylsäurephenylester, Salicylsäure-4-tert-butylphenylester, Salicylsäure-4-tert-octylphenylester; Zimtsäureesterderivaten, wie z.B. α-Cyano-β-methyl-4-methoxyzimtsäuremethylester, α-Cyano-β-methyl-4-methoxyzimtsäurebutyl-ester, α-Cyano-β-phenylzimtsäureethylester, α-Cyano-β-phenylzimtsäureisooctylester; und Malonesterderivaten, wie z.B. 4-Methoxybenzylidenmalonsäuredimethylester, 4-Methoxybenzylidenmalonsäurediethylester, 4-Butoxybenzylidenmalonsäuredimethylester. Diese bevorzugten Lichtstabilisatoren können sowohl einzeln als auch in beliebigen Kombinationen untereinander zum Einsatz kommen.

Besonders bevorzugte UV-Stabilisatoren sind solche, die Strahlung einer Wellenlänge < 400 nm vollständig absorbieren. Hierzu zählen beispielsweise die genannten Benztriazolderivate. Ganz besonders bevorzugte UV-Stabilisatoren sind 2-(5-Chlor-2H-benzotriazol-2-yl)-6-(1,1-dimethylethyl)-4-methylphenol, 2-(2H-benzotriazol-2-yl)-4-(1,1,3,3-tetramethylbutyl)phenol und/oder 2-(5-Chlor-2H-benzotriazol-2-yl)-4,6-bis(1,1-dimethylethyl)phenol.

Gegebenenfalls werden ein oder mehrere der beispielhaft genannten UV-Stabilisatoren dem radikalisch vernetzbaren Aufbaumaterial vorzugsweise in Mengen von 0,001 bis 3,0 Gew.-%, besonders bevorzugt 0,005 bis 2 Gew.-%, berechnet als Gesamtmenge an eingesetzten UV-Stabilisatoren bezogen auf das Gesamtgewicht des radikalisch vernetzbaren Aufbaumaterials, zugesetzt.

Geeignete Antioxidantien sind vorzugsweise sterisch gehinderten Phenole, welche vorzugsweise ausgewählt werden können aus der Gruppe, bestehend aus 2,6-Di-tert-butyl-4-methylphenol (Ionol), Pentaerythrit-tetrakis(3-(3,5-di-tert-butyl-4-hydroxy-phenyl)-propionat), Octadecyl-3-(3,5-di-tert-butyl-4-hydroxyphenyl)-propionat, Triethylen-glykol-bis(3-tert-butyl-4-hydroxy-5-methylphenyl)propionat, 2,2'-Thio-bis(4-methyl-6-tert-butylphenol) und 2,2'-Thiodiethyl-bis[3-(3,5-di-tert-butyl-4-hydroxyphenyl)propionat]. Diese können bei Bedarf sowohl einzeln als auch in beliebigen Kombinationen untereinander eingesetzt werden. Diese Antioxidantien werden vorzugsweise in Mengen von 0,01 bis 3,0 Gew.-%, besonders bevorzugt 0,02 bis 2,0 Gew.-%, berechnet als Gesamtmenge an eingesetzten Antioxidantien bezogen auf das Gesamtgewicht des radikalisch vernetzbaren Aufbaumaterials, eingesetzt.

Geeignete Radikalinhibitoren bzw. Verzögerer sind besonders solche, die eine unkontrollierte radikalische Polymerisation der Harzformulierung außerhalb des gewünschten (bestrahlten) Bereiches gezielt inhibieren. Diese sind für eine gute Randschärfe und Abbildungsgenauigkeit im Vorläufer entscheidend. Geeignete Radikalinhibitoren müssen entsprechend der gewünschten Radikalausbeute aus dem Bestrahlungs-/Belichtungsschritt und der Polymerisationsgeschwindigkeit und Reaktivität/Selektivität der Doppelbindungsträger ausgesucht werden. Geeignete Radikalinhibitoren sind z. B. 2,2-(2,5-thiophendiyl)bis(5-tertbutylbenzoxazol), Phenothiazin, Hydrochinone, Hydrochinonether, Quinonalkyde und Nitroxylverbindungen sowie Mischungen davon, Benzoquinone, Kupfer Salze, Brenzcatechine, Cresole, Nitrobenzol und Sauerstoff. Diese Antioxidantien werden vorzugsweise in Mengen von 0,001 Gew% bis 3 Gew.% eingesetzt.

Vorzugsweise ist die molare Konzentration von Zerewitinoff-aktiven H-Atomen im Verhältnis zu freien Isocyanaten ≥ 0,6 und ≤ 1,5, bevorzugt, ≥ 0,8 und ≤ 1,4, besonders bevorzugt, ≥ 0,9 und ≤ 1,3, und ganz besonders bevorzugt ≥ 1 und ≤ 1,2.

In einer bevorzugten Ausführungsform ist der Gegenstand ein dreidimensionaler Gegenstand, der Gegenstand wird aus einem Vorläufer erhalten und das Verfahren umfasst die Schritte:

I) Abscheiden von radikalisch vernetztem Aufbaumaterial auf einem Träger, so dass eine Lage eines mit dem Träger verbundenen Aufbaumaterials erhalten wird, welche einem ersten ausgewählten Querschnitt des Vorläufers entspricht;

II) Abscheiden von radikalisch vernetztem Aufbaumaterial auf eine zuvor aufgetragene Lage des Aufbaumaterials, so dass eine weitere Lage des Aufbaumaterials erhalten wird, welche einem weiteren ausgewählten Querschnitt des Vorläufers entspricht und welche mit der zuvor aufgetragenen Lage verbunden ist;

III) Wiederholen des Schritts II), bis der Vorläufer gebildet ist;
wobei das Abscheiden von radikalisch vernetztem Aufbaumaterial wenigstens in Schritt II) durch Belichten und/oder Bestrahlen eines ausgewählten Bereichs eines radikalisch vernetzbaren Aufbaumaterials, entsprechend dem jeweils ausgewählten Querschnitt des Vorläufers, erfolgt und wobei das radikalisch vernetzbare Aufbaumaterial eine Viskosität (23 °C, DIN EN ISO 2884-1) von ≥ 5 mPas bis ≤ 1000000 mPas aufweist, wobei das radikalisch vernetzbare Aufbaumaterial eine härtbare Komponente umfasst, in der NCO-Gruppen und olefinische C=C-Doppelbindungen vorliegen und nach Schritt III) weiterhin Schritt IV) durchgeführt wird:

IV) Erwärmen des nach Schritt III) erhaltenen Vorläufers auf eine Temperatur von ≥ 50 °C, so dass der Gegenstand erhalten wird.

In dieser Variante wird somit der Gegenstand mittels eines additiven Herstellungsverfahrens und in zwei Herstellungsabschnitten erhalten. Der erste Herstellungsabschnitt kann als Aufbauabschnitt angesehen werden. Dieser Aufbauabschnitt lässt sich mittels strahlenoptischer additiver Fertigungsverfahren wie dem Inkjet-Verfahren, der Stereolithographie oder dem DLP (digital light processing)-Verfahren realisieren und ist Gegenstand der Schritte I), II) und III). Der zweite Herstellungsabschnitt kann als Härtungsabschnitt angesehen werden und ist Gegenstand des Schritts IV). Hier wird der nach dem Aufbauabschnitt erhaltene Vorläufer oder intermediäre Gegenstand ohne seine Form weiter zu verändern in einen mechanisch dauerhafteren Gegenstand überführt.

In Schritt I) dieser Variante des Verfahrens erfolgt das Abscheiden eines radikalisch vernetzten Aufbaumaterials auf einem Träger. Dieses ist gewöhnlich der erste Schritt in Inkjet-, Stereolithographie- und DLP-Verfahren. Auf diese Weise wird eine Lage eines mit dem Träger verbundenen Aufbaumaterials erhalten, welche einem ersten ausgewählten Querschnitt des Vorläufers entspricht.

Gemäß der Anweisung von Schritt III) wird Schritt II) so lange wiederholt, bis der gewünschte Vorläufer gebildet ist. In Schritt II) erfolgt das Abscheiden eines radikalisch vernetzten Aufbaumaterials auf eine zuvor aufgetragene Lage des Aufbaumaterials, so dass eine weitere Lage des Aufbaumaterials erhalten wird, welche einem weiteren ausgewählten Querschnitt des Vorläufers entspricht und welche mit der zuvor aufgetragenen Lage verbunden ist. Bei der zuvor aufgetragenen Lage des Aufbaumaterials kann es sich um die erste Lage aus Schritt I) oder um eine Lage aus einer vorigen Durchlauf des Schritts II) handeln.

Es ist in dieser Verfahrensvariante vorgesehen, dass das Abscheiden eines radikalisch vernetzten Aufbaumaterials wenigstens in Schritt II) (vorzugsweise auch in Schritt I) durch Belichten und/oder Bestrahlen eines ausgewählten Bereichs eines radikalisch vernetzbaren Harzes, entsprechend dem jeweils ausgewählten Querschnitt des Gegenstandes, erfolgt. Dieses kann sowohl durch selektives Belichten (Stereolithographie, DLP) des vernetzbaren Aufbaumaterials als auch durch selektives Auftragen des vernetzbaren Aufbaumaterials, gefolgt von einem Belichtungsschritt, der aufgrund des vorigen selektiven Auftragens des vernetzbaren Aufbaumaterials nicht mehr selektiv sein muss (Inkjet-Verfahren).

Im Kontext dieser Erfindung werden die Begriffe "radikalisch vernetzbares Aufbaumaterial" und "radikalisch vernetztes Aufbaumaterial" benutzt. Hierbei wird das radikalisch vernetzbare Aufbaumaterial durch das Belichten und/oder Bestrahlen, welches radikalische Vernetzungsreaktionen auslöst, in das radikalisch vernetzte Aufbaumaterial überführt. Unter "Belichten" wird hierbei die Einwirkung von Licht im Bereich zwischen nahem IR- und nahem UV-Licht (1400 nm bis 315 nm Wellenlänge) verstanden. Die übrigen kürzeren Wellenlängenbereiche werden durch den Begriff "Bestrahlen" abgedeckt, zum Beispiel fernes UV-Licht, Röntgenstrahlung, Gammastrahlung und auch Elektronenstrahlung.

Das Auswählen des jeweiligen Querschnitts erfolgt zweckmäßigerweise durch ein CAD-Programm, mit dem ein Modell des herzustellenden Gegenstandes erzeugt wurde. Diese Operation wird auch "Slicing" genannt, und dient als Grundlage für die Steuerung der Belichtung und/oder Bestrahlung des radikalisch vernetzbaren Harzes.

Das radikalisch vernetzbare Aufbaumaterial weist in dieser Verfahrensvariante eine Viskosität (23 °C, DIN EN ISO 2884-1) von $\geq$ 5 mPas bis $\leq$ 1000000 mPas auf. Somit ist es zumindest für die Zwecke der additiven Fertigung als flüssiges Harz anzusehen. Vorzugsweise beträgt die Viskosität $\geq$ 50 mPas bis $\leq$ 100000 mPas, mehr bevorzugt $\geq$ 500 mPas bis $\leq$ 50000 mPas.

Weiterhin umfasst in dem Verfahren das radikalisch vernetzbare Harz eine härtbare Komponente, in der NCO-Gruppen und olefinische C=C-Doppelbindungen vorliegen. In dieser härtbaren Komponente kann das molare Verhältnis von NCO-Gruppen und olefinischen C=C-Doppelbindungen in einem Bereich von $\geq$ 1:5 bis $\leq$ 5:1 (bevorzugt > 1:4 bis $\leq$ 4:1, mehr bevorzugt $\geq$ 1:3 bis $\leq$ 3:1) liegen. Das molekulare Verhältnis dieser funktionellen Gruppen lässt sich durch die Integration der Signale einer Probe im $^{13}$C-NMR-Spektrum ermitteln.

Neben der härtbaren Komponente kann das radikalisch vernetzbare Aufbaumaterial auch eine nicht härtbare Komponente umfassen, in der beispielsweise Stabilisatoren, Füllstoffe und dergleichen zusammengefasst sind. In der härtbaren Komponente können die NCO-Gruppen und die olefinischen C=C-Doppelbindungen in getrennten Molekülen und/oder in einem gemeinsamen Molekül vorliegen. Wenn NCO-Gruppen und olefinische C=C-Doppelbindungen in getrennten Molekülen vorliegen, kann der nach Schritt IV) dieser Verfahrensvariante erhaltene Körper ein interpenetrierendes Polymer-Netzwerk aufweisen

In dieser Variante des Verfahrens wird weiterhin nach Schritt III) weiterhin Schritt IV) durchgeführt. In diesem Schritt erfolgt das Erwärmen des nach Schritt III) erhaltenen Vorläufers auf eine Temperatur von $\geq$ 50 °C, vorzugsweise $\geq$ 65 °C, mehr bevorzugt $\geq$ 80 °C, besonders bevorzugt $\geq$ 80 °C bis $\leq$ 200 °C, so dass der Gegenstand erhalten wird. Das Erwärmen kann für eine Zeitspanne von $\geq$ 1 Minute, bevorzugt $\geq$ 5 Minuten, mehr bevorzugt $\geq$ 10 Minuten bis $\leq$ 24 Stunden bevorzugt $\leq$ 8 Stunden, besonders bevorzugt < 4 Stunden, erfolgen.

Vorzugsweise wird die Reaktion durchgeführt, bis $\leq$ 30 Gew.-%, bevorzugt $\leq$ 20 Gew.-% oder bevorzugt $\leq$ 15 Gew.-% der ursprünglich vorhandenen NCO-Gruppen noch vorhanden sind. Dieses lässt sich mittels quantitativer IR-Spektroskopie bestimmen.

Es ist bevorzugt, dass Schritt IV) erst dann durchgeführt wird, wenn das gesamte Aufbaumaterial des Vorläufers

seinen Gelpunkt erreicht hat. Der Gelpunkt wird als erreicht angesehen, wenn in einer dynamisch-mechanischen Analyse (DMA) mit einem Platte/Platte-Oszillationsviskosimeter gemäß ISO 6721-10 bei 20 °C sich die Graphen des Speichermoduls G' und des Verlustmoduls G" kreuzen. Gegebenenfalls wird der Vorläufer weiterer Belichtung und/oder Bestrahlung zur Vervollständigung der radikalischen Vernetzung ausgesetzt. Das radikalisch vernetzte Aufbaumaterial kann ein Speichermodul G' (DMA, Platte/Platte-Oszillationsviskosimeter gemäß ISO 6721-10 bei 20 °C und einer Scherrate von 1/s) von ≥ $10^6$ Pa aufweisen.

In einer weiteren bevorzugten Ausführungsform weist das Verfahren die Merkmale auf:

- der Träger ist innerhalb eines Behälters angeordnet und ist vertikal in Schwerkraftrichtung absenkbar,
- der Behälter enthält das radikalisch vernetzbare Aufbaumaterial in einer Menge, welche ausreicht, um wenigstens den Träger und eine in vertikaler Richtung gesehenen obersten Oberfläche von auf dem Träger abgeschiedenem vernetztem Aufbaumaterial zu bedecken,
- vor jedem Schritt II) wird der Träger um eine vorbestimmte Strecke abgesenkt, so dass über der in vertikaler Richtung gesehen obersten Lage des vernetzten Aufbaumaterials sich eine Schicht des radikalisch vernetzbaren Aufbaumaterials bildet und
- in Schritt II) belichtet und/oder bestrahlt ein Energiestrahl den ausgewählten Bereich der Schicht des radikalisch vernetzbaren Aufbaumaterials, entsprechend dem jeweils ausgewählten Querschnitt des Vorläufers.

Somit wird gemäß dieser Ausführungsform das additive Fertigungsverfahren der Stereolithographie (SLA) abgedeckt. Der Träger kann beispielsweise jeweils um eine vorbestimmte Strecke von ≥ 1 µm bis ≤ 2000 µm abgesenkt werden.

In einer weiteren bevorzugten Ausführungsform weist das Verfahren die Merkmale auf:

- der Träger ist innerhalb eines Behälters angeordnet ist und vertikal entgegen der Schwerkraftrichtung anhebbar,
- der Behälter stellt das radikalisch vernetzbare Aufbaumaterial bereit,
- vor jedem Schritt II) wird der Träger um eine vorbestimmte Strecke angehoben, so dass unter der in vertikaler Richtung gesehen untersten Lage des vernetzten Aufbaumaterials sich eine Schicht des radikalisch vernetzbaren Aufbaumaterials bildet und
- in Schritt II) belichtet und/oder bestrahlt eine Mehrzahl von Energiestrahlen den ausgewählten Bereich der Schicht des radikalisch vernetzbaren Aufbaumaterials, entsprechend dem jeweils ausgewählten Querschnitt des Vorläufers, gleichzeitig.

Somit wird gemäß dieser Ausführungsform das additive Fertigungsverfahren der DLP-Technologie abgedeckt, wenn die Mehrzahl von Energiestrahlen über ein Array von einzeln ansteuerbaren Mikrospiegeln das per Belichtung und/oder Bestrahlung bereitzustellende Bild erzeugen. Der Träger kann beispielsweise jeweils um eine vorbestimmte Strecke von ≥ 1 µm bis ≤ 2000 µm angehoben werden.

In einer weiteren bevorzugten Ausführungsform weist das Verfahren die Merkmale auf:

- in Schritt II) wird das radikalisch vernetzbare Aufbaumaterial aus einem oder mehreren Druckköpfen, entsprechend dem jeweils ausgewählten Querschnitt des Vorläufers, aufgetragen und wird anschließend belichtet und/oder bestrahlt.

Somit wird gemäß dieser Ausführungsform das additive Fertigungsverfahren der Inkjet-Methode abgedeckt: es wird das vernetzbare Aufbaumaterial gegebenenfalls separat von den erfindungsgemäßen Katalysatoren selektiv durch einen oder mehrere Druckköpfe aufgetragen und die anschließende Härtung durch Bestrahlen und/oder Belichtung kann unselektiv sein, beispielsweise durch eine UV-Lampe. Bei dem oder den Druckköpfen zum Auftragen des vernetzbaren Aufbaumaterials kann es sich um einen (modifizierten) Druckkopf für Tintenstrahldruckverfahren handeln. Der Träger kann vom Druckkopf weg bewegbar ausgestaltet sein oder der Druckkopf kann vom Träger weg bewegbar ausgestaltet sein. Die Inkremente der Abstandsbewegungen zwischen Träger und Druckkopf können beispielsweise in einem Bereich von ≥ 1 µm bis ≤ 2000 µm liegen.

In einer weiteren bevorzugten Ausführungsform ist der Gegenstand eine Beschichtung und das Verfahren umfasst mindestens die Schritte:

- Auftragen des Aufbaumaterials auf ein Substrat
- Einwirken von Wärme und/oder UV-Strahlung auf das aufgetragene Aufbaumaterial, so dass im aufgetragenen Aufbaumaterial eine zumindest teilweise Vernetzung der radikalisch vernetzbaren Gruppen erfolgt
- Erwärmen des aufgetragenen Aufbaumaterials auf eine Temperatur von ≥ 50 °C, so dass im aufgetragenen Aufbaumaterial zumindest teilweise eine Reaktion zwischen NCO-Gruppen und Gruppen mit Zerewitinoff-aktiven H-Atomen erfolgt.

Das Einwirken von Wärme kann zum Beispiel zum thermischen Zerfall von Peroxid-basierten Radikalstartern führen. Das Einwirken von UV-Strahlung erfolgt mittels UV-Licht (1400 nm bis 315 nm Wellenlänge) und aktiviert photochemische Radikalstarter. In einem weiteren Schritt wird der latente Sn-Urethanisierungskatalysator aktiviert, um den zweiten Härtungsmechanismus ablaufen zu lassen.

In einer weiteren bevorzugten Ausführungsform ist der Gegenstand eine Klebeverbindung für eine Zahnschiene und das Verfahren umfasst mindestens die Schritte:

- Auftragen des Aufbaumaterials auf ein erstes Substrat
- Kontaktieren des aufgetragenen Aufbaumaterials mit einem zweiten Substrat
- Einwirken von Wärme und/oder UV-Strahlung auf das aufgetragene Aufbaumaterial, so dass im aufgetragenen Aufbaumaterial eine zumindest teilweise Vernetzung der radikalisch vernetzbaren Gruppen erfolgt
- Erwärmen des aufgetragenen Aufbaumaterials auf eine Temperatur von ≥ 50 °C, so dass im aufgetragenen Aufbaumaterial zumindest teilweise eine Reaktion zwischen NCO-Gruppen und Gruppen mit Zerewitinoff-aktiven H-Atomen erfolgt.

Das Einwirken von Wärme kann zum Beispiel zum thermischen Zerfall von Peroxid-basierten Radikalstartern führen. Das Einwirken von UV-Strahlung erfolgt mittels UV-Licht (1400 nm bis 315 nm Wellenlänge) und aktiviert photochemische Radikalstarter. In einem weiteren Schritt wird der latente Sn-Urethanisierungskatalysator aktiviert, um den zweiten Härtungsmechanismus ablaufen zu lassen.

In einer weiteren bevorzugten Ausführungsform umfasst das Aufbaumaterial weiterhin einen Radikalstarter und/oder einen Isocyanat-Trimerisierungskatalysator. Um eine unerwünschte Erhöhung der Viskosität des radikalisch vernetzbaren Aufbaumaterials zu verhindern, können Radikalstarter und/oder Isocyanat-Trimerisierungskatalysator erst unmittelbar vor Beginn des erfindungsgemäßen Verfahrens dem Aufbaumaterial hinzugefügt werden.

Als Radikalstarter kommen thermische und/oder photochemische Radikalstarter (Photoinitiatoren) in Betracht. Es ist auch möglich, dass gleichzeitig thermische und photochemische Radikalstarter eingesetzt werden. Geeignete thermische Radikalstarter sind beispielsweise (AIBN), Dibenzoylperoxid (DBPO), Di-tert-butylperoxid, Dicumylperoxid und/oder anorganische Peroxide wie Peroxodisulfate.

Bei den Photoinitiatoren wird prinzipiell zwischen zwei Typen unterschieden, dem unimolekularen Typ (I) und dem bimolekularen Typ (II). Geeignete Typ (I)-Systeme sind aromatische Ketonverbindungen, wie z. B. Benzophenone in Kombination mit tertiären Aminen, Alkylbenzophenone, 4,4'-Bis(dimethylamino)benzophenon (Michlers Keton), Anthron und halogenierte Benzophenone oder Mischungen der genannten Typen. Weiter geeignet sind Typ (II)-Initiatoren wie Benzoin und seine Derivate, Benzilketale, Acylphosphinoxide, 2,4,6-Trimethylbenzoyldiphenylphosphinoxid, Bisacylphosphinoxide, Phenylglyoxylsäureester, Campherchinon, $\alpha$-Aminoalkylphenone, $\alpha,\alpha$-Dialkoxyacetophenone und $\alpha$-Hydroxyalkylphenone. Spezielle Beispiele sind Irgacur®500 (eine Mischung von Benzophenon und (1-Hydroxycyclohexyl)phenylketon, Fa. Ciba, Lampertheim, DE), Irgacure 819 DW (Phenylbis-(2, 4, 6-trimethylbenzoyl)phosphinoxid, Fa. Ciba, Lampertheim, DE) oder Esacure KIP EM (Oligo-[2-hydroxy-2-methyl-1-[4-(1-methylvinyl)-phenyl]-propanone], Fa. Lamberti, Aldizzate, Italien) und Bis-(4-methoxybenzoyl)diethylgerman. Es können auch Gemische dieser Verbindungen eingesetzt werden.

Bei den Photoinitiatoren sollte darauf geachtet werden, dass sie eine ausreichende Reaktivität gegenüber der verwendeten Strahlenquelle haben. Es ist eine Vielzahl von Photoinitiatoren auf dem Markt bekannt. Durch kommerziell verfügbare Photoinitiatoren wird der Wellenlängenbereich im gesamten UV-VIS Spektrum abgedeckt. Photoinitiatoren finden Einsatz bei der Herstellung von Lacken, Druckfarben und Klebstoffen sowie im Dentalbereich.

In dieser Verfahrensvariante kommt der Photoinitiator im Allgemeinen in einer auf die Menge der eingesetzten härtbaren olefinisch ungesättigte Doppelbindungen tragenden Komponente bezogenen Konzentration von 0,01 bis 6,0 Gew.-%, bevorzugt von 0,05 bis 4,0 Gew.-% und besonders bevorzugt von 0,1 bis 3,0 Gew.-% zum Einsatz.

In einer weiteren bevorzugten Ausführungsform wird das Aufbaumaterial aus der Vermischung einer NCO-Gruppen enthaltenden Komponente sowie einer Gruppe mit Zerewitinoff-aktiven H-Atomen enthaltenden Komponente erhalten und die Vermischung erfolgt ≤ 5 Minuten vor Beginn des Verfahrens. In Verfahren wie DLP-Verfahren ist es ferner bevorzugt, dass die Mischung des Aufbaumaterials kontinuierlich erzeugt wird und dem Aufbauprozess zugeführt wird. Um unerwünschte Nebenreaktionen zu vermeiden können die übrigen Bestandteile des Aufbaumaterials in der Zerewitinoff-aktive H-Atomen enthaltenden Komponente vorliegen.

In einer weiteren bevorzugten Ausführungsform ist in der Definition gemäß den vorstehenden Ausführungen D -N(R1)- und R1 ist Wasserstoff oder ein Alkyl-, Aralkyl-, Alkaryl- oder Arylrest mit bis zu 20 C-Atomen ist oder der Rest

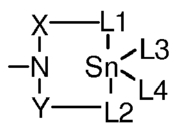

.

In einer weiteren bevorzugten Ausführungsform ist in der Definition gemäß den vorstehenden Ausführungen R1 Wasserstoff oder ein Methyl-, Ethyl-, Propyl-, Butyl-, Hexyl-, Octyl-, Ph-, oder $CH_3$Ph-Rest oder der Rest

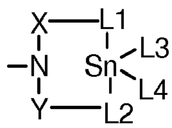

und Propyl-, Butyl-, Hexyl-, und Octyl stehen für alle isomeren Propyl-, Butyl-, Hexyl- sowie Octylreste.

In einer weiteren bevorzugten Ausführungsform ist in der Definition gemäß den vorstehenden Ausführungen D* -O-.

Weitere bevorzugte Merkmale für die Zinnverbindungen gemäß den vorstehenden Ausführungen werden nachfolgend aufgeführt:

Bevorzugt handelt es sich bei X, Y und Z um die Alkylenreste C(R2)(R3)-, C(R2)(R3), C(R4)(R5)- oder den ortho-Arylenrest

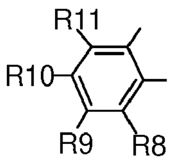

.

Bevorzugt handelt es sich bei R2 bis R7 um Wasserstoff oder Alkyl-, Aralkyl-, Alkaryl- oder Arylreste mit bis zu 20 C-Atomen, besonders bevorzugt um Wasserstoff oder Alkyl-, Aralkyl-, Alkaryl- oder Arylreste mit bis zu 8 C-Atomen, ganz besonders bevorzugt um Wasserstoff oder Alkylreste mit bis zu 8 C-Atomen, noch weiter bevorzugt um Wasserstoff oder Methyl.

Bevorzugt handelt es sich bei R8 bis R11 um Wasserstoff oder Alkylreste mit bis zu 8 C-Atomen, besonders bevorzugt um Wasserstoff oder Methyl.

Bevorzugt handelt es sich bei L1, L2 und L5 um -NR12-, -S-, -SC(=S)-, -SC(=O)-, -OC(=S)-, -O-, oder -OC(=O)-, besonders bevorzugt um -O-, oder -OC(=O)-.

Bevorzugt handelt es sich bei R12 um Wasserstoff oder einen Alkyl-, Aralkyl-, Alkaryl- oder Arylrest mit bis zu 20 C-Atomen, besonders bevorzugt um Wasserstoff oder einen Alkyl-, Aralkyl-, Alkaryl- oder Arylrest mit bis zu 12 C-Atomen, ganz besonders bevorzugt um Wasserstoff oder einen Methyl-, Ethyl-, Propyl-, Butyl-, Hexyl-oder Octyl-Rest, wobei Propyl-, Butyl-, Hexyl-, und Octyl für alle isomeren Propyl-, Butyl-, Hexyl- sowie Octylreste stehen.

Bevorzugt handelt es sich bei L3 und L4 um -Hal, -OH, -SH, -OR13, -OC(=O)R14, wobei die Reste R13 und R14 bis zu 20 Kohlenstoffatome, bevorzugt bis zu 12 Kohlenstoffatome aufweisen.

Besonders bevorzugt handelt es sich bei L3 und L4 um Cl-, MeO-, EtO-, PrO-, BuO-, HexO-, OctO-, PhO-, Formiat, Acetat, Propanoat, Butanoat, Pentanoat, Hexanoat, Octanoat, Laurat, Lactat oder Benzoat, wobei Pr, Bu, Hex und Oct für alle isomeren Propyl-, Butyl-, Hexyl- sowie Octylreste stehen, noch weiter bevorzugt um Cl-, MeO-, EtO-, PrO-, BuO-, HexO-, OctO-, PhO-, Hexanoat, Laurat, oder Benzoat, wobei Pr, Bu, Hex und Oct für alle isomeren Propyl-, Butyl-, Hexyl- sowie Octylreste stehen.

Bevorzugt handelt es bei R15 bis R20 um Wasserstoff oder Alkyl-, Aralkyl-, Alkaryl- oder Arylreste mit bis zu

20 C-Atomen, besonders bevorzugt um Wasserstoff oder Alkyl-, Aralkyl-, Alkaryl- oder Arylreste mit bis zu 12 C-Atomen, ganz besonders bevorzugt um Wasserstoff, Methyl-, Ethyl-, Propyl-, Butyl-, Hexyl-, oder Octyl-Reste, wobei Propyl-, Butyl-, Hexyl-, und Octyl für alle isomeren Propyl-, Butyl-, Hexyl- sowie Octylreste stehen.

Die Einheiten L1-X, L2-Y und L5-Z stehen bevorzugt für $-CH_2CH_2O-$, $-CH_2CH(Me)O-$, $CH(Me)CH_2O-$, $CH_2C(Me)_2O-$, $-C(Me)_2CH_2O-$ oder $-CH_2C(=O)O-$.

Die Einheit L1-X-D-Y-L2 steht bevorzugt für: $HN[CH_2CH_2O-]_2$, $HN[CH_2CH(Me)O-]_2$, $HN[CH_2CH(Me)O-][CH(Me)CH_2O-]$, $HN[CH_2C(Me)_2O-]_2$, $HN[CH_2C(Me)_2O-][C(Me)_2CH_2O-]$, $HN[CH_2C(=O)O-]_2$, $MeN[CH_2CH_2O-]_2$, $MeN[CH_2CH(Me)O-]_2$, $MeN[CH_2CH(Me)O]$ $[CH(Me)CH_2O-]$, $MeN[CH_2C(Me)_2O-]_2$, $MeN[CH_2C(Me)_2O]$ $[C(Me)_2CH_2O-]$, $MeN[CH_2C(=O)O-]_2$, $EtN[CH_2CH_2O-]_2$, $EtN[CH_2CH(Me)O-]_2$, $EtN[CH_2CH(Me)O-]$ $[CH(Me)CH_2O-]$, $EtN[CH_2C(Me)_2O-]_2$, $EtN[CH_2C(Me)_2O-][C(Me)_2CH_2O-]$, $EtN[CH_2C(=O)O-]_2$, $PrN[CH_2CH_2O]_2$, $PrN[CH_2CH(Me)O-]_2$, $PrN[CH_2CH(Me)O-][CH(Me)CH_2O-]$, $PrN[CH_2C(Me)_2O-]_2$, $PrN[CH_2C(Me)_2O-][C(Me)_2CH_2O-]$, $PrN[CH_2C(=O)O-]_2$, $BuN[CH_2CH_2O-]_2$, $BuN[CH_2CH(Me)O-]_2$, $BuN[CH_2CH(Me)O-][CH(Me)CH_2O-]$, $BuN[CH_2C(Me)_2O-]_2$, $BuN[CH_2C(Me)_2O-][C(Me)_2CH_2O-]$, $BuN[CH_2C(=O)O-]_2$, $HexN[CH_2CH_2O-]_2$, $HexN[CH_2CH(Me)O-]_2$, $HexN[CH_2CH(Me)O-]$ $[CH(Me)CH_2O-]$, $HexN[CH_2C(Me)_2O-]_2$, $HexN[CH_2C(Me)_2O-][C(Me)_2CH_2O-]$, $HexN[CH_2C(=O)O-]_2$, $OctN[CH_2CH_2O-]_2$, $OctN[CH_2CH(Me)O-]_2$, $OctN[CH_2CH(Me)O-]$ $[CH(Me)CH_2O-]$, $OctN[CH_2C(Me)_2O-]_2$, $OctN[CH_2C(Me)_2O-][C(Me)_2CH_2O-]$, $OctN[CH_2C(=O)O-]_2$, wobei Pr, Bu, Hex und Oct für alle isomeren Propyl-, Butyl- sowie Octylreste stehen können, $PhN[CH_2CH_2O-]_2$, $PhN[CH_2CH(Me)O-]_2$, $PhN[CH_2CH(Me)O-][CH(Me)CH_2O-]$, $PhN[CH_2C(Me)_2O-]_2$, $PhN[CH_2C(Me)_2O-][C(Me)_2CH_2O-]$, $PhN[CH_2C(=O)O-]_2$,

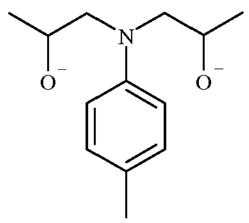

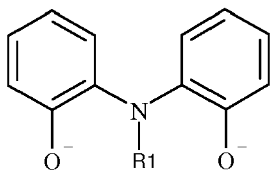

oder

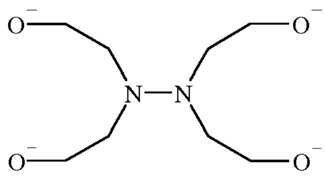

.

Die Zinnverbindungen - wie dem Fachmann bekannt ist - neigen zur Oligomerisierung, so dass häufig mehrkernige Zinnverbindungen oder Gemische aus ein- und mehrkernigen Zinnverbindungen vorliegen. In den mehrkernigen Zinnverbindungen sind die Zinnatome bevorzugt über Sauerstoffatome (‚Sauerstoffbrücken', *vide intra*) miteinander verbunden. Typische oligomere Komplexe (mehrkernige Zinnverbindungen) entstehen z.B. durch Kondensation der Zinnatome über Sauerstoff oder Schwefel, z.B.

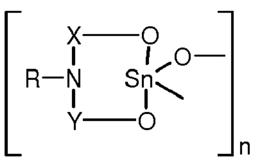

mit n > 1 (vgl. Formel F-II). Bei niedrigen Oligomerisierungsgraden findet man häufig cyclische, bei höheren Oligomerisierungsgraden lineare Oligomere mit OH- bzw. SH-Endgruppen (vgl. Formel F-III).

In einer weiteren bevorzugten Ausführungsform ist die zyklische Zinnverbindung ausgewählt aus der Gruppe mono- oder polyzyklischer Zinnverbindungen vom Typ: 1,1-Di-"R"-5-"organyl"-5-aza-2,8-dioxa-1-stanna-cyclooctane, 1,1-Di-"R"-5-(N-"organyl")aza-3,7-di-"organyl"-2,8-dioxa-1-stanna-cyclooctane, 1,1-Di-"R"-5-(N-"organyl")aza-3,3,7,7-tetra-"organyl"-2,8-dioxa-1-stanna-cyclooctane, 4,12-Di-"organyl"-1,7,9,15-tetraoxa-4,12-diaza-8-stannaspiro[7.7]pentadecane, 4,12-Di-"organyl"-2,6,10,14-tetra-"organyl"-1,7,9,15-tetraoxa-4,12-diaza-8-stannaspiro[7.7]pentadecane, 4,12-Di-"organyl"-2,2,6,6,10,10,14,14-octa-"organyl"-1,7,9,15-tetraoxa-4,12-diaza-8-stannaspiro[7.7]pentadecane, wobei "R" für D*, L3 oder L4, wie oben definiert, steht und "organyl" für R1, wie oben definiert, steht.

In einer weiteren bevorzugten Ausführungsform werden als zyklische Zinnverbindung eine oder mehrere der nachfolgenden Verbindungen eingesetzt: 4,12-Di-n-butyl-1,7,9,15-tetraoxa-4,12-diaza-8-stannaspiro[7.7]pentadecan, 4,12-Di-n-butyl-2,6,10,14-tetramethyl-1,7,9,15-tetraoxa-4,12-diaza-8-stannaspiro[7.7]pentadecan, 2,4,6,10,12,14-Hexamethyl-1,7,9,15-tetraoxa-4,12-diaza-8-stannaspiro[7.7]pentadecan, 4,12-Di-n-octyl-2,6,10,14-tetramethyl-1,7,9,15-tetraoxa-4,12-diaza-8-stannaspiro[7.7]pentadecan, 4,12-Di-n-octyl-1,7,9,15-tetraoxa-4,12-diaza-8-stannaspiro[7.7]-pentadecan, 4,12-Dimethyl-1,7,9,15-tetraoxa-4,12-diaza-8-stannaspiro[7.7]pentadecan, 1,1-Dichloro-5-methyl-5-aza-2,8-dioxa-1-stannacyclooctan, 1,1-Diisopropyl-5-methyl-5-aza-2,8-dioxa-1-stannacyclooctan, 1,1-Dibenzoyl-3,3,7,7-tetramethyl 5-n-octyl-5-aza-2,8-dioxa-1-stannacyclooctan, 1,1-Dibenzoyl- 5-n-octyl-5-aza-2,8-dioxa-1-stannacyclooctan, 1,1-Bis(p-dodecylphenylsulfonyl)- 5-n-octyl-5-aza-2,8-dioxa-1-stannacyclooctan, 2-Benzoyloxy-6-octyl-4,8-dioxo-1,3,6,2-dioxazastannocan-2-ylbenzoat oder Mischungen davon.

In einer bevorzugten Ausgestaltung des Verfahrens weist das Harz freie Isocyanatgruppen gemessen mittels $^{13}C$ NMR in einer Konzentration ≥ 1 Gew.-% bezogen auf das Harz auf. Insbesondere die Konzentration an freien Isocyanatgruppen kann die mechanischen Eigenschaften des ausreagierten Materials der Zahnschiene günstig beeinflussen und zu einer verbesserten Elastizität der Zahnschiene unter Anwendungsbedingungen beitragen. In einer bevorzugten Ausführungsform kann das Harz freie Isocyanatgruppen in einer Konzentration ≥ 1,5, bevorzugt ≥ 2,0, des Weiteren bevorzugt ≥ 2, 5 und weiterhin bevorzugt ≥ 3,0 Gew.-% enthalten.

Innerhalb einer weiteren bevorzugten Ausführungsform des Verfahrens weist das Harz Uretdiongruppen gemessen mittels $^{13}C$ NMR in einer Konzentration ≥ 1 Gew.-% bezogen auf das Harz auf. Insbesondere die Konzentration an Uretdiongruppen kann die mechanischen Eigenschaften des ausreagierten Materials der Zahnschiene günstig beeinflussen und zu einer verbesserten Elastizität der Zahnschiene unter Anwendungsbedingungen beitragen. In einer bevorzugten Ausführungsform kann das Harz Uretdiongruppen in einer Konzentration ≥ 1,5, bevorzugt ≥ 2,0, des Weiteren bevorzugt ≥ 2,5 und weiterhin bevorzugt ≥ 3,0 Gew.-% enthalten.

In einer bevorzugten Ausgestaltung des Verfahrens weist das Harz freie Alkoholgruppen gemessen mittels $^{13}C$ NMR in einer Konzentration ≥ 0,5 Gew.-% bezogen auf die Gesamtmasse des Harz auf. Insbesondere die Konzentration freier Alkoholgruppen im Harz kann die mechanischen Eigenschaften des ausreagierten Materials der Zahnschiene günstig beeinflussen und zu einer verbesserten Elastizität der Zahnschiene unter Anwendungsbedingungen beitragen. In einer bevorzugten Ausführungsform enthält das Harz freie Alkoholgruppen in einer Konzentration ≥ 1,0 Gew.-%, bevorzugt ≥ 1,5 Gew.-%, oder bevorzugt ≥ 2,0 Gew.-% oder bevorzugt ≥ 2,5 Gew.-%, bezogen auf die Gesamtmasse des Harz.

Im Rahmen eines weiteren Aspektes des Verfahrens kann das Harz während und/oder nach dem Druckprozess durch mindestens eine weitere, nicht radikalische Vernetzung vernetzt werden, sich die Netzbogenlänge dabei um mindestens 20% verringern, der $T_g$ um mindestens 3°C, der Modul um mindestens 15% und die Bruchfestigkeit um mindestens 10% steigen, bezogen auf die Eigenschaften des Harzes vor dem Druckprozess. Es hat sich zur Herstellung der erfindungsgemäßen Zahnschienen als besonders günstig erwiesen, dass die mechanische Festigkeit der Zahnschiene über einen zweistufigen Prozess erhalten wird. Dies kann die Freiheitsgrade im 3D-Druckprozess von den mechanischen Eigenschaften der ausreagierten Zahnschiene entkoppeln, sodass die erfindungsgemäßen mechanischen Eigenschaften der Zahnschiene bei Anwendungstemperatur leichter erhältlich sind. Bevorzugt kann die Härtung des erfindungsgemäßen Harzes zum Produkt innerhalb eines 2-stufigen Prozesses erfolgen, wobei zuerst ein Grünkörper im 3D Drucker erzeugt wird, welcher im Anschluss entnommen, von nicht umgesetzten Harz gereinigt und in einem zweiten Schritt mittels Strahlen und/oder thermisch nachgehärtet wird. In einer bevorzugten Ausführungsform wird dabei der zu härtende Grünkörper für einen Zeitraum von ≥ 1 min bis ≤ 72h, bevorzugt ≥ 5 min bis ≤ 24h, oder bevorzugt ≥ 10 min bis ≤ 12h, auf eine Temperatur von ≥ 50°C bis ≤ 250°C, bevorzugt ≥ 80°C ≤ 200°C, oder bevorzugt ≥ 100°C ≤ 180°C aufgeheizt.

Die Netzbogenlänge wird bevorzugt über den 2-stufigen Prozess um 25%, oder bevorzugt um 30% oder bevorzugt um 40% verringert, bezogen auf die Netzbogenlänge des Harzes vor dem Druckprozess.

Die Glasübergangstemperatur, $T_g$, steigt bevorzugt über den 2-stufigen Prozess um 5°C, oder bevorzugt um 7°C, oder bevorzugt um 8°C, oder bevorzugt um 9°C, bezogen auf $T_g$ des Harzes vor dem Druckprozess.

Das Modul steigt bevorzugt über den 2-stufigen Prozess bevorzugt um 20%, oder bevorzugt um 30%, oder bevorzugt um 50%, oder bevorzugt um 100%, bezogen auf das Modul des Harzes vor dem Druckprozess.

Die Bruchfestigkeit kann über den 2-stufigen Prozess bevorzugt um ≥ 15%, oder bevorzugt um ≥ 20%, um ≥ 25% und weiterhin bevorzugt um ≥ 30%, bezogen auf die Bruchfestigkeit des Harzes vor dem Druckprozess steigen.

Innerhalb eines bevorzugten Aspektes des Verfahrens kann das Druckverfahren aus der Gruppe bestehend aus DLP (Dynamic Light Processing), CLIP (Continuous liquid interface production) Inkjet oder SLA (laserbasierter Stereolithografie) ausgesucht sein. Diese Herstellverfahren aus der Gruppe der additiven Fertigungsverfahren, respektive dem 3D-Druck, haben sich zu erhalt der erfindungsgemäßen Zahnschienen als besonders effizient und schnell erwiesen. Mittels dieser Verfahren lassen sich die erfindungsgemäß einsetzbaren Polymer mit einer hohen Ortsauflösung drucken und die erfindungsgemäßen Parameter der Zahnschienen lassen sich reproduzierbar einhalten. Zu möglichen konkreten Ausgestaltungen und möglichen Vorteilen dieser Verfahren wurde schon vorstehend ausgeführt.

Beispiele:

Das Aufbaumaterial im erfindungsgemäßen Verfahren kann beispielsweise die folgende Zusammensetzung haben, wobei alle Zahlenangaben in Gew.-% sind und sich die Angaben in Gew.-% zu ≤ 100 Gew.-% addieren:

| | |
|---|---|
| NCO-funktionelles Urethan(meth)acrylat | 0 - 90 |

(fortgesetzt)

| | |
|---|---|
| Urethan(meth)acrylat | 5-90 |
| Polyisocyanate | 0-50 |
| Polyester(meth) acrylat | 0-90 |
| Epoxy(meth)acrylat | 0-90 |
| Ether(meth)acrylat | 0-90 |
| Blockiertes Urethan(meth)acrylat | 0-90 |
| Polylacton(meth)acrylat | 0-90 |
| Polycarbonat(meth)acrylat | 0-90 |
| Polyamid(meth)acrylat | 0-90 |
| Triallylisocyanurat | 0-20 |
| Diallylcarbonat | 0-30 |
| Styrol | 0-30 |
| Divinylbenzol | 0-20 |
| Methylstyrol | 0-20 |
| Monofunktionelles (Meth)Acrylat | 0-80 |
| Difunktionelles (Meth)Acrylat | 0-80 |
| Polyole (monofuntionell) | 0-40 |
| Polyole (multifuntionell) | 0-40 |
| (Meth)Acrylsäure | 0-10 |
| Polyamine (monofuntionel) | 0-40 |
| Polyamine (multifunktionel) | 0-40 |
| Photoinitiator 1 | 0,1-3 |
| Photoinitiator 2 | 0-3 |
| UV-Inhibitor 1 | 0,01-0,3 |
| UV-Inihibitor 2 | 0-0,3 |
| Stabilisator (gegen radikalische Polymerisation) 1 | 0-1 |
| Stabilisator (gegen radikalische Polymerisation) 2 | 0-1 |
| Stabilisator (gegen Oxidation) 3 | 0-1 |
| Stabilisator (gegen Oxidation) 4 | 0-1 |
| Stabilisator (gegen Vergilbung) 5 | 0-1 |
| Stabilisator (gegen Oxidation) 6 | 0-1 |
| Katalysator 1 | 0,001- 3 |
| Katalysator 2 | 0-3 |
| Katalysator 3 | 0-3 |
| Additive | 0-20 |
| Weichmacher | 0-40 |
| Füllstoffe | 0-60 |
| Funktionalisierte Silane (-OH,-SH,NH,Vinyl,Acryl, Iso) | 0-10 |

Im Speziellen:

| | |
|---|---|
| NCO-funktionelles Urethanacrylat | 20 |
| Urethanacrylat | 30 |
| Monofunktionelles Acrylat | 30 |
| Polyol | 10 |
| Photoinitiator 1 | 2 |
| UV-Inhibitor | 0,2 |
| Stabilisator (gegen radikalische Polymerisation) 1 | 0,05 |
| Kat | 0,1 |

NCO-funktionelles Urethanacrylat: Beispielsweise ein Urethanacrylat, welches aus der Umsetzung von trimerem HDI-Isocyanurat mit Hydroxypropylacrylat bei einer NCO-Kennzahl von 200 durch Rühren bei 60 °C, bis alle OH Gruppen umgesetzt sind, erhalten werden kann.

Urethaneacrylat: Beispielsweise ein Urethanacrylat, welches aus der Umsetzung von Polyethylenglycol mit einem mittleren Molekulargewicht von ca.300 mit einem 10fachen molaren Überschuss Hexamethylendiisocyanat und nach Abdestillation des überschüssigen Isocyanats auf einen Restgehalt von < 1% mit Hydroxymethylmethacrylat titriert wurde, bis kein Isocyanat mehr vorhanden war.

Acrylat: beispielsweise Isobornylacrylat

Polyol: beispielsweise Butandiol

Photoinitiator 1: Germanium-basierter Photoinitiator wie beispielsweise Bis-4-(methoxybenzoyl)diethylgerman

UV-Inhibitor: beispielsweise Mayzo OB+ (2,2'-(2,5-thiophendiyl)bis(5-tertbutylbenzoxazol))

Kat: zyklische Zinnverbindung der Formel F-I, F-II oder F-III

Ein weiterer Gegenstand der vorliegenden Erfindung ist ein Gegenstand, erhalten durch ein erfindungsgemäßes Verfahren, wobei der Gegenstand in Aufbaurichtung seines Herstellungsverfahrens wenigstens abschnittsweise eine Höhe von ≥ 1 mm, vorzugsweise ≥ 5 mm, aufweist.

Die Erfindung betrifft ebenfalls die Verwendung von zyklischen Zinnverbindungen der Formel F-I, F-II und/oder F-III, wie in den vorstehenden Ausführungen definiert, als thermisch latente Urethanisierungskatalysatoren in Aufbaumaterialien für additive Fertigungsverfahren.

**Patentansprüche**

1. Kieferorthopädische Zahnschiene,
**dadurch gekennzeichnet, dass**
die Zahnschiene ein vernetztes Polymer umfasst oder aus einem solchen besteht, wobei das vernetzte Polymer eine Glasübergangstemperatur $T_g$, bestimmt mittels mechanisch dynamischer Analyse (DMA) bei einer Frequenz von 1/s DMA als Peak tan $\delta$, von ≥ 25°C und ≤ 60°C, ein Elastizitätsmodul, bestimmt mittels mechanisch dynamischer Analyse (DMA) als Speichermodul E' bei einer Frequenz von 1/s bei 35°C, von ≥ 500 MPa und ≤ 4000 MPa und einen Verlustfaktor tan $\delta$, bestimmt mittels mechanisch dynamischer Analyse (DMA) bei einer Frequenz von 1/s bei 35°C, von ≥ 0,08 aufweist.

2. Zahnschiene gemäß Anspruch 1, wobei das Elastizitätsmodul bestimmt mittels mechanisch dynamischer Analyse (DMA) als Speichermodul E' im Temperaturbereich von ≥ 30°C und ≤ 60°C größer oder gleich 500 MPa und kleiner oder gleich 4000 MPa beträgt.

3. Zahnschiene gemäß einem der vorhergehenden Ansprüche, wobei das vernetzte Polymer ein vernetztes Polyurethan umfasst.

**4.** Zahnschiene gemäß Anspruch 3, wobei das vernetzte Polymer einen Isocyanuratanteil ermittelt über $^{13}C$-NMR von ≥ 3% aufweist.

**5.** Zahnschiene gemäß Anspruch 3 oder 4, wobei das vernetzte Polymer einen Urethananteil ermittelt über $^{13}C$ NMR von ≥ 3% aufweist.

**6.** Zahnschiene gemäß einem der vorhergehenden Ansprüche, wobei das vernetzte Polymer einen Brechungsindex gemessen mit einem Abbe Refraktometer von >1,48 RI und < 1,58 RI aufweist.

**7.** Zahnschiene gemäß einem der vorhergehenden Ansprüche, wobei das vernetzte Polymer eine mittlere Netzbogenlänge nach Flory und Huggins von > 300 g/mol und < 5000 g/mol aufweist.

**8.** Zahnschiene nach einem der vorhergehenden Ansprüche, wobei das Polymer ein transparentes Polymer mit einer Lichttransmission gemessen in einem UV-VIS Spektrometer an einer Probe mit einer Dicke von 1 mm im Wellenlängenbereich von 400-800 nm von >50% ist.

**9.** Zahnschiene nach einem der vorhergehenden Ansprüche, wobei das Polymer ein transparentes Polymer enthaltend Polyurethane und / oder Polysilicone ist und eine Abbe Zahl von > 20 aufweist.

**10.** Ein Verfahren zur Herstellung einer kieferorthopädischen Zahnschiene,
**dadurch gekennzeichnet, dass**
mittels eines 3D-Druckverfahrens ein Harz enthaltend actinisch polymerisierbare Doppelbindungen in Form einer kieferorthopädischen Zahnschiene ausgedruckt und polymerisiert wird.

**11.** Verfahren nach Anspruch 10, wobei das Harz freie Isocyanatgruppen gemessen mittels $^{13}C$ NMR in einer Konzentration ≥ 1 Gew.-% bezogen auf das Harz aufweist.

**12.** Verfahren nach einem der Ansprüche 10 - 11, wobei das Harz Uretdiongruppen gemessen mittels $^{13}C$ NMR in einer Konzentration ≥ 1 Gew.-% bezogen auf das Harz aufweist.

**13.** Verfahren nach einem der Ansprüche 10 - 12, wobei das Harz freie Alkoholgruppen gemessen mittels $^{13}C$ NMR in einer Konzentration ≥ 0,5 Gew.-% bezogen auf das Harz aufweist.

**14.** Verfahren nach einem der Ansprüche 10 - 13, wobei das Harz während und/oder nach dem Druckprozess durch mindestens eine, nicht radikalische Vernetzung vernetzt wird und sich die Netzbogenlänge dabei um mindestens 20% verringert, der $T_g$ um mindestens 3°C, der Modul um mindestens 15% und die Bruchfestigkeit um mindestens 10% steigt.

**15.** Verfahren gemäß einem der Ansprüche 10 bis 14, wobei das Druckverfahren ausgesucht ist aus der Gruppe bestehend aus DLP (Dynamic Light Processing), CLIP (Continuous liquid interface production) Inkjet oder SLA (laserbasierter Stereolithografie).

Europäisches Patentamt
European Patent Office
Office européen des brevets

# EUROPÄISCHER RECHERCHENBERICHT

Nummer der Anmeldung
EP 18 19 5715

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2018/005501 A1 (3M INNOVATIVE PROPERTIES CO [US]) 4. Januar 2018 (2018-01-04) * Seite 6, Zeile 15 - Seite 7, Zeile 19 * * Seite 10, Zeile 5 - Seite 11, Zeile 22 * | 1-9 | INV. A61C7/08 |
| X | US 2009/148813 A1 (SUN BENJAMIN J [US] ET AL) 11. Juni 2009 (2009-06-11) * Absatz [0025] * * Absatz [0026] - Absatz [0028] * * Absatz [0097] - Absatz [0101] * | 1-9 | |
| X | WO 2018/119026 A1 (3M INNOVATIVE PROPERTIES CO [US]) 28. Juni 2018 (2018-06-28) * Seite 12, Zeile 9 - Seite 13, Zeile 21 * * Seite 17, Zeile 13 - Seite 18, Zeile 20 * | 1-9 | |
| X | US 2017/007386 A1 (MASON DAVID [US] ET AL) 12. Januar 2017 (2017-01-12) * Absatz [0089] - Absatz [0091] * * Absatz [0108] * * Absatz [0119] * | 1-3,5-9 | RECHERCHIERTE SACHGEBIETE (IPC) A61C |

Der vorliegende Recherchenbericht wurde für alle Patentansprüche erstellt

2

| Recherchenort | Abschlußdatum der Recherche | Prüfer |
|---|---|---|
| München | 19. März 2019 | Salvatore, Claudio |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt
European Patent Office
Office européen des brevets

Nummer der Anmeldung

EP 18 19 5715

## GEBÜHRENPFLICHTIGE PATENTANSPRÜCHE

Die vorliegende europäische Patentanmeldung enthielt bei ihrer Einreichung Patentansprüche, für die eine Zahlung fällig war.

☐ Nur ein Teil der Anspruchsgebühren wurde innerhalb der vorgeschriebenen Frist entrichtet. Der vorliegende europäische Recherchenbericht wurde für jene Patentansprüche erstellt, für die keine Zahlung fällig war, sowie für die Patentansprüche, für die Anspruchsgebühren entrichtet wurden, nämlich Patentansprüche:

☐ Keine der Anspruchsgebühren wurde innerhalb der vorgeschriebenen Frist entrichtet. Der vorliegende europäische Recherchenbericht wurde für die Patentansprüche erstellt, für die keine Zahlung fällig war.

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

Siehe Ergänzungsblatt B

☐ Alle weiteren Recherchengebühren wurden innerhalb der gesetzten Frist entrichtet. Der vorliegende europäische Recherchenbericht wurde für alle Patentansprüche erstellt.

☐ Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

☒ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

1-9

☐ Der vorliegende ergänzende europäische Recherchenbericht wurde für die Teile der Anmeldung erstellt, die sich auf die zuerst in den Patentansprüchen erwähnte Erfindung beziehen (Regel 164 (1) EPÜ).

Europäisches Patentamt
European Patent Office
Office européen des brevets

# MANGELNDE EINHEITLICHKEIT DER ERFINDUNG ERGÄNZUNGSBLATT B

Nummer der Anmeldung
EP 18 19 5715

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

1. Ansprüche: 1-9

Kieferorthopädische Zahnschiene, dadurch gekennzeichnet, dass die Zahnschiene ein vernetztes Polymer umfasst oder aus einem solchen besteht, wobei das vernetzte Polymer eine Glasübergangstemperatur Tg , bestimmt mittels mechanisch dynamischer Analyse (DMA) bei einer Frequenz von 1/s DMA als Peak tan , von 25°C und 60°C, ein Elastizitätsmodul, bestimmt mittels mechanisch dynamischer Analyse (DMA) als Speichermodul E' bei einer Frequenz von 1/s bei 35°C, von 500 MPa und 4000 MPa und einen Verlustfaktor tan , bestimmt mittels mechanisch dynamischer Analyse (DMA) bei einer Frequenz von 1/s bei 35°C, von 0,08 aufweist. Die Erfindung betrifft weiterhin ein Verfahren zur Herstellung ebensolcher Zahnschienen.

---

2. Ansprüche: 10-15

Ein Verfahren zur Herstellung einer kieferorthopädischen Zahnschiene, dadurch gekennzeichnet, dass mittels eines 3D-Druckverfahrens ein Harz enthaltend actinisch polymerisierbare Doppelbindungen in Form einer kieferorthopädischen Zahnschiene ausgedruckt und polymerisiert wird.

---

## ANHANG ZUM EUROPÄISCHEN RECHERCHENBERICHT ÜBER DIE EUROPÄISCHE PATENTANMELDUNG NR. EP 18 19 5715

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

19-03-2019

| Im Recherchenbericht angeführtes Patentdokument | Datum der Veröffentlichung | Mitglied(er) der Patentfamilie | Datum der Veröffentlichung |
|---|---|---|---|
| WO 2018005501 A1 | 04-01-2018 | AU 2017289257 A1 | 17-01-2019 |
| | | BR 112018077517 A2 | 02-04-2019 |
| | | CN 109716234 A | 03-05-2019 |
| | | EP 3479171 A1 | 08-05-2019 |
| | | JP 2019519405 A | 11-07-2019 |
| | | KR 20190022777 A | 06-03-2019 |
| | | US 2019163060 A1 | 30-05-2019 |
| | | WO 2018005501 A1 | 04-01-2018 |
| US 2009148813 A1 | 11-06-2009 | CA 2698189 A1 | 12-03-2009 |
| | | CA 2928909 A1 | 12-03-2009 |
| | | EP 2187835 A1 | 26-05-2010 |
| | | JP 5297460 B2 | 25-09-2013 |
| | | JP 2010537720 A | 09-12-2010 |
| | | US 2009148813 A1 | 11-06-2009 |
| | | WO 2009032228 A2 | 12-03-2009 |
| WO 2018119026 A1 | 28-06-2018 | KEINE | |
| US 2017007386 A1 | 12-01-2017 | US 2017007386 A1 | 12-01-2017 |
| | | US 2017008333 A1 | 12-01-2017 |
| | | US 2019133732 A1 | 09-05-2019 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 20160256240 A1
- US 20130095446 A1
- US 20130122448 A1
- US 5975893 A
- EP 0206059 A
- EP 0540985 A
- GB 2221465 A
- GB 2222161 A
- DE 3240613 A
- DE 3219608 A
- EP 0100129 A
- GB PS1391066 A
- GB PS1386399 A
- GB 809809 A
- EP 0056158 A
- EP 0056159 A
- EP 0033581 A
- EP 13196508 A
- DE 102009051445 A1


### In der Beschreibung aufgeführte Nicht-Patentliteratur


- *European Polymer Journal,* 1979, vol. 16, 147-148
- *J. Organomet. Chem.,* 2009, vol. 694, 3184-3189
- *Chem. Heterocycl. Comp.,* 2007, vol. 43, 813-834
- *Indian J. Chem.,* 1967, vol. 5, 643-645